(19) Europäisches Patentamt
European Patent Office
Office européen des brevets

(11) EP 1 112 273 B1

(12) EUROPÄISCHE PATENTSCHRIFT

(45) Veröffentlichungstag und Bekanntmachung des Hinweises auf die Patenterteilung:
**13.10.2004 Patentblatt 2004/42**

(21) Anmeldenummer: **99946075.1**

(22) Anmeldetag: **31.08.1999**

(51) Int Cl.[7]: **C07F 7/04**, C08G 77/18, C11D 9/44, A61K 7/06, A61K 7/48, A61K 7/32

(86) Internationale Anmeldenummer:
**PCT/EP1999/006393**

(87) Internationale Veröffentlichungsnummer:
**WO 2000/014091 (16.03.2000 Gazette 2000/11)**

# (54) KIESELSÄUREESTER

SILICIC-ACID ESTERS

ESTERS D'ACIDE SILICIQUE

(84) Benannte Vertragsstaaten:
**AT BE CH CY DE DK ES FI FR GB GR IE IT LI LU MC NL PT SE**

(30) Priorität: **09.09.1998 DE 19841147**

(43) Veröffentlichungstag der Anmeldung:
**04.07.2001 Patentblatt 2001/27**

(73) Patentinhaber: **Henkel Kommanditgesellschaft auf Aktien**
**40589 Düsseldorf (DE)**

(72) Erfinder:
- **GERKE, Thomas**
  **D-41468 Neuss (DE)**
- **SCHAPER, Ulf-Armin**
  **D-47804 Krefeld (DE)**
- **FABER, Werner**
  **D-47877 Willich (DE)**
- **JESCHKE, Rainer**
  **D-40595 Düsseldorf (DE)**
- **MEINE, Georg**
  **D-40822 Mettmann (DE)**

(56) Entgegenhaltungen:
**EP-A- 0 878 497** **WO-A-96/28497**
**DE-A- 3 003 494** **DE-A- 19 750 706**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft die Verwendung oligomerer Kieselsäureester, die Reste von Duftstoffalkoholen enthalten und sich beispielsweise zur Beduftung von Wasch- und Reinigungsmitteln eignen, da sie bei der Hydrolyse die duftenden Alkohole freisetzen.

Die kontrollierte Freisetzung von Inhaltsstoffen in den unterschiedlichsten Präparaten, gerne als "controlled release" bezeichnet, ist Gegenstand zahlreicher Veröffentlichungen und Patentanmeldungen. Auf dem Gebiet der Wasch- und Reinigungsmittel sind dabei beschleunigte oder verzögerte Freisetzungen von Inhaltsstoffen aus der Gruppe der Bleichmittel, Bleichaktivatoren, Tenside usw. von besonderem Interesse. Von herausragender Bedeutung ist auf diesem Gebiet die Freisetzung von Duftstoffen, da sowohl das Produkt, als auch die Wasch- und Reinigungslösung und die mit diesen Mitteln behandelten Gegenstände intensiv und langanhaltend beduftet werden sollen. Neben den Methoden, Duftstoffe auf Trägermaterialien aufzubringen und die bedufteten Träger zu beschichten, oder Duftstoffe zu verkapseln oder in Verbindungen einzulagern (beispielsweise Cyclodextrin-Parfüm-Komplexe), existiert die Möglichkeit, die Duftstoffe chemisch an Trägermedien zu binden, wobei die chemische Bindung langsam gespalten und der Duftstoff freigesetzt wird. Dieses Prinzip ist beispielsweise bei der Veresterung von Duftstoffalkoholen verwirklicht worden, wobei zu dieser Stoffgruppe ein breiter Stand der Technik existiert.

Im Stand der Technik existieren einige Vorschläge, duftende Alkohole an nicht flüchtige Siloxane zu binden, aus denen sie durch Hydrolyse langsam freigesetzt werden. Obwohl auch zu Siloxanestern von Duftstoffalkoholen ein breiter Stand der Technik existiert, treten beim Einsatz der genannten Verbindungen in Wasch- und Reinigungsmitteln Probleme auf. So sind viele der bekannten Verbindungen in wäßrigen Wasch- und Reinigungsmitteln nicht einsetzbar, da sie bereits im Produkt hydrolysieren und die verzögerte Freisetzung hierdurch bedingt später nicht mehr auftritt. Dies ist um so mehr der Fall, als übliche Wasch- und Reinigungsmittel oft pH-Werte aufweisen, die die Hydrolyse noch verstärken. Aber auch in pulverförmigen Wasch- und Reinigungsmitteln gelingt die Einarbeitung der bislang bekannten Siloxanester nicht, Unter üblichen Herstellbedingungen verdichteter Teilchengemische wie Granulierung oder Preßagglomeration neigen die Siloxanester ebenfalls dazu, den Duftstoffalkohol bereits bei der Herstellung, d.h. verfrüht, freizusetzen. Es ist also ein Bedürfnis, Duftstoff-Angebotsformen bereitzustellen, die sowohl das Produkt beduften, als auch die Waschund Reinigungslösung und die mit den Produkten behandelten Substrate, wobei der Duft insbesondere auf Textilien noch möglichst lange anhalten soll.

Monomere Orthokieselsäureester von Duftstoffalkoholen werden beispielsweise in der **US 3,215,719** (Dan River Mills) beschrieben. Diese Schrift nennt auch die verzögerte Freisetzung duftender Alkohole aus gemischten Estern wie beispielsweise Bis(eugenoxy)diethoxysilan oder Bis(cinnamoyloxy)diethoxysilan, wobei das zentrale Si nicht zwingend nur an Sauerstoff gebunden sein muß. Oligomere Siloxanester werden in dieser Schrift nicht beschrieben.

Pulverförmige oder granulatartige Wasch- und Reinigungsmittelzusammensetzungen, die "Wohlgeruch verleihende" Silicium-Verbindungen enthalten, werden in der **DE 28 44 789** (Dow Corning) beschrieben. Die hierin offenbarten mono-, oligo- und polymeren Siliciumverbindungen weisen nicht zwingend ein von vier Sauerstoffatomen umgebenes Si-Zentralatom auf. Oligomere Si-Verbindungen mit mehr als einer Duftstoffalkohol-Estergruppe werden in dieser Schrift ebenfalls nicht beschrieben.

Flüssige oder pastenförmige Seifenzusammensetzungen, die "Wohlgeruch verleihende" Silicium-Verbindungen enthalten, werden in der **DE 30 03 494** (Dow Coming) beschrieben. Auch in dieser Schrift werden oligomere Si-Verbindungen mit mehr als einer Duftstoffalkohol-Estergruppe nicht beschrieben.

Duftgebende, nicht flüchtige Siloxane der allgemeinen Formel $M_aM'_{a'}D_bD'_{b'}T_cT'_{c'}Q_d$ mit M und M'= $R^1R^2R^3SiO_{1/2}$, D und D'= $R^4R^5SiO_{2/2}$, T und T'= $R^6SiO_{3/2}$ und Q = $SiO_{4/2}$, wobei $R^1$ bis $R^6$ unabhängig voneinander ausgewählt sind aus $C_{1-40}$-Alkyl- oder -Alkoxysowie -Aryl- oder -Aryloxyresten und die Indizes a, a' positiv und ein oder mehrere der Indizes b, b', c, c' und d positiv oder 0 sind, werden in der **DE 197 50 706** (General Electric) beschrieben. Formal fallen auch oligomere Si-Verbindungen mit vier Duftstoffalkohol-Estergruppen unter die allgemeine Formel, doch werden in dieser Schrift lediglich Verbindungen explizit offenbart, bei denen mindestens zwei C-Atome direkt an ein Si-Atom gebunden sind. Der Einsatz der duftgebenden Siloxane in Waschund Reinigungsmitteln wird in dieser Schrift ebenfalls nicht erwähnt.

Es bestand also nach wie vor die Aufgabe, hydrolysestabilere Siloxanester von Duftstoffalkoholen bereitzustellen, die sich auch in wäßrige Wasch- und Reinigungsmittel einarbeiten lassen, ohne bereits im Produkt übermäßigen Hydrolyseerscheinungen zu unterliegen. Auch die Einarbeitbarkeit der Verbindungen in granulare Wasch- und Reinigungsmittelzusammensetzungen, ohne daß es im Herstellprozeß zu Zersetzungen kommt, ist eine Anforderung an die bereitzustellenden Verbindungen. Die herzustellenden Substanzen sollten dabei den mit der Lösung behandelten Substraten einen angenehmen und langanhaltenden Duft verleihen.

Es wurde nun gefunden, daß die Verwendung von Estern von Oligokieselsäuren mit Duftstoffalkoholen das geforderte Anforderungsprofil erfüllt. Es wurde überraschend festgestellt, daß diese in Kombination mit üblichen Duftstoffen auch den Duftstoffen eine länger anhaltende Wirkung verleihen, die nicht mit den Oligokieselsäuren verestert

sind. Unabhängig von der chemischen Zusammensetzung der Duftstoffe lassen sich die Kieselsäureester also in Duftstoffmischungen einsetzen, um der gesamten Parfümzusammensetzung eine verlängerte Duftfreisetzung zu verleihen.

Gegenstand der Erfindung ist daher die Verwendung von Kieselsäureestern der Formel I

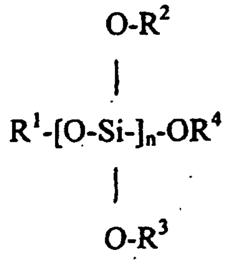

(I)

in der $R^1$ und $R^4$ unabhängig voneinander ausgewählt sind aus der Gruppe der geradkettigen oder verzweigten, gesättigten oder ungesättigten, substituierten oder unsubstituierten $C_{1-6}$-Kohlenwasserstoffreste und der Duftstoffalkoholreste, jedes $R^2$ und $R^3$ unabhängig voneinander ausgewählt ist aus der Gruppe der Duftstoffalkoholreste und n Werte zwischen 2 und 20 annimmt, zur Verlängerung der Duftwirkung von anderen Duftstoffen.

Die Herstellung der genannten Verbindungen gelingt durch einfache Umesterung von Oligokieselsäureestern niederer Alkohole mit Duftstoffalkoholen, wobei sowohl einzelne Duftstoffalkohole als auch Duftstoffalkoholgemische eingesetzt werden können. Je nach Reaktionszeit und -bedingungen werden die niederen Alkohole abgespalten und die Duftstoffalkohole gebunden, wobei die Alkohole entlang der Si-O-Si-Kette leichter ausgetauscht werden als die terminalen Alkohole. Auf diese Weise lassen sich oligomere Kieselsäureester herstellen, in denen die Reste $R^2$ und $R^3$ Duftstoffalkoholreste sind, während die terminalen Reste $R^1$ und $R^4$ noch unumgesetzt sind, also Reste niederer Alkohole darstellen.

Oligokieselsäureester niederer Alkohole sind kommerziell erhältlich, wobei üblicherweise Methanol, Ethanol, *n*-Propanol, *iso*-Propanol, *n*-Butanol, *iso*-Butanol und *tert*-Butanol zur Veresterung eingesetzt wurden. Die Darstellung nicht vollständig mit Duftstoffalkoholen umgeesterter Oligokieselsäureester führt zu Kieselsäureestern der Formel I, in denen $R^1$ und $R^4$ unabhängig voneinander ausgewählt sind aus der Gruppe Methyl, Ethyl, *n*-Propyl, *iso*-Propyl, *n*-Butyl, *iso*-Butyl und *tert*-Butyl. Solche Verbindungen sind im Rahmen der vorliegenden Erfindung bevorzugt.

Werden nicht-vollständig umgeesterte Oligokieselsäureester hergestellt, sind die Reste $R^2$ und $R^3$ vorzugsweise aus der Gruppe der Duftstoffalkoholreste ausgewählt. Unter dem Begriff "Duftstoffalkohole" werden im Rahmen der vorliegenden Erfindung Duftstoffe verstanden, die über freie Hydroxylgruppen verfügen, welche veresterbar sind, unabhängig davon, wie das Molekül weiter aufgebaut ist. So lassen sich auch Salicylsäureester als Duftstoffalkohole einsetzen. Aus der großen Gruppe der Duftstoffalkohole lassen sich bevorzugte Vertreter nennen, so daß im Rahmen der vorliegenden Erfindung Kieselsäureester bevorzugt sind, in denen jedes $R^2$ und $R^3$ unabhängig voneinander ausgewählt ist aus der Gruppe der Reste folgender Duftstoffalkohole: 10-Undecen-1-ol., 2,6-Dimethylheptan-2-ol, 2-Methylbutanol, 2-Methylpentanol, 2-Phenoxyethanol, 2-Phenylpropanol, 2-tert-Butycyclohexanol, 3,5,5-Trimethylcyclohexanol, 3-Hexanol, 3-Methyl-5-phenyl pentanol, 3-Octanol, 3-Phenylpropanol, 4-Heptenol, 4-Isopropylcyclohexanol, 4-tert-Butycyclohexanol, 6,8-Dimethyl-2-nonanol, 6-Nonen-1-ol, 9-Decen-1-ol, alpha-Methylbenzylalkohol, alpha-Terpineol, Amylsalicylat, Benzylalkohol, Benzylsalicylat, beta-Terpineol, Butylsalicylat, Citronellol, Cyclohexylsalicylat, Decanol, Dihydromyrcenol, Dimethylbenzylcarbinol, Dimethylheptanol, Dimethyloctanol, Ethylsalicylat, Ethylvanilin, Eugenol, Geraniol, Heptanol, Hexylsalicylat, Isoborneol, Isoeugenol, Isopulegol, Linalool, Menthol, Myrtenol, n-Hexanol, Nerol, Nonanol, Octanol, para-Menthan-7-ol, Phenylethylalkohol, Phenol, Phenylsalicylat, Tetrahydrogeraniol, Tetrahydrolinalool, Thymol, trans-2-cis-6-Nonadicnol, trans-2-Nonen-1-ol, trans-2-Octenol, Undecanol, Vanillin, Zimtalkohol.

Die vollständig umgeesterten Oligokieselsäureester sind im Rahmen der vorliegenden Erfindung besonders bevorzugt. Es sind dies Produkte, in denen jeder Rest $R^1$ bis $R^4$ einen Duftstoffalkoholrest darstellt, wobei Oligokieselsäureester bevorzugt sind, in denen jeder Rest $R^1$ bis $R^4$ unabhängig voneinander ausgewählt ist aus der Gruppe der Reste folgender Duftstoffalkohole: 10-Undecen-1-ol., 2,6-Dimethylheptan-2-ol, 2-Methylbutanol, 2-Methylpentanol, 2-Phenoxyethanol, 2-Phenylpropanol, 2-tert-Butycyclohexanol, 3,5,5-Trimethylcyclohexanol, 3-Hexanol, 3-Methyl-5-phenyl pentanol, 3-Octanol, 3-Phenylpropanol, 4-Heptenol, 4-Isopropylcyclohexanol, 4-tert-Butycyclohexanol, 6,8-Dimethyl-2-nonanol, 6-Nonen-1-ol, 9-Decen-1-ol, alpha-Methylbenzylalkohol, alpha-Terpineol, Amylsalicylat, Benzylalkohol, Benzylsalicylat, beta-Terpineol, Butylsalicylat, Citronellol, Cyclohexylsalicylat, Decanol, Dihydromyrcenol, Dimethylbenzylcarbinol, Dimethylheptanol, Dimethyloctanol, Ethylsalicylat, Ethylvanilin, Eugenol, Geraniol, Heptanol, Hexylsalicylat, Isobomeol, Isoeugenol, Isopulegol, Linalool, Menthol, Myrtenol, n-Hexanol, Nerol, Nonanol, Octanol, para-Menthan-7-ol, Phenylethylalkohol, Phenol, Phenylsalicylat, Tetrahydrogeraniol, Tetrahydrolinalool, Thymol, trans-2-cis-6-Nonadicnol, trans-2-Nonen-1-ol, trans-2-Octenol, Undecanol, Vanillin, Zimtalkohol.

Die Oligomerisierungsgrade "n" der erfindungsgemäß zu verwendenden Kieselsäureester liegen zwischen 2 und 20. In bevorzugten Verbindungen nimmt n Werte zwischen 2 und 15, vorzugsweise zwischen 2 und 12 und insbesondere zwischen 3 und 10 unter besonderer Bevorzugung der Werte 4, 5, 6, 7 und 8, an. Besonders bevorzugte Oligokieselsäureester lassen sich also durch die Formeln II, III, IV, V und VI beschreiben:

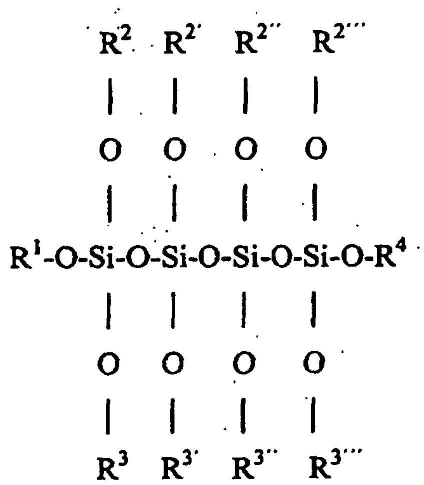

(II)

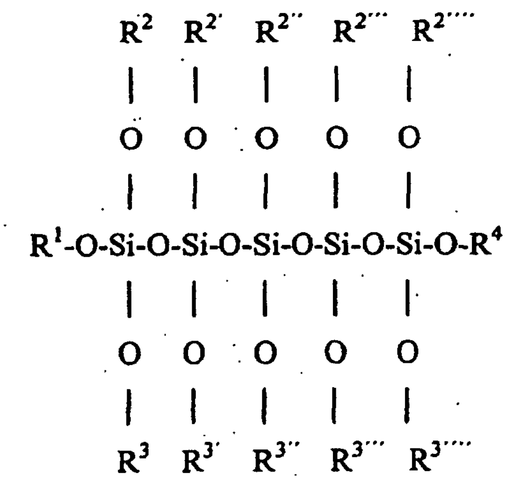

(III)

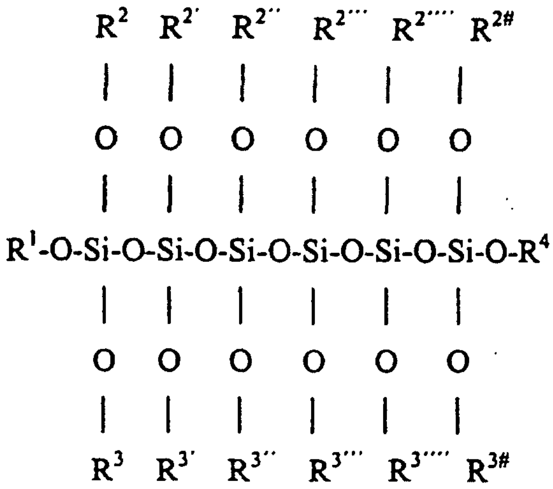

(IV)

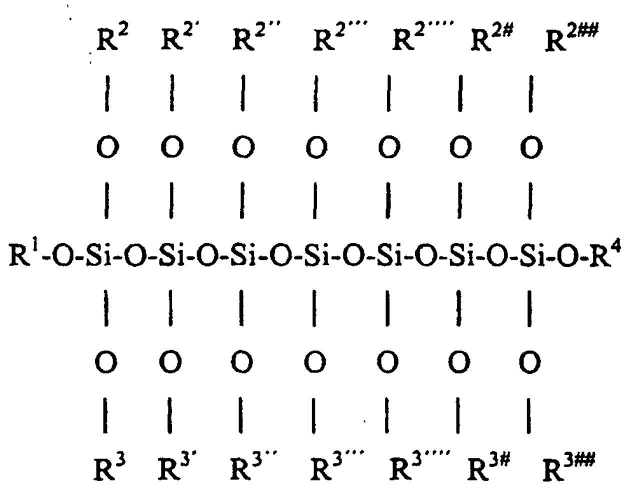

(V)

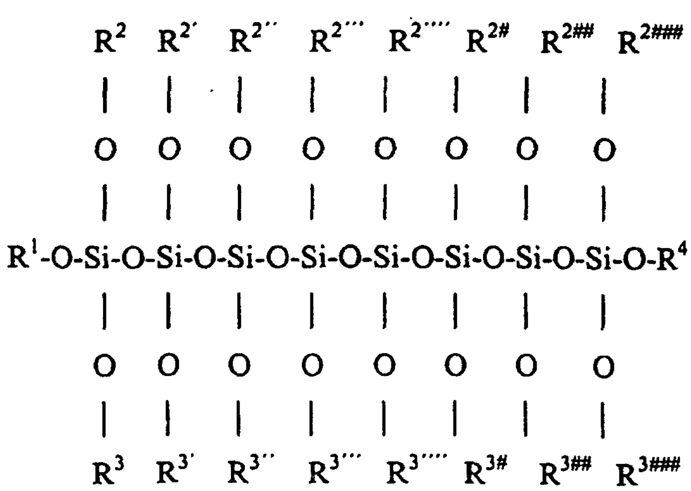

(VI)

In den Formeln II, III, IV, V und VI können die Reste $R^2$, $R^{2'}$, $R^{2''}$, $R^{2'''}$, $R^{2''''}$ , $R^{2\#}$, $R^{2\#\#}$, $R^{2\#\#\#}$, $R^3$, $R^{3'}$, $R^{3''}$, $R^{3'''}$, $R^{3''''}$, $R^{3\#}$, $R^{3\#\#}$ und $R^{3\#\#\#}$ von jeweils ein und demselben Duftstoffalkohol abgeleitet sein oder aus unterschiedlichen Duftstoffalkoholen stammen. Im letzteren Fall setzt man bei der Umesterung Gemische verschiedener Duftstoffalkohole ein und erhält auf diese Weise in Abhängigkeit von den eingesetzten Mengenverhältnissen der Duftstoffalkohole gemischte Oligokieselsäureester; in denen die Reste $R^2$, $R^{2'}$, $R^{2''}$, $R^{2'''}$, $R^{2''''}$, $R^{2\#}$, $R^{2\#\#}$, $R^{2\#\#\#}$, $R^3$, $R^{3'}$, $R^{3''}$, $R^{3'''}$, $R^{3''''}$, $R^{3\#}$, $R^{3\#\#}$ und $R^{3\#\#\#}$ voneinander unterschiedlich sein können.

Obwohl auf diese Weise die unterschiedlichsten Ester herstellbar sind, ist es im Rahmen der vorliegenden Erfindung bevorzugt, vollständig umgeesterte Oligokieselsäureester herzustellen, die nur auf einem einzigen Duftstoffalkohol basieren, d.h. daß die Reste $R^1$ bis $R^4$ identisch sind, also vom gleichen Duftstoffalkohol stammen.

Ein weiterer Gegenstand der Erfindung sind daher Kieselsäureester der Formel (Ia)

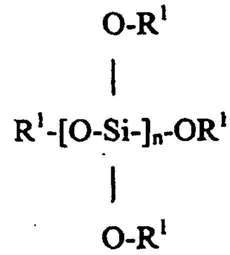

(Ia)

in der $R^1$ ausgewählt ist aus der Gruppe der Duftstoffalkoholreste, insbesondere aus der Gruppe der Reste folgender Duftstoffalkohole, 10-Undecen-1-ol., 2,6-Dimethylheptan-2-ol, 2-Methylbutanol, 2-Methylpentanol, 2-Phenoxyethanol, 2-Phenylpropanol, 2-tert-Butycyclohexanol, 3,5,5-Trimethylcyclohexanol, 3-Hexanol, 3-Methyl-5-phenyl pentanol, 3-Octanol, 3-Phenylpropanol, 4-Heptenol, 4-Isopropyl cyclohexanol, 4-tert-Butycyclohexanol, 6,8-Dimethyl-2-nonanol, 6-Nonen-1-ol, 9-Decen-1-ol, alpha-Methylbenzylalkohol, alpha-Terpineol, Amylsalicylat, Benzylalkohol, Benzylsalicylat, beta-Terpineol, Butylsalicylat, Citronellol, Cyclohexylsalicylat, Decanol, Dihydromyrcenol, Dimethylbenzylcarbinol, Dimethylheptanol, Dimethyloctanol, Ethylsalicylat, Ethylvanilin, Eugenol, Geraniol, Heptanol, Hexylsalicylat, Isoborneol, Isoeugenol, Isopulegol, Linalool, Menthol, Myrtenol, n-Hexanol, Nerol, Nonanol, Octanol, para-Menthan-7-ol, Phenylethylalkohol, Phenol, Phenylsalicylat, Tetrahydrogeraniol, Tetrahydrolinalool, Thymol, trans-2-cis-6-Nonadicnol, trans-2-Nonen-1-ol, trans-2-Octenol, Undecanol, Vanillin, Zimtalkohol, und n Werte zwischen 2 und 20 annimmt.

Da die Ausgangsverbindungen für die Herstellung der erfindungsgemäßen und/oder erfindungsgemäß zu verwendenden Verbindungen aus ökonomischen Gründen vorzugsweise keine Reinstoffe sind, sondern technische Gemische von Oligokieselsäureestern niederer Alkohole mit unterschiedlichen Oligomerisierungsgraden, findet sich die Verteilung der Oligomerisierungsgrade auch in den erfindungsgemäßen und/oder erfindungsgemäß zu verwendenden Estern wieder. Die in den Formeln II, III, IV, V und VI angegebenen Verbindungen stellen dabei einzelne Spezies dar, die in technischen Gemischen der erfindungsgemäßen und/oder erfindungsgemäß zu verwendenden Kieselsäureester bevorzugt in hohen Mengen vorkommen. Erfindungsgemäß einsetzbar sind aber auch die Gemische der genannten Verbindungen und damit gebrochene Oligomerisierungsgrade.

Die erfindungsgemäßen und/oder erfindungsgemäß zu verwendenden Kieselsäureester zeichnen sich durch eine gute Hydrolysestabilität aus und können auch in wäßrigen Medien bzw. in Herstellprozessen für Granulate eingesetzt werden, ohne dabei übermäßige Aktivitätsverluste zu erleiden. Auf diese Weise sind flüssige Wasch- und Reinigungsmittel wie Flüssigwaschmittel, Weichspülmittel, Handgeschirrspülmittel, Reinigungsmittel für harte Oberflächen, Bodenwischmittel usw. ebenso denkbar wie feste Wasch- und Reinigungsmittel, beispielsweise Textilwaschmittelgranulate, maschinelle Geschirrspülmittel oder Putz- und Scheuermittel. Ebenso können die erfindungsgemäßen und/oder erfindungsgemäß zu verwendenden Kieselsäureester in kosmetischen Mitteln zur Haut- und Haarbehandlung eingesetzt werden. Auch hier sind dabei sowohl flüssige Mittel, wie beispielsweise Duschbäder, Deodorants und Haarshampoo gemeint, wie auch feste Mittel, wie beispielsweise Seifenstücke, gemeint.

Aufgrund der hervorragenden Eignung der erfindungsgemäßen und/oder erfindungsgemäß zu verwendenden Verbindungen zum Einsatz in Wasch- und Reinigungsmitteln ist die Verwendung von Kieselsäureestem der allgemeinen Formel I

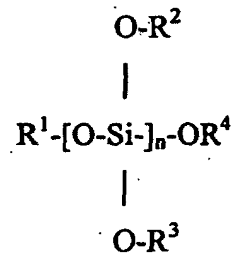

(I)

in der $R^1$ und $R^4$ unabhängig voneinander ausgewählt sind aus der Gruppe der geradkettigen oder verzweigten, gesättigten oder ungesättigten, substituierten oder unsubstituierten $C_{1-6}$-Kohlenwasserstoffreste und der Duftstoffalkoholreste, jedes $R^2$ und $R^3$ unabhängig voneinander ausgewählt ist aus der Gruppe der Duftstoffalkoholreste und n Werte zwischen 2 und 20 annimmt, als Duftstoff in flüssigen oder festen Wasch- und Reinigungsmitteln zusammen mit anderen Duftstoffen ein weiterer Gegenstand der vorliegenden Erfindung.

Ebenso eignen sich die Kieselsäureester hervorragend zum Einsatz in kosmetischen Mitteln, daher ist ein weiterer Gegenstand der vorliegenden Erfindung die Verwendung von Kieselsäureestern der allgemeinen Formel I

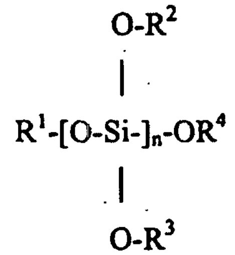

(I)

in der $R^1$ und $R^4$ unabhängig voneinander ausgewählt sind aus der Gruppe der geradkettigen oder verzweigten, gesättigten oder ungesättigten, substituierten oder unsubstituierten $C_{1-6}$-Kohlenwasserstoffreste und der Duftstoffalkoholreste, jedes $R^2$ und $R^3$ unabhängig voneinander ausgewählt ist aus der Gruppe der Duftstoffalkoholreste und n Werte zwischen 2 und 20 annimmt, als Duftstoff zusammen mit anderen Duftstoffen in kosmetischen Mitteln zur Haut- und Haarbehandlung.

Ebenso ist die Verwendung von Kieselsäureestern der allgemeinen Formel Ia

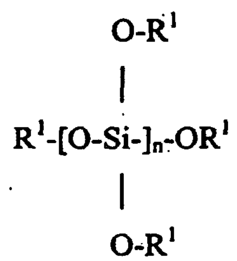

(Ia)

in der $R^1$ ausgewählt ist aus der Grußße der Duftstoffalkoholreste, und n Werte zwischen 2 und 20 annimmt, als Duftstoff in flüssigen oder festen Wasch- und Reinigungsmitteln ein weiterer Gegenstand der vorliegenden Erfindung.

Die erfindungsgemäßen und/oder erfindungsgemäß zu verwendenden Kieselsäureester können dabei je nach Art und Verwendungszweck der zu beduftenden Mittel in variierenden Mengen eingebracht werden. Üblicherweise werden die Kieselsäureester in Mengen von 0,001 bis 10 Gew.-%, vorzugsweise von 0,01 bis 5 Gew.-%, besonders bevorzugt von 0,02 bis 3 und insbesondere in Mengen von 0,05 bis 2 Gew.-%, jeweils bezogen auf das betroffene Mittel, eingesetzt.

Die Kieselsäureester können als alleiniger Duftstoff eingesetzt werden, es ist aber auch möglich, Duftstoffgemische einzusetzen, die lediglich zu einem Teil aus den Kieselsäureestern bestehen. Solche Gemische haben den Vorteil, daß auch die Bestandteile des Duftstoffgemischs, die nicht als veresterte Duftstoffalkohole vorliegen, in der Haltbarkeit des Dufteindrucks verbessert werden. So können insbesondere Duftstoffgemische eingesetzt werden, die 1 bis 50 Gew.-%, vorzugsweise 5 bis 40 und insbesondere maximal 30 Gew.-% an Kieselsäureester enthalten. In anderen Ausführungsformen, bei denen insbesondere die verzögerte Duftwirkung der veresterten Duftalkohole genutzt

werden soll, werden bei der erfindungsgemäßen Verwendung vorteilhaft mindestens 30 Gew.-%, vorzugsweise mindestens 40 Gew.-% und insbesondere mindestens 50 Gew.-% des insgesamt im Mittel enthaltenen Parfüms über die erfindungsgemäßen und/oder erfindungsgemäß zu verwendenden Kieselsäureester in die Mittel eingebracht, während die restlichen 70 Gew.-%, vorzugsweise 60 Gew.-% und insbesondere 50 Gew.-% des insgesamt im Mittel enthaltenen Parfüms auf übliche Weise aufgesprüht oder anders in die Mittel eingebracht werden. Die erfindungsgemäße Verwendung kann also vorteilhaft dadurch gekennzeichnet sein, daß die Kieselsäureester der Formel Ia zusammen mit anderen Duftstoffen eingesetzt werden.

Durch die Aufteilung des Gesamt-Parfümgehaltes der Mittel in Parfüm, welches in den Kieselsäureestern enthalten ist und Parfüm, das herkömmlich eingearbeitet wurde, läßt sich eine Vielzahl von Produktcharakteristiken realisieren, die erst durch die erfindungsgemäße Verwendung möglich werden. So ist es beispielsweise denkbar und möglich, den Gesamt-Parfümgehalt der Mittel in zwei Portionen x und y aufzuteilen, wobei der Anteil x aus haftfesten, d.h. weniger flüchtigen und der Anteil y aus leichter flüchtigen Parfümölen besteht.

Es sind nun beispielsweise Wasch- oder Reinigungsmittel herstellbar, in denen der Anteil des Parfüms, der über die Kieselsäureester in die Mittel eingebracht wird, hauptsächlich aus haftfesten Riechstoffen zusammengesetzt ist: Auf diese Weise können haftfeste Riechstoffe, die die behandelten Gegenstände, insbesondere Textilien, beduften sollen, im Produkt "festgehalten" werden und ihre Wirkung dadurch hauptsächlich auf der behandelten Wäsche entfalten. Demgegenüber tragen die leichter flüchtigen Riechstoffe zu einer intensiveren Beduftung der Mittel an sich bei. Auf diese Weise ist es auch möglich, Wasch- und Reinigungsmittel herzustellen, die als Mittel einen Geruch aufweisen, der sich vom Geruch der behandelten Gegenstände unterscheidet. Der Kreativität von Parfümeuren sind dabei kaum Grenzen gesetzt, da über die Wahl der Riechstoffe einerseits und über die Wahl der Einarbeitungsmethode in die Mittel andererseits nahezu grenzenlose Möglichkeiten existieren, die Mittel und über die Mittel die mit ihnen behandelten Gegenstände zu beduften.

Das oben beschriebene Prinzip läßt sich selbstverständlich auch umkehren, indem die leichter flüchtigen Riechstoffe in die Kieselsäureester inkorporiert und die schwerer flüchtigen, haftfesten Riechstoffe auf die Mittel aufgesprüht oder anders eingearbeitet werden. Auf diese Weise wird der Verlust der leichter flüchtigen Riechstoffe aus der Verpackung bei Lagerung und Transport minimiert, während die Duftcharakteristik der Mittel von den haftfesteren Parfümen bestimmt wird.

Die einzige Grenze dieser Vorgehensweise ist, daß die Duftstoffe, die über die erfindungsgemäßen und/oder erfindungsgemäß zu verwendenden Kieselsäureester eingebracht werden sollen, aus der Gruppe der Duftstoffalkohole stammen. Die Duftstoffe, die auf herkömmliche Weise in die Mittel inkorporiert werden, sind keinerlei Beschränkungen unterworfen. So können als Parfümöle bzw. Duftstoffe einzelne Riechstoffverbindungen, z.B. die synthetischen Produkte vom Typ der Ester, Ether, Aldehyde, Ketone, Alkohole und Kohlenwasserstoffe verwendet werden. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat (DMBCA), Phenylethylacetat, Benzylacetat, Ethylmethylphenylglycinat, Allylcyclohexylpropionat, Styrallylpropionat, Benzylsalicylat, Cyclohexylsalicylat, Floramat, Melusat und Jasmacyclat. Zu den Ethern zählen beispielsweise Benzylethylether und Ambroxan , zu den Aldehyden z.B. die linearen Alkanale mit 8 - 18 C-Atomen, Citral, Citronellal, Citronellyloxy-acetaldehyd, Cyclamenaldehyd, Lilial und Bourgeonal, zu den Ketonen z.B. die Jonone, $\alpha$-Isomethylionon und Methyl-cedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene wie Limonen und Pinen. Bevorzugt werden jedoch Mischungen verschiedener Riechstoffe verwendet, die gemeinsam eine ansprechende Duftnote erzeugen.

Solche Parfümöle können auch natürliche Riechstoffgemische enthalten, wie sie aus pflanzlichen Quellen zugänglich sind, z.B. Pine-, Citrus-, Jasmin-, Patchouly-, Rosenoder Ylang-Ylang-Öl. Ebenfalls geeignet sind Muskateller-Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliöl, Orangenschalenöl und Sandelholzöl.

Die allgemeine Beschreibung der einsetzbaren Parfüme (siehe oben) stellt allgemein die unterschiedlichen Substanzklassen von Riechstoffen dar. Um wahrnehmbar zu sein, muß ein Riechstoff flüchtig sein, wobei neben der Natur der funktionellen Gruppen und der Struktur der chemischen Verbindung auch die Molmasse eine wichtige Rolle spielt. So besitzen die meisten Riechstoffe Molmassen bis etwa 200 Dalton, während Molmassen von 300 Dalton und darüber eher eine Ausnahme darstellen. Aufgrund der unterschiedlichen Flüchtigkeit von Riechstoffen verändert sich der Geruch eines aus mehreren Riechstoffen zusammengesetzten Parfüms bzw. Duftstoffs während des Verdampfens, wobei man die Geruchseindrücke in "Kopfnote" (top note), "Herz- bzw. Mittelnote" (middle note bzw. body) sowie "Basisnote" (end note bzw. dry out) unterteilt. Da die Geruchswahrnehmung zu einem großen Teil auch auf der Geruchsintensität beruht, besteht die Kopfnote eines Parfüms bzw. Duftstoffs nicht allein aus leichtflüchtigen Verbindungen, während die Basisnote zum größten Teil aus weniger flüchtigen, d.h. haftfesten Riechstoffen besteht. Bei der Komposition von Parfüms können leichter flüchtige Riechstoffe beispielsweise an bestimmte Fixative gebunden werden, wodurch ihr zu schnelles Verdampfen verhindert wird. Die oben beschriebene Ausführungsform der vorliegenden Erfindung, in der die leichter flüchtigen Riechstoffe bzw. Duftstoffe in den erfindungsgemäßen und/oder erfindungsgemäß

zu verwendenden Kieselsäureestern gebunden vorliegen, ist eine solche Methode zur Riechstoffixierung. Bei der nachfolgenden Einteilung der Riechstoffe in "leichter flüchtige" bzw. "haftfeste" Riechstoffe ist also über den Geruchseindruck und darüber, ob der entsprechende Riechstoff als Kopf- oder Herznote wahrgenommen wird, nichts ausgesagt.

Haftfeste Riechstoffe, die im Rahmen der vorliegenden Erfindung einsetzbar sind, sind beispielsweise die ätherischen Öle wie Angelikawurzelöl, Anisöl, Arnikablütenöl, Basilikumöl, Bayöl, Champacablütenöl, Edeltannenöl, Edeltannenzapfenöl, Elemiöl, Eukalyptusöl, Fenchelöl, Fichtennadelöl, Galbanumöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefernnadelöl, Kopaivabalsamöl, Korianderöl, Krauseminzeöl, Kümmelöl, Kuminöl, Lavendelöl, Lemongrasöl, Limetteöl, Mandarinenöl, Melissenöl, Moschuskömeröl, Myrrhenöl, Nelkenöl, Neroliöl, Niaouliöl, Olibanumöl, Origanumöl, Palmarosaöl, Patchuliöl, Perubalsamöl, Petitgrainöl, Pfefferöl, Pfefferminzöl, Pimentöl, Pine-Öl, Rosenöl, Rosmarinöl, Sandelholzöl, Sellerieöl, Spiköl, Stemanisöl, Terpentinöl, Thujaöl, Thymianöl, Verbenaöl, Vetiveröl, Wacholderbeeröl, Wermutöl, Wintergrünöl, Ylang-Ylang-Öl, Ysop-Öl, Zimtöl, Zimtblätteröl, Zitronellöl, Zitronenöl sowie Zypressenöl. Aber auch die höhersiedenden bzw. festen Riechstoffe natürlichen oder synthetischen Ursprungs können im Rahmen der vorliegenden Erfindung als haftfeste Riechstoffe bzw. Riechstoffgemische, also Duftstoffe, eingesetzt werden. Zu diesen Verbindungen zählen die nachfolgend genannten Verbindungen sowie Mischungen aus diesen: Ambrettolid, Ambroxan, $\alpha$-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylalkohol, Benzylacetat, Benzylbenzoat, Benzylformiat, Benzylvalerianat, Borneol, Bornylacetat, Boisambrene forte, $\alpha$-Bromstyrol, n-Decylaldehyd, n-Dodecylaldehyd, Eugenol, Eugenolmethylether, Eukalyptol, Famesol, Fenchon, Fenchylacetat, Geranylacetat, Geranylformiat, Heliotropin, Heptincarbonsäuremethylester, Heptaldehyd, Hydrochinon-Dimethylether, Hydroxyzimtaldehyd, Hydroxyzimtalkohol, Indol, Iron, Isoeugenol, Isoeugenolmethylether, Isosafrol, Jasmon, Kampfer, Karvakrol, Karvon, p-Kresolmethylether, Cumarin, p-Methoxyacetophenon, Methyl-n-amylketon, Methylanthranilsäuremethylester, p-Methylacetophenon, Methylchavikol, p-Methylchinolin, Methyl-$\beta$-naphthylketon, Methyl-n-nonylacetaldehyd, Methyl-n-nonylketon, Muskon, $\beta$-Naphtholethylether, $\beta$-Naphtholmethylether, Nerol, n-Nonylaldehyd, Nonylalkohol, n-Octylaldehyd, p-Oxy-Acetophenon, Pentadekanolid, $\beta$-Phenylethylalkohol, Phenylacetaldehyd-Dimethyacetal, Phenylessigsäure, Pulegon, Safrol, Salicylsäureisoamylester, Salicylsäuremethylester, Salicylsäurehexylester, Salicylsäurecyclohexylester, Santalol, Sandelice, Skatol, Terpineol, Thymen, Thymol, Troenan, $\gamma$-Undelacton, Vanilin, Veratrumaldehyd, Zimtaldehyd, Zimatalkohol, Zimtsäure, Zimtsäureethylester, Zimtsäurebenzylester.

Zu den leichter flüchtigen Riechstoffen zählen insbesondere die niedriger siedenden Riechstoffe natürlichen oder synthetischen Ursprungs, die allein oder in Mischungen eingesetzt werden können. Beispiele für leichter flüchtige Riechstoffe sind Diphenyloxid, Limonen, Linalool, Linalylacetat und -propionat, Melusat, Menthol, Menthon, Methylnheptenon, Pinen, Phenylacetaldehyd, Terpinylacetat, Citral, Citronellal.

Daß nicht nur die chemisch gebundenen Duftstoffalkohole verzögert und damit länger anhaltend freigesetzt werden, sondern auch die Duftstoffe, die lediglich im Gemisch mit den erfindungsgemäßen und/oder erfindungsgemäß zu verwendenden Kieselsäureestern vorliegen, ist überraschend. Ein weiterer Gegenstand der vorliegenden Erfindung sind daher Duftstoffmischungen oder Parfümzusammensetzungen, enthaltend Kieselsäureester der Formel I

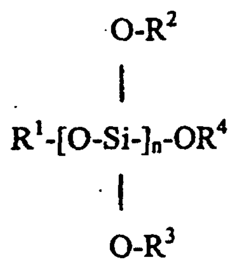

(I)

in der $R^1$ und $R^4$ unabhängig voneinander ausgewählt sind aus der Gruppe der geradkettigen oder verzweigten, gesättigten oder ungesättigten, substituierten oder unsubstituierten $C_{1-6}$-Kohlenwasserstoffreste und der Duftstoffalkoholreste, jedes $R^2$ und $R^3$ unabhängig voneinander ausgewählt ist aus der Gruppe der Duftstoffalkoholreste und n Werte zwischen 2 und 20 annimmt, sowie andere Duftstoffe.

Ein weiterer Gegenstand der vorliegenden Erfindung, sind Wasch- und Reinigungsmittel, die solche Duftstoffmischungen oder Parfümzusammensetzungen, enthaltend Kieselsäureester der Formel I

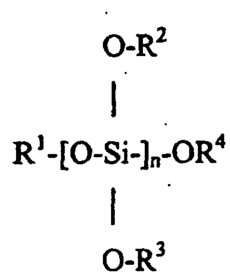

(I)

in der $R^1$ und $R^4$ unabhängig voneinander ausgewählt sind aus der Gruppe der geradkettigen oder verzweigten, gesättigten oder ungesättigten, substituierten oder unsubstituierten $C_{1-6}$-Kohlenwasserstoffreste und der Duftstoffalkoholreste, jedes $R^2$ und $R^3$ unabhängig voneinander ausgewählt ist aus der Gruppe der Duftstoffalkoholreste und n Werte zwischen 2 und 20 annimmt, enthalten. Vorzugsweise enthalten die Mittel dabei Kieselsäureester der Formel I in Mengen von 0,001 bis 10 Gew.-%, vorzugsweise von 0,01 bis 5 Gew.-%, besonders bevorzugt von 0,02 bis 3 und insbesondere in Mengen von 0,05 bis 2 Gew.-%, jeweils bezogen auf das Wasch- und Reinigungsmittel. Dabei enthalten die Mittel, wie bereits oben beschrieben wurde, neben diesen Kieselsäureestern noch weitere Duftstoffe. Die geeigneten Duftstoffe sind im einzelnen bereits weiter oben aufgeführt worden.

Neben den Duftstoffen können die Wasch- und Reinigungsmittel selbstverständlich übliche Inhaltsstoffe von solchen Mitteln enthalten. Hier sind in erster Linie Tenside; Buildersubstanzen sowie Bleichmittel, Enzyme und andere Aktivstoffe zu nennen. Zu den wesentlichen Inhaltsstoffen von Wasch- und Reinigungsmitteln gehören dabei insbesondere Tenside.

Je nach Einsatzzweck der erfindungsgemäßen Mittel wird man den Tensidgehalt höher oder niedriger wählen. Üblicherweise liegt der Tensidgehalt von Waschmitteln zwischen 10 und 40 Gew.-%, vorzugsweise zwischen 12,5 und 30 Gew.-% und insbesondere zwischen 15 und 25 Gew.-%, während Reinigungsmittel für das maschinelle Geschirrspülen zwischen 0,1 und 10 Gew.-%, vorzugsweise zwischen 0,5 und 7,5 Gew.-% und insbesondere zwischen 1 und 5 Gew.-% Tenside enthalten.

Diese grenzflächenaktive Substanzen stammen aus der Gruppe der anionischen, nichtionischen, zwitterionischen oder kationischen Tenside, wobei anionische Tenside aus ökonomischen Gründen und aufgrund ihres Leistungsspektrums beim Waschen und Reinigen deutlich bevorzugt sind.

Als anionische Tenside werden beispielsweise solche vom Typ der Sulfonate und Sulfate eingesetzt. Als Tenside vom Sulfonat-Typ kommen dabei vorzugsweise $C_{9-13}$-Alkylbenzolsulfonate, Olefinsulfonate, d.h. Gemische aus Alken- und Hydroxyalkansulfonaten sowie Disulfonaten, wie man sie beispielsweise aus $C_{12-18}$-Monoolefinen mit end- oder innenständiger Doppelbindung durch Sulfonieren mit gasförmigem Schwefeltrioxid und anschließende alkalische oder saure Hydrolyse der Sulfonierungsprodukte erhält, in Betracht. Geeignet sind auch Alkansulfonate, die aus $C_{12-18}$-Alkanen beispielsweise durch Sulfochlorierung oder Sulfoxidation mit anschließender Hydrolyse bzw. Neutralisation gewonnen werden. Ebenso sind auch die Ester von $\alpha$-Sulfofettsäuren (Estersulfonate), z.B. die $\alpha$-sulfonierten Methylester der hydrierten Kokos-, Palmkern- oder Talgfettsäuren geeignet.

Weitere geeignete Aniontenside sind sulfierte Fettsäureglycerinester. Unter Fettsäureglycerinestern sind die Mono-, Di- und Triester sowie deren Gemische zu verstehen, wie sie bei der Herstellung durch Veresterung von einem Monoglycerin mit 1 bis 3 Mol Fettsäure oder bei der Umesterung von Triglyceriden mit 0,3 bis 2 Mol Glycerin erhalten werden. Bevorzugte sulfierte Fettsäureglycerinester sind dabei die Sulfierprodukte von gesättigten Fettsäuren mit 6 bis 22 Kohlenstoffatomen, beispielsweise der Capronsäure, Caprylsäure, Caprinsäure, Myristinsäure, Laurinsäure, Palmitinsäure, Stearinsäure oder Behensäure.

Als Alk(en)ylsulfate werden die Alkali- und insbesondere die Natriumsalze der Schwefelsäurehalbester der $C_{12}$-$C_{18}$-Fettalkohole, beispielsweise aus Kokosfettalkohol, Talgfettalkohol, Lauryl-, Myristyl-, Cetyl- oder Stearylalkohol oder der $C_{10}$-$C_{20}$-Oxoalkohole und diejenigen Halbester sekundärer Alkohole dieser Kettenlängen bevorzugt. Weiterhin bevorzugt sind Alk(en)ylsulfate der genannten Kettenlänge, welche einen synthetischen, auf petrochemischer Basis hergestellten geradkettigen Alkylrest enthalten, die ein analoges Abbauverhalten besitzen wie die adäquaten Verbindungen auf der Basis von fettchemischen Rohstoffen. Aus waschtechnischem Interesse sind die $C_{12}$-$C_{16}$-Alkylsulfate und $C_{12}$-$C_{15}$-Alkylsulfate sowie $C_{14}$-$C_{15}$-Alkylsulfate bevorzugt. Auch 2,3-Alkylsulfate, welche beispielsweise gemäß den US-Patentschriften 3,234,258 oder 5,075,041 hergestellt werden und als Handelsprodukte der Shell Oil Company unter dem Namen DAN® erhalten werden können, sind geeignete Aniontenside.

Auch die Schwefelsäuremonoester der mit 1 bis 6 Mol Ethylenoxid ethoxylierten geradkettigen oder verzweigten $C_{7-21}$-Alkohole, wie 2-Methyl-verzweigte $C_{9-11}$-Alkohole mit im Durchschnitt 3,5 Mol Ethylenoxid (EO) oder $C_{12-18}$-Fettalkohole mit 1 bis 4 EO, sind geeignet. Sie werden in Reinigungsmitteln aufgrund ihres hohen Schaumverhaltens nur in relativ geringen Mengen, beispielsweise in Mengen von 1 bis 5 Gew.-%, eingesetzt.

Weitere geeignete Aniontenside sind auch die Salze der Alkylsulfobernsteinsäure, die auch als Sulfosuccinate oder als Sulfobernsteinsäureester bezeichnet werden und die Monoester und/oder Diester der Sulfobernsteinsäure mit Alkoholen, vorzugsweise Fettalkoholen und insbesondere ethoxylierten Fettalkoholen darstellen. Bevorzugte Sulfosuccinate enthalten $C_{8-18}$-Fettalkoholreste oder Mischungen aus diesen. Insbesondere bevorzugte Sulfosuccinate enthalten einen Fettalkoholrest, der sich von ethoxylierten Fettalkoholen ableitet, die für sich betrachtet nichtionische Tenside darstellen (Beschreibung siehe unten). Dabei sind wiederum Sulfosuccinate, deren Fettalkohol-Reste sich von ethoxylierten Fettalkoholen mit eingeengter Homologenverteilung ableiten, besonders bevorzugt. Ebenso ist es auch möglich, Alk(en)ylbemsteinsäure mit vorzugsweise 8 bis 18 Kohlenstoffatomen in der Alk(en)ylkette oder deren Salze einzusetzen.

Als weitere anionische Tenside kommen insbesondere Seifen in Betracht. Geeignet sind gesättigte Fettsäureseifen, wie die Salze der Laurinsäure, Myristinsäure, Palmitinsäure, Stearinsäure, hydrierte Erucasäure und Behensäure sowie insbesondere aus natürlichen Fettsäuren, z.B. Kokos-, Palmkern- oder Talgfettsäuren, abgeleitete Seifengemische.

Die anionischen Tenside einschließlich der Seifen können in Form ihrer Natrium-, Kalium- oder Ammoniumsalze sowie als lösliche Salze organischer Basen, wie Mono-, Dioder Triethanolamin, vorliegen. Vorzugsweise liegen die anionischen Tenside in Form ihrer Natrium-, Kalium- oder Magnesiumsalze, insbesondere in Form der Natriumsalze vor.

Bei der Auswahl der anionischen Tenside stehen der Formulierungsfreiheit keine einzuhaltenden Rahmenbedingungen im Weg. Bevorzugte Mittel weisen jedoch einen Gehalt an Seife auf, der 0,2 Gew.-%, bezogen auf das Gesamtgewicht des in Schritt d) hergestellten Wasch- und Reinigungsmittel, übersteigt. Bevorzugt einzusetzende anionische Tenside sind dabei die Alkylbenzolsulfonate und Fettalkoholsulfate, wobei bevorzugte Waschmittelformkörper 2 bis 20 Gew.-%, vorzugsweise 2,5 bis 15 Gew.-% und insbesondere 5 bis 10 Gew.-% Fettalkoholsulfat(e), jeweils bezogen auf das Gewicht der Mittel, enthalten

Als nichtionische Tenside werden vorzugsweise alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt, in denen der Alkoholrest linear oder bevorzugt in 2-Stellung methylverzweigt sein kann bzw. lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, z.B. aus Kokos-, Palm-, Talgfett- oder Oleylalkohol, und durchschnittlich 2 bis 8 EO pro Mol Alkohol bevorzugt. Zu den bevorzugten ethoxylierten Alkoholen gehören beispielsweise $C_{12-14}$-Alkohole mit 3 EO oder 4 EO, $C_{9-11}$-Alkohol mit 7 EO, $C_{13-15}$-Alkohole mit 3 EO, 5 EO, 7 EO oder 8 EO, $C_{12-18}$-Alkohole mit 3 EO, 5 EO oder 7 EO und Mischungen aus diesen, wie Mischungen aus $C_{12-14}$-Alkohol mit 3 EO und $C_{12-18}$-Alkohol mit 5 EO. Die angegebenen Ethoxylierungsgrade stellen statistische Mittelwerte dar, die für ein spezielles Produkt eine ganze oder eine gebrochene Zahl sein können. Bevorzugte Alkoholethoxylate weisen eine eingeengte Homologenverteilung auf (narrow range ethoxylates, NRE). Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO.

Eine weitere Klasse bevorzugt eingesetzter nichtionischer Tenside, die entweder als alleiniges nichtionisches Tensid oder in Kombination mit anderen nichtionischen Tensiden eingesetzt werden, sind alkoxylierte, vorzugsweise ethoxylierte oder ethoxylierte und propoxylierte Fettsäurealkylester, vorzugsweise mit 1 bis 4 Kohlenstoffatomen in der Alkylkette, insbesondere Fettsäuremethylester, wie sie beispielsweise in der japanischen Patentanmeldung JP 58/217598 beschrieben sind oder die vorzugsweise nach dem in der internationalen Patentanmeldung WO-A-90/13533 beschriebenen Verfahren hergestellt werden.

Eine weitere Klasse von nichtionischen Tensiden, die vorteilhaft eingesetzt werden kann, sind die Alkylpolyglycoside (APG). Einsetzbare Alkylpolyglycoside genügen der allgemeinen Formel $RO(G)_z$, in der R für einen linearen oder verzweigten, insbesondere in 2-Stellung methylverzweigten, gesättigten oder ungesättigten, aliphatischen Rest mit 8 bis 22, vorzugsweise 12 bis 18 C-Atomen bedeutet und G das Symbol ist, das für eine Glykoseeinheit mit 5 oder 6 C-Atomen, vorzugsweise für Glucose, steht. Der Glycosidierungsgrad z liegt dabei zwischen 1,0 und 4,0, vorzugsweise zwischen 1,0 und 2,0 und insbesondere zwischen 1,1 und 1,4.

Bevorzugt eingesetzt werden lineare Alkylpolyglycoside, also Alkylpolyglycoside, in denen der Polyglycosylrest ein Glucoserest und der Alkylrest ein n-Alkylrest ist.

Die Tensidgranulate können bevorzugt Alkylpolyglycoside enthalten, wobei Gehalte an APG über 0,2 Gew.-%, bezogen auf den gesamten Formkörper, bevorzugt sind. Besonders bevorzugte Waschmittelformkörper enthalten APG in Mengen von 0,2 bis 10 Gew.-%, vorzugsweise 0,2 bis 5 Gew.-% und insbesondere von 0,5 bis 3 Gew.-%.

Auch nichtionische Tenside vom Typ der Aminoxide, beispielsweise N-Kokosalkyl-N,N-dimethylaminoxid und N-Talgalkyl-N,N-dihydroxyethylaminoxid, und der Fettsäurealkanolamide können geeignet sein. Die Menge dieser nichtionischen Tenside beträgt vorzugsweise nicht mehr als die der ethoxylierten Fettalkohole, insbesondere nicht mehr als die Hälfte davon.

Weitere geeignete Tenside sind Polyhydroxyfettsäureamide der Formel (I),

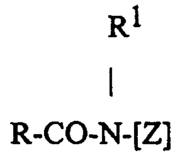

(I)

in der RCO für einen aliphatischen Acylrest mit 6 bis 22 Kohlenstoffatomen, $R^1$ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 10 Kohlenstoffatomen und 3 bis 10 Hydroxylgruppen steht. Bei den Polyhydroxyfettsäureamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können.

Zur Gruppe der Polyhydroxyfettsäureamide gehören auch Verbindungen der Formel (II),

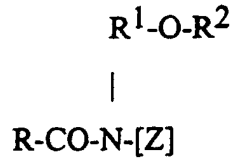

(II)

in der R für einen linearen oder verzweigten Alkyl- oder Alkenylrest mit 7 bis 12 Kohlenstoffatomen, $R^1$ für einen linearen, verzweigten oder cyclischen Alkylrest oder einen Arylrest mit 2 bis 8 Kohlenstoffatomen und $R^2$ für einen linearen, verzweigten oder cyclischen Alkylrest oder einen Arylrest oder einen Oxy-Alkylrest mit 1 bis 8 Kohlenstoffatomen steht, wobei $C_{1-4}$-Alkyl- oder Phenylreste bevorzugt sind und [Z] für einen linearen Polyhydroxyalkylrest steht, dessen Alkylkette mit mindestens zwei Hydroxylgruppen substituiert ist, oder alkoxylierte, vorzugsweise ethoxylierte oder propoxylierte Derivate dieses Restes.

[Z] wird vorzugsweise durch reduktive Aminierung eines reduzierten Zuckers erhalten, beispielsweise Glucose, Fructose, Maltose, Lactose, Galactose, Mannose oder Xylose. Die N-Alkoxy- oder N-Aryloxy-substituierten Verbindungen können dann beispielsweise nach der Lehre der internationalen Anmeldung WO-A-95/07331 durch Umsetzung mit Fettsäuremethylestern in Gegenwart eines Alkoxids als Katalysator in die gewünschten Polyhydroxyfettsäureamide überführt werden.

Eine andere bedeutende Gruppe von Wasch- und Reinigungsmittelinhaltsstoffen sind die Buildersubstanzen. Unter dieser Substanzklasse, werden sowohl organische als auch anorganische Gerüstsubstanzen verstanden. Es handelt sich dabei um Verbindungen, die sowohl eine Trägerfunktion in den erfindungsgemäßen Mitteln wahrnehmen können als auch bei der Anwendung als wasserenthärtende Substanz wirken.

Brauchbare organische Gerüstsubstanzen sind beispielsweise die in Form ihrer Natriumsalze einsetzbaren Polycarbonsäuren, wobei unter Polycarbonsäuren solche Carbonsäuren verstanden werden, die mehr als eine Säurefunktion tragen. Beispielsweise sind dies Citronensäure, Adipinsäure, Bernsteinsäure, Glutarsäure, Äpfelsäure, Weinsäure, Maleinsäure, Fumarsäure, Zuckersäuren, Aminocarbonsäuren, Nitrilotriessigsäure (NTA), sofern ein derartiger Einsatz aus ökologischen Gründen nicht zu beanstanden ist, sowie Mischungen aus diesen. Bevorzugte Salze sind die Salze der Polycarbonsäuren wie Citronensäure, Adipinsäure, Bernsteinsäure, Glutarsäure, Weinsäure, Zuckersäuren und Mischungen aus diesen. Auch die Säuren an sich können eingesetzt werden. Die Säuren besitzen neben ihrer Builderwirkung typischerweise auch die Eigenschaft einer Säuerungskomponente und dienen somit, wie beispielsweise in den erfindungsgemäßen Granulaten, auch zur Einstellung eines niedrigeren und milderen pH-Wertes von Wasch- oder Reinigungsmitteln. Insbesondere sind hierbei Citronensäure, Bernsteinsäure, Glutarsäure, Adipinsäure, Gluconsäure und beliebige Mischungen aus diesen zu nennen.

Als Builder sind weiter polymere Polycarboxylate geeignet, dies sind beispielsweise die Alkalimetallsalze der Polyacrylsäure oder der Polymethacrylsäure, beispielsweise solche mit einer relativen Molekülmasse von 500 bis 70000 g/mol. Diese Substanzklasse wurde im Detail bereits weiter oben beschrieben. Die (co-)polymeren Polycarboxylate können entweder als Pulver oder als wäßrige Lösung eingesetzt werden. Der Gehalt der Mittel an (co-)polymeren Polycarboxylaten beträgt vorzugsweise 0,5 bis 20 Gew.-%, insbesondere 3 bis 10 Gew.-%.

Zur Verbesserung der Wasserlöslichkeit können die Polymere auch Allylsulfonsäuren, wie beispielsweise in der EP-B-727448 Allyloxybenzolsulfonsäure und Methallylsulfonsäure, als Monomer enthalten. Insbesondere bevorzugt sind auch biologisch abbaubare Polymere aus mehr als zwei verschiedenen Monomereinheiten, beispielsweise solche, die gemäß der DE-A-43 00 772 als Monomere Salze der Acrylsäure und der Maleinsäure sowie Vinylalkohol bzw. Vinylalkohol-Derivate oder gemäß der DE-C-42 21 381 als Monomere Salze der Acrylsäure und der 2-Alkylallylsulfonsäure sowie Zucker-Derivate enthalten. Weitere bevorzugte Copolymere sind solche, die in den deutschen Patentanmeldungen DE-A-43 03 320 und DE-A-44 17 734 beschrieben werden und als Monomere vorzugsweise Acrolein und Acrylsäure/Acrylsäuresalze bzw. Acrolein und Vinylacetat aufweisen. Ebenso sind als weitere bevorzugte Buildersubstanzen polymere Aminodicarbonsäuren, deren Salze oder deren Vorläufersubstanzen zu nennen. Besonders bevorzugt sind Polyasparaginsäuren bzw. deren Salze und Derivate, von denen in der deutschen Patentanmeldung DE-A-195 40 086 offenbart wird, daß sie neben Cobuilder-Eigenschaften auch eine bleichstabilisierende Wirkung aufweisen.

Weitere geeignete Buildersubstanzen sind Polyacetale, welche durch Umsetzung von Dialdehyden mit Polyolcarbonsäuren, welche 5 bis 7 C-Atome und mindestens 3 Hydroxylgruppen aufweisen, beispielsweise wie in der europäischen Patentanmeldung EP-A-0 280 223 beschrieben, erhalten werden können. Bevorzugte Polyacetale werden aus Dialdehyden wie Glyoxal, Glutaraldehyd, Terephthalaldehyd sowie deren Gemischen und aus Polyolcarbonsäuren wie Gluconsäure und/oder Glucoheptonsäure erhalten.

Weitere geeignete organische Buildersubstanzen sind Dextrine, beispielsweise Oligomere bzw. Polymere von Kohlenhydraten, die durch partielle Hydrolyse von Stärken erhalten werden können. Die Hydrolyse kann nach üblichen, beispielsweise säure- oder enzymkatalysierten Verfahren durchgeführt werden. Vorzugsweise handelt es sich um Hydrolyseprodukte mit mittleren Molmassen im Bereich von 400 bis 500000 g/mol. Dabei ist ein Polysaccharid mit einem Dextrose-Äquivalent (DE) im Bereich von 0,5 bis 40, insbesondere von 2 bis 30 bevorzugt, wobei DE ein gebräuchliches Maß für die reduzierende Wirkung eines Polysaccharids im Vergleich zu Dextrose, welche ein DE von 100 besitzt, ist. Brauchbar sind sowohl Maltodextrine mit einem DE zwischen 3 und 20 und Trockenglucosesirupe mit einem DE zwischen 20 und 37 als auch sogenannte Gelbdextrine und Weißdextrine mit höheren Molmassen im Bereich von 2000 bis 30000 g/mol. Ein bevorzugtes Dextrin ist in der britischen Patentanmeldung 94 19 091 beschrieben. Bei den oxidierten Derivaten derartiger Dextrine handelt es sich um deren Umsetzungsprodukte mit Oxidationsmitteln, welche in der Lage sind, mindestens eine Alkoholfunktion des Saccharidrings zur Carbonsäurefunktion zu oxidieren. Derartige oxidierte Dextrine und Verfahren ihrer Herstellung sind beispielsweise aus den europäischen Patentanmeldungen EP-A-0 232 202, EP-A-0 427 349, EP-A-0 472 042 und EP-A-0 542 496 sowie den internationalen Patentanmeldungen WO 92/18542, WO 93/08251, WO 93/16110, WO 94/28030, WO 95/07303, WO 95/12619 und WO 95/20608 bekannt. Ebenfalls geeignet ist ein oxidiertes Oligosaccharid gemäß der deutschen Patentanmeldung DE-A-196 00 018. Ein an $C_6$ des Saccharidrings oxidiertes Produkt kann besonders vorteilhaft sein.

Auch Oxydisuccinate und andere Derivate von Disuccinaten, vorzugsweise Ethylendiamindisuccinat, sind weitere geeignete Cobuilder. Dabei wird Ethylendiamin-N,N'-disuccinat (EDDS), dessen Synthese beispielsweise in US 3 158 615 beschrieben wird, bevorzugt in Form seiner Natrium- oder Magnesiumsalze verwendet. Weiterhin bevorzugt sind in diesem Zusammenhang auch Glycerindisuccinate und Glycerintrisuccinate, wie sie beispielsweise in den US-amerikanischen Patentschriften US 4 524 009, US 4 639 325, in der europäischen Patentanmeldung EP-A-0 150 930 und der japanischen Patentanmeldung JP 93/339896 beschrieben werden. Geeignete Einsatzmengen liegen in zeolithhaltigen und/oder silicathaltigen Formulierungen bei 3 bis 15 Gew.-%.

Weitere brauchbare organische Cobuilder sind beispielsweise acetylierte Hydroxycarbonsäuren bzw. deren Salze, welche gegebenenfalls auch in Lactonform vorliegen können und welche mindestens 4 Kohlenstoffatome und mindestens eine Hydroxygruppe sowie maximal zwei Säuregruppen enthalten. Derartige Cobuilder werden beispielsweise in der internationalen Patentanmeldung WO 95/20029 beschrieben.

Eine weitere Substanzklasse mit Cobuildereigenschaften stellen die Phosphonate dar. Dabei handelt es sich insbesondere um Hydroxyalkan- bzw. Aminoalkanphosphonate. Unter den Hydroxyalkanphosphonaten ist das 1-Hydroxyethan-1,1-diphosphonat (HEDP) von besonderer Bedeutung als Cobuilder. Es wird vorzugsweise als Natriumsalz eingesetzt, wobei das Dinatriumsalz neutral und das Tetranatriumsalz alkalisch (pH 9) reagiert. Als Aminoalkanphosphonate kommen vorzugsweise Ethylendiamintetramethylenphosphonat (EDTMP), Diethylentriaminpentamethylenphosphonat (DTPMP) sowie deren höhere Homologe in Frage. Sie werden vorzugsweise in Form der neutral reagierenden Natriumsalze, z. B. als Hexanatriumsalz der EDTMP bzw. als Hepta- und Octa-Natriumsalz der DTPMP, eingesetzt. Als Builder wird dabei aus der Klasse der Phosphonate bevorzugt HEDP verwendet. Die Aminoalkanphosphonate besitzen zudem ein ausgeprägtes Schwermetallbindevermögen. Dementsprechend kann es, insbesondere wenn die Mittel auch Bleiche enthalten, bevorzugt sein, Aminoalkanphosphonate, insbesondere DTPMP, einzusetzen, oder Mischungen aus den genannten Phosphonaten zu verwenden.

Darüber hinaus können alle Verbindungen, die in der Lage sind, Komplexe mit Erdalkaliionen auszubilden, als Cobuilder eingesetzt werden.

Ein bevorzugt eingesetzter anorganischer Builder ist feinkristalliner, synthetischer und gebundenes Wasser enthaltender Zeolith. Der eingesetzte feinkristalline, synthetische und gebundenes Wasser enthaltende Zeolith ist vorzugsweise Zeolith A und/oder P. Als Zeolith P wird beispielsweise Zeolith MAP z.B. Doucil A24® (Handelsprodukt der Firma Crosfield) eingesetzt. Geeignet sind jedoch auch Zeolith X sowie Mischungen aus A, X und/oder P, beispielsweise

ein Co-Kristallisat aus den Zeolithen A und X, der Vegobond® AX (Handelsprodukt der Condea Augusta S.p.A.). Der Zeolith kann als sprühgetrocknetes Pulver oder auch als ungetrocknete, von ihrer Herstellung noch feuchte, stabilisierte Suspension zum Einsatz kommen. Für den Fall, daß der Zeolith als Suspension eingesetzt wird, kann diese geringe Zusätze an nichtionischen Tensiden als Stabilisatoren enthalten, beispielsweise 1 bis 3 Gew.-%, bezogen auf Zeolith, an ethoxylierten $C_{12}$-$C_{18}$-Fettalkoholen mit 2 bis 5 Ethylenoxidgruppen, $C_{12}$-$C_{14}$-Fettalkoholen mit 4 bis 5 Ethylenoxidgruppen oder ethoxylierten Isotridecanolen. Geeignete Zeolithe weisen eine mittlere Teilchengröße von weniger als 10 µm (Volumenverteilung; Meßmethode: Coulter Counter) auf und enthalten vorzugsweise 18 bis 22 Gew.-%, insbesondere 20 bis 22 Gew.-% an gebundenem Wasser. In bevorzugten Ausführungsformen sind Zeolithe in Mengen von 10 bis 94,5 Gew.-% in dem Vorgemisch enthalten, wobei es kann besonders bevorzugt ist, wenn Zeolithe in Mengen von 20 bis 70, insbesondere 30 bis 60 Gew.-% enthalten sind.

Geeignete Teilsubstitute für Zeolithe sind Schichtsilicate natürlichen und synthetischen Ursprungs. Derartige Schichtsilicate sind beispielsweise aus den Patentanmeldungen DE-B-23 34 899, EP-A-0 026 529 und DE-A-35 26 405 bekannt. Ihre Verwendbarkeit ist nicht auf eine spezielle Zusammensetzung bzw. Strukturformel beschränkt. Bevorzugt sind hier jedoch Smectite, insbesondere Bentonite. Auch kristalline, schichtförmige Natriumsilicate der allgemeinen Formel $NaMSi_xO_{2x+1} \cdot yH_2O$, wobei M Natrium oder Wasserstoff bedeutet, x eine Zahl von 1,9 bis 4 und y eine Zahl von 0 bis 20 ist und bevorzugte Werte für x 2, 3 oder 4 sind, eigenen sich zur Substitution von Zeolithen oder Phosphaten. Derartige kristalline Schichtsilicate werden beispielsweise in der europäischen Patentanmeldung EP-A-0 164 514 beschrieben. Bevorzugte kristalline Schichtsilicate der angegebenen Formel sind solche, in denen M für Natrium steht und x die Werte 2 oder 3 annimmt. Insbesondere sind sowohl β- als auch δ-Natriumdisilicate $Na_2Si_2O_5 \cdot yH_2O$ bevorzugt.

Zu den bevorzugten Builder-Substanzen gehören auch amorphe Natriumsilicate mit einem Modul $Na_2O : SiO_2$ von 1:2 bis 1:3,3, vorzugsweise von 1:2 bis 1:2,8 und insbesondere von 1:2 bis 1:2,6, welche löseverzögert sind und Sekundärwascheigenschaften aufweisen. Die Löseverzögerung gegenüber herkömmlichen amorphen Natriumsilicaten kann dabei auf verschiedene Weise, beispielsweise durch Oberflächenbehandlung, Compoundierung, Kompaktierung/Verdichtung oder durch Übertrocknung hervorgerufen worden sein. Im Rahmen dieser Erfindung wird unter dem Begriff "amorph" auch "röntgenamorph" verstanden. Dies heißt, daß die Silicate bei Röntgenbeugungsexperimenten keine scharfen Röntgenreflexe liefern, wie sie für kristalline Substanzen typisch sind, sondern allenfalls ein oder mehrere Maxima der gestreuten Röntgenstrahlung, die eine Breite von mehreren Gradeinheiten des Beugungswinkels aufweisen. Es kann jedoch sehr wohl sogar zu besonders guten Buildereigenschaften führen, wenn die Silicatpartikel bei Elektronenbeugungsexperimenten verwaschene oder sogar scharfe Beugungsmaxima liefern. Dies ist so zu interpretieren, daß die Produkte mikrokristalline Bereiche der Größe 10 bis einige Hundert nm aufweisen, wobei Werte bis max. 50 nm und insbesondere bis max. 20 nm bevorzugt sind. Derartige sogenannte röntgenamorphe Silicate, welche ebenfalls eine Löseverzögerung gegenüber den herkömmlichen Wassergläsern aufweisen, werden beispielsweise in der deutschen Patentanmeldung DE-A- 44 00 024 beschrieben. Insbesondere bevorzugt sind verdichtete/kompaktierte amorphe Silicate, compoundierte amorphe Silicate und übertrocknete röntgenamorphe Silicate, wobei insbesondere die übertrockneten Silicate bevorzugt auch als Träger in den erfindungsgemäßen Granulaten vorkommen bzw. als Träger in dem erfindungsgemäßen Verfahren eingesetzt werden.

Selbstverständlich ist auch ein Einsatz der allgemein bekannten Phosphate als Buildersubstanzen möglich, sofern ein derartiger Einsatz nicht aus ökologischen Gründen vermieden werden sollte. Geeignet sind insbesondere die Natriumsalze der Orthophosphate, der Pyrophosphate und insbesondere der Tripolyphosphate. Ihr Gehalt im allgemeinen nicht mehr als 25 Gew.-%, vorzugsweise nicht mehr als 20 Gew.-%, jeweils bezogen auf das fertige Mittel. In einigen Fällen hat es sich gezeigt, daß insbesondere Tripolyphosphate schon in geringen Mengen bis maximal 10 Gew.-%, bezogen auf das fertige Mittel, in Kombination mit anderen Buildersubstanzen zu einer synergistischen Verbesserung des Sekundärwaschvermögens führen.

Neben den genannten Bestandteilen können die erfindungsgemäßen Wasch- und Reinigungsmittel zusätzlich einen oder mehrere Stoffe aus den Gruppen der Bleichmittel, Bleichaktivatoren, Enzyme, pH-Stellmittel, Fluoreszenzmittel, Farbstoffe, Schauminhibitoren, Silikonöle, Antiredepositionsmittel, optischen Aufheller, Vergrauungsinhibitoren, Farbübertragungsinhibitoren, Korrosionsinhibitoren und Silberschutzmittel enthalten. Diese Stoffe werden nachfolgend beschrieben.

Unter den als Bleichmittel dienenden, in Wasser $H_2O_2$ liefernden Verbindungen haben das Natriumperborattetrahydrat, das Natriumperboratmonohydrat und das Natriumpercarbonat besondere Bedeutung. Weitere brauchbare Bleichmittel sind beispielsweise Peroxypyrophosphate, Citratperhydrate sowie $H_2O_2$ liefernde persaure Salze oder Persäuren, wie Perbenzoate, Peroxophthalate, Diperazelainsäure, Phthaloiminopersäure oder Diperdodecandisäure. Auch beim Einsatz der Bleichmittel ist es möglich, auf den Einsatz von Tensiden und/oder Gerüststoffen zu verzichten, so daß reine Bleichmitteltabletten herstellbar sind. Sollen solche Bleichmitteltabletten zur Textilwäsche eingesetzt werden, ist eine Kombination von Natriumpercarbonat mit Natriumsesquicarbonat bevorzugt, unabhängig davon, welche weiteren Inhaltsstoffe in den Formkörpern enthalten sind. Werden Reinigungs- oder Bleichmitteltabletten für das maschinelle Geschirrspülen hergestellt, so können auch Bleichmittel aus der Gruppe der organischen Bleichmittel eingesetzt werden. Typische organische Bleichmittel sind die Diacylperoxide, wie z.B. Dibenzoylperoxid. Weitere typische organische Bleichmittel sind die Peroxysäuren, wobei als Beispiele besonders die Alkylperoxysäuren und die Arylperoxysäuren genannt werden. Bevorzugte Vertreter sind (a) die Peroxybenzoesäure und ihre ringsubstituierten Derivate, wie Allrylperoxybenzoesäuren, aber auch Peroxy-$\alpha$-Naphtoesäure und Magnesium-monoperphthalat, (b) die aliphatischen oder substituiert aliphatischen Peroxysäuren, wie Peroxylaurinsäure, Peroxystearinsäure, $\varepsilon$-Phthalimidoperoxycapronsäure [Phthaloiminoperoxyhexansäure (PAP)], o-Carboxybenzamidoperoxycapronsäure, N-nonenylamidoperadipinsäure und N-nonenylamidopersuccinate, und (c) aliphatische und araliphatische Peroxydicarbonsäuren, wie 1,12-Diperoxycarbonsäure, 1,9-Diperoxyazelainsäure, Diperocysebacinsäure, Diperoxybrassylsäure, die Diperoxyphthalsäuren, 2-Decyldiperoxybutan-1,4-disäure, N,N-Terephthaloyl-di(6-aminopercapronsäue) können eingesetzt werden.

Als Bleichmittel in Mitteln für das maschinelle Geschirrspülen können auch Chlor oder Brom freisetzende Substanzen eingesetzt werden. Unter den geeigneten Chlor oder Brom freisetzenden Materialien kommen beispielsweise heterocyclische N-Brom- und N-Chloramide, beispielsweise Trichlorisocyanursäure, Tribromisocyanursäure, Dibromisocyanursäure und/oder Dichlorisocyanursäure (DICA) und/oder deren Salze mit Kationen wie Kalium und Natrium in Betracht. Hydantoinverbindungen, wie 1,3-Dichlor-5,5-dimethylhydanthoin sind ebenfalls geeignet.

Um beim Waschen oder Reinigen bei Temperaturen von 60 °C und darunter eine verbesserte Bleichwirkung zu erreichen, können Bleichaktivatoren in die erfindungsgemäßen Wasch- und Reinigungsmittel eingearbeitet werden. Als Bleichaktivatoren können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. iso-NOBS), Carbonsäureanhydride, insbesondere Phthalsäureanhydrid, acylierte mehrwertige Alkohole, insbesondere Triacetin, Ethylenglykoldiacetat und 2,5-Diacetoxy-2,5-dihydrofuran.

Zusätzlich zu den konventionellen Bleichaktivatoren oder an deren Stelle können auch sogenannte Bleichkatalysatoren enthalten sein. Bei diesen Stoffen handelt es sich um bleichverstärkende Übergangsmetallsalze bzw. Übergangsmetallkomplexe wie beispielsweise Mn-, Fe-, Co-, Ru - oder Mo-Salenkomplexe oder -carbonylkomplexe. Auch Mn-, Fe-, Co-, Ru-, Mo-, Ti-, V- und Cu-Komplexe mit N-haltigen Tripod-Liganden sowie Co-, Fe-, Cu- und Ru-Amminkomplexe sind als Bleichkatalysatoren verwendbar.

Als Enzyme kommen solche aus der Klasse der Proteasen, Lipasen, Amylasen, Cellulasen bzw. deren Gemische in Frage. Besonders gut geeignet sind aus Bakterienstämmen oder Pilzen, wie Bacillus subtilis, Bacillus licheniformis und Streptomyces griseus gewonnene enzymatische Wirkstoffe. Vorzugsweise werden Proteasen vom Subtilisin-Typ und insbesondere Proteasen, die aus Bacillus lentus gewonnen werden, eingesetzt. Dabei sind Enzymmischungen, beispielsweise aus Protease und Amylase oder Protease und Lipase oder Protease und Cellulase oder aus Cellulase und Lipase oder aus Protease, Amylase und Lipase oder Protease, Lipase und Cellulase, insbesondere jedoch Cellulase-haltige Mischungen von besonderem Interesse. Auch Peroxidasen oder Oxidasen haben sich in einigen Fällen als geeignet erwiesen. Die Enzyme können an Trägerstoffen adsorbiert und/oder in Hüllsubstanzen eingebettet sein, um sie gegen vorzeitige Zersetzung zu schützen. Der Anteil der Enzyme, Enzymmischungen oder Enzymgranulate in den erfindungsgemäßen Formkörpern kann beispielsweise etwa 0,1 bis 5 Gew.-%, vorzugsweise 0,1 bis etwa 2 Gew.-% betragen. Zu den am häufigsten verwendeten Enzymen gehören Lipasen, Amylasen, Cellulasen und Proteasen. Bevorzugte Proteasen sind z. B. BLAP®140 der Fa. Biozym, Optimase®-M-440 und Opticlean®-M-250 der Fa. Solvay Enzymes; Maxacal®CX und Maxapem® oder Esperase® der Fa. Gist Brocades oder auch Savinase® der Fa. Novo. Besonders geeignete Cellulasen und Lipasen sind Celluzym® 0,7 T und Lipolase® 30 T der Fa. Novo Nordisk. Besondere Verwendung als Amylasen finden Duramyl® und Termamyl® 60 T, und Termamyl® 90 T der Fa. Novo, Amylase-LT® der Fa. Solvay Enzymes oder Maxamyl® P5000 der Fa. Gist Brocades. Auch andere Enzyme können verwendet werden.

Zusätzlich können die Wasch- und Reinigungsmittel auch Komponenten enthalten, welche die Öl- und Fettauswaschbarkeit aus Textilien positiv beeinflussen (sogenannte soil repellents). Dieser Effekt wird besonders deutlich, wenn ein Textil verschmutzt wird, das bereits vorher mehrfach mit einem erfindungsgemäßen Waschmittel, das diese öl- und fettlösende Komponente enthält, gewaschen wurde. Zu den bevorzugten öl- und fettlösenden Komponenten zählen beispielsweise nichtionische Celluloseether wie Methylcellulose und Methylhydroxy-propylcellulose mit einem Anteil an Methoxyl-Gruppen von 15 bis 30 Gew.-% und an Hydroxypropoxyl-Gruppen von 1 bis 15 Gew.-%, jeweils bezogen auf den nichtionischen Celluloseether, sowie die aus dem Stand der Technik bekannten Polymere der Phthalsäure und/oder der Terephthalsäure bzw. von deren Derivaten, insbesondere Polymere aus Ethylenterephthalaten und/oder Polyethylenglykolterephthalaten oder anionisch und/oder nichtionisch modifizierten Derivaten von diesen. Besonders bevorzugt von diesen sind die sulfonierten Derivate der Phthalsäure- und der Terephthalsäure-Polymere.

Die Mittel können als optische Aufheller Derivate der Diaminostilbendisulfonsäure bzw. deren Alkalimetallsalze enthalten. Geeignet sind z.B. Salze der 4,4'-Bis(2-anilino-4-morpholino-1,3,5-triazinyl-6-amino)stilben-2,2'-disulfonsäure oder gleichartig aufgebaute Verbindungen, die anstelle der Morpholino-Gruppe eine Diethanolaminogruppe, eine Methylaminogruppe, eine Anilinogruppe oder eine 2-Methoxyethylaminogruppe tragen. Weiterhin können Aufheller vom Typ der substituierten Diphenylstyryle anwesend sein, z.B. die Alkalisalze des 4,4'-Bis(2-sulfostyryl)-diphenyls, 4,4'-Bis(4-chlor-3-sulfostyryl)-diphenyls, oder 4-(4-Chlorstyryl)-4'-(2-sulfostyryl)-diphenyls. Auch Gemische der vorgenannten Aufheller können verwendet werden.

Um den ästhetischen Eindruck der erfindungsgemäßen Mittel zu verbessern, können sie mit geeigneten Farbstoffen eingefärbt werden. Bevorzugte Farbstoffe, deren Auswahl dem Fachmann keinerlei Schwierigkeit bereitet, besitzen eine hohe Lagerstabilität und Unempfindlichkeit gegenüber den übrigen Inhaltsstoffen der Mittel und gegen Licht sowie keine ausgeprägte Substantivität gegenüber Textilfasern, um diese nicht anzufärben.

Erfindungsgemäße Geschirrspülmittel können zum Schutze des Spülgutes oder der Maschine Korrosionsinhibitoren enthalten, wobei besonders Silberschutzmittel im Bereich des maschinellen Geschirrspülens eine besondere Bedeutung haben. Allgemein können vor allem Silberschutzmittel ausgewählt aus der Gruppe der Triazole, der Benzotriazole, der Bisbenzotriazole, der Aminotriazole, der Alkylaminotriazole und der Übergangsmetallsalze oder -komplexe eingesetzt werden. Besonders bevorzugt zu verwenden sind Benzotriazol und/oder Alkylaminotriazol. Man findet in Reinigerformulierungen darüber hinaus häufig aktivchlorhaltige Mittel, die das Korrodieren der Silberoberfläche deutlich vermindern können. In chlorfreien Reinigern werden besonders sauerstoff- und stickstoffhaltige organische redoxaktive Verbindungen, wie zwei- und dreiwertige Phenole, z.B. Hydrochinon, Brenzkatechin, Hydroxyhydrochinon, Gallussäure, Phloroglucin, Pyrogallol bzw. Derivate dieser Verbindungsklassen. Auch salz- und komplexartige anorganische Verbindungen, wie Salze der Metalle Mn, Ti, Zr, Hf, V, Co und Ce finden häufig Verwendung. Bevorzugt sind hierbei die Übergangsmetallsalze, die ausgewählt sind aus der Gruppe der Mangan und/oder Cobaltsalze und/oder -komplexe, besonders bevorzugt der Cobalt(ammin)-Komplexe, der Cobalt(acetat)-Komplexe, der Cobalt-(Carbonyl) -Komplexe, der Chloride des Cobalts oder Mangans und des Mangansulfats. Ebenfalls können Zinkverbindungen zur Verhinderung der Korrosion am Spülgut eingesetzt werden.

Besondere Inhaltsstoffe, die in erfindungsgemäßen Mitteln für das maschinelle Geschirrspülen oder die Reinigung harter Oberflächen genutzt werden können, sind Substanzen, die das Wiederanschmutzen von Oberflächen verhindern und/oder die Schmutzablösung nach einmaliger Anwendung erleichtern (sogenannte "Soil-Release-Verbindungen").

Zu den verwendbaren Soil-Release Verbindungen zählen alle im Stand der Technik bekannten Verbindungen. Besonders geeignet sind kationische Polymere, wie beispielsweise Hydroxypropyltrimethylammonium-Guar; Copolymere von Aminoethylmethacrylat und Acrylamid sowie Copolymere von Dimethyldiallylammoniumchlorid und Acrylamid, Polymere mit Imino-Gruppen, kationische Cellulosederivate, kationische Homo- und/oder Copolymere (Monomereinheiten: quaternisierte Ammoniumalkylmethacrylatgruppen).

Besonders bevorzugt sind die kationischen Polymeren ausgewählt aus kationischen Polymerisaten von Copolymeren von Monomeren wie Trialkylammoniumalkyl(meth)acrylat bzw. -acrylamid; Dialkyldiallyldiammoniumsalze; polymeranalogen Umsetzungsprodukten von Ethern oder Estern von Polysacchariden mit Ammoniumseitengruppen, insbesondere Guar-, Cellulose- und Stärkederivate; Polyaddukte von Ethylenoxid mit Ammoniumgruppen; quaternäre Ethyleniminpolymere und Polyester und Polyamide mit quaternären Seitengruppen als Soil-Release-Verbindungen. Außergewöhnlich bevorzugt im Rahmen dieser Anmeldung sind auch natürliche Polyuronsäuren und verwandte Substanzen, sowie Polyampholyte und hydrophobierte Polyampholyte, bzw. Gemische dieser Substanzen.

Diese Aufzählung von Wasch- und Reinigungsmittelinhaltsstoffen ist keineswegs abschließend, sondern gibt lediglich die wesentlichsten typischen Inhaltsstoffe derartiger Mittel wieder. Insbesondere können, soweit es sich um flüssige oder gelförmige Zubereitungen handelt, in den Mitteln auch organische Lösungsmittel enthalten sein. Vorzugsweise handelt es sich um ein- oder mehrwertige Alkohole mit 1 bis 4 C-Atomen. Bevorzugte Alkohole in solchen Mitteln sind Ethanol, 1,2-Propandiol, Glycerin sowie Gemische aus diesen Alkoholen. In bevorzugten Ausführungsformen enthalten derartige Mittel 2 bis 12 Gew.-% solcher Alkohole.

Grundsätzlich können die Mittel verschiedene Aggregatszustände aufweisen. In einer bevorzugten Ausführungsform handelt es sich bei den Wasch- oder Reinigungsmitteln um flüssige oder gelförmige Mittel, insbesondere um Flüssigwaschmittel oder flüssige Geschirrspülmittel oder Reinigungsgele, wobei es sich insbesondere auch um gelförmige Reinigungsmittel für Spültoiletten handeln kann.

Derartige gelförmige Reinigungsmittel für Spültoiletten werden beispielsweise in der deutschen Patentanmeldung DE 197 158 72 beschrieben. Es handelt sich dabei vorzugsweise um gelförmige, strukturviskose Reinigungsmittel mit einer Viskosität von 30.000 - 150.000 mPas, das als Gelbildner ein Polysaccharid, als Emulgator und netzaktive Komponente ein C8-C10 Alkylpolyglycosid oder C12-C14 Alkylpolyglycosid und Parfümöl enthalten. Als zusätzliche Co-Tenside können Fettalkoholethersulfate (FAEOS) und Fettalkoholsulfate (FAS) enthalten sein. Das Verhältnis APG zu Co-Tensid ist dann in der Regel größer als 1, vorzugsweise liegt es zwischen 50:1 und 1:1, besonders bevorzugt zwischen 10:1 und 1,5 zu 1 und ganz besonders bevorzugt zwischen 5:1 und 1,8:1. Insbesondere handelt es sich

dabei um stabile, gelförmige, scherverdünnende Reinigungsmittel enthaltend Polysaccharid, ein Tensidsystem und Parfumkomponenten, die dadurch gekennzeichnet sind, daß

- ein Polysaccharid, bevorzugt ein Xanthan-Gum, in Mengen zwischen 1 und 5 Gew.-% bevorzugt 1 bis 4 Gew.-%, besonders bevorzugt 1,5 bis 3,5 Gew.-% und ganz besonders bevorzugt 1,8 bis 3 Gew.-%,
- als eine Komponente des Tensidsystems ein C8 bis C22 Alkylpolyglycosid in Mengen zwischen 3 und 25 Gew.-% bevorzugt 4 und 20 Gew.-% besonders bevorzugt 5 und 15 Gew.-% und ganz besonders bevorzugt 5 und 12 Gew.-% und
- die Parfumkomponente oder die Parfumkomponenten bis 15 Gew.-% bevorzugt in 2 bis 12 Gew.-% besonders bevorzugt in 3 bis 8 Gew.-%
- sowie ggf. weitere Inhaltsstoffe wie kalklösende Mittel, Farbstoffe, keimhemmende Mittel, Perlglanzmittel, Stabilisatoren Reinigungsverstärker und Geruchsabsorber enthalten sind
- und die Mittel eine Viskosität von 30.000 bis 150.000 mPas aufweisen, gemessen mit einem Brookfield Rotationsviskosimeter, Typ RVT mit Helipath-Einrichtung und der Spindel TA bei 1 U/min und 23 °C.

Derartige Reinigungsgele werden üblicherweise in Behältern, die in einer WC-Schüssel oder in Wasserkästen angebracht werden können, dosiert. Ein spezielles Behältnis, das für die gelförmigen Reinigungsmittel besonders geeignet ist, wird in der deutschen Patentanmeldung DE 195 201 45 beschrieben.

Es hat sich gezeigt, daß mit Polysacchariden in den beschriebenen Kombinationen je nach Wahl der Type mit hohen Parfüm- und APG-Konzentrationen optisch ansprechende transluzente oder klare strukturviskose Gelstrukturen erzielt werden können, die in den dafür geeigneten Behältnissen vergleichbare Haltbarkeiten von festen WC-Blöken erreichen.

Andere übliche Gelbildner wie z.B. Polyacrylsäure (Carbopol), tensidverdickte Systeme, MHPC (Natrosol) oder kochsalz-, bzw. elektrolytverdickte Tensidsysteme zeigen bei Einsatz der notwendigen hohen Tensid- und Parfümanteile nicht diese hohe Gelstabilität und sich daher weniger bevorzugt. Diese Formulierungen sind häufig nicht ausreichend strukturviskos, verdünnen sich beim Überströmen mit Wasser und tropfen wegen ihres ungenügenden Viskositätsverhalten trotz geeigneter Behältnisse undosiert in die WC-Schüssel. Dagegen sind die bevorzugten Formulierungen ausgesprochen strukturviskos und widerstehen dabei dem überströmenden Wasser in der Form, daß nur geringe Teilmengen abgegeben werden und die gewünschte Haltbarkeit erzielt wird. Die erhaltenen Mittel dürfen sich in dem in die dafür vorgesehenen Behältnissen eindringenden Wasser auch nicht zu gut lösen, damit sie nicht nach einer geringen Anzahl von verwendungsgemäßen wäßrigen Überströmungen bereits aufgelöst und damit verbraucht sind.

Insbesondere ist es dabei bevorzugt, daß unter bestimmten Bedingungen beim Produktionsprozeß Luftblasen in die Mittel eingebracht werden, die über einen Zeitraum von mehreren Wochen in Größe und Form stabil sind und damit ein für den Verbraucher optisch noch ansprechenderes Produkt bedeuten.

Dabei darf die Größe der Luftblasen, die sich z. B. über die Rührgeschwindigkeit im Produktionsprozeß und die Viskosität der Mittel steuern läßt weder zu groß noch zu klein sein, sowie die Menge der Luftblasen ebenfalls nur in einem bevorzugten Bereich ausgewählt sein. Sollte also die Anwesenheit von Luftblasen erwünscht sein, so sollte nicht mehr als 30 Vol-% Luft enthalten sein, bevorzugt wischen 2 und 25 Vol-% Luft und ganz besonders bevorzugt zwischen 5 und 20 Vol.-% Luft. Die ganz besonders bevorzugten Ausführungsformen enthalten Luftblasen zwischen 0,1 mm und 20 mm Durchmesser, äußerst bevorzugt zwischen 1 mm und 15 mm Durchmesser.

Die Viskosität der bevorzugten Mittel erlaubt aber auch, die im Produktionsprozeß bereits eingetragenen Luftblasen durch kurzzeitiges Anlegen eines Unterdrucks, der in einem Bereich knapp unterhalb Raumdruck bis nahe einem Vakuum liegen kann. Dabei ist die Dauer der Unterdruckbehandlung abhängig von der Stärke des Unterdrucks. Bei stärkerem Druck muß die Behandlung nicht so lange durchgeführt werden. Der Fachmann weiß aber auch, daß bei zu starkem Unterdruck unerwünschte Nebeneffekte auftreten können, wie z. B. das verstärkte Abdampfen von leichterflüchtigen Parfumkomponenten und u. U. Probleme bei der Rührbarkeit des Systems. Ein Entgasen der erfindungsgemäßen Mittel durch die Behandlung in einer Zentrifuge oder durch ultraschnelles Rühren ist zwar möglich, aber weniger bevorzugt.

Bei der Herstellung der Rezepturen, die in den unterschiedlichsten Ansatzgrößen bis zum Tonnenmaßstab durchführbar ist, kann der Fachmann auf unterschiedliche Weise vorgehen.

Üblicherweise wird Wasser in einer handelsüblichen Mischeranlage, wie z. B. einer Beco-Mix-Anlage vorgelegt, und der Farbstoff eingerührt. Der verwendete Xanthan Gum wird mit Lösungsmittel, bevorzugt Ethanol und dem gewünschten Parfumöl separat aufgeschlämmt. Die Suspension wird der Vorlage zugegeben und mit geringen Geschwindigkeiten, beispielsweise 30 U/min gerührt.

Es zeigt sich bei den Untersuchungen, daß nach Zugabe aller Komponente eine Zeit zwischen wenigen Minuten und einigen Stunden zur Erreichung der erfindungsgemäßen Konsistenz wünschenswert ist. Im vorliegenden Fall wurde nach 30 min das Tensid (Alkylpolyglycosid) langsam zudosiert. Anschließend werden die weiteren Komponente zugesetzt.

Soll ein blasenfreies Gel gewährleistet werden, ist die Mischung, wie oben bereits beschrieben in einem geeigneten Behälter in Abhängigkeit von der Viskosität in der Regel aber für eine kurze Zeit, beispielsweise 15 min unter reduzierten Druck oder ein Vakuum zu setzen.

Der Fachmann kann aber auch anders vorgehen. Dies empfiehlt sich z. B. bei der Einarbeitung von Desinfektionsmitteln. Üblicherweise wird hier Wasser in einer handelsüblichen Mischeranlage, wie z. B. einer Beco-Mix-Anlage vorgelegt, und der verwendete Xanthan Gum eingerührt . Die Suspension wird der Vorlage zugegeben und mit geringen Geschwindigkeiten, beispielsweise 30 U/min gerührt, bevor nach 30 min das Tensidgemisch (Alkylpolyglycosid/Fettalkoholethersulfat) langsam zudosiert wird. Anschließend wird der Farbstoff zugegeben, bevor eine ethanolische Lösung des Parfums nachdosiert wurde.

Danach erfolgt die Zugabe des Desinfektionsmittels, bevorzugt ausgewählt aus der Gruppe der Isothiazoline, der Benzoate oder der Salicylsäure oder Salicylate.

Die Abfüllung kann in diesem Fall z. B. über einen Flaschenrundläufer in eine handelsübliche Dosierflasche erfolgen.

Besondere Vorsicht muß man walten lassen, wenn man Substanzen in das vorbereitete und gequollene wäßrige Xanthan-Gel zugibt, damit sich die erfindungsgemäße Struktur bilden kann. Bei einer zu schnellen Zugabe kann es zu Phasentrennungsproblemen kommen.

Ebenfalls darf bei der Herstellung der Xanthan-Gel-Komponente kein Tensid zugegen sein, da es die Gel-Bildung verhindern könnte. Daher ist es äußerst bevorzugt die tensidische Komponente erst nach der Bildung des Gels zuzugeben.

Zur Messung der Viskosität können die gängigen Methoden der Viskositätsbestimmung verwendet werden. Im vorliegenden Fall wurde mit Brookfield-Viskosimetern gearbeitet, welche eine für Gele vorgesehene Spindel bereithalten. Mit dieser Helipath-Spindel wurden die erfindungsgemäßen Viskositäten gemessen.

Die bevorzugten Gel-Formulierungen können in einer Rahmenrezeptur folgende Komponenten enthalten:

1,0 - 5,0 Gew.-% Polysaccharid
3,0 - 25,0 Gew.-% C8-C22 Alkylpolyglykosid
0 - 15,0 Gew.-% Co-Tenside (FAS, FAEOS)
0- 5,0 Gew.-% Zitronensäure
0 - 5,0 Gew.-% Komplexbildner
bis 15 Gew.-% bevorzugt 2,0 - 12,0 Gew.-% Parfüm
bis 5,0 Gew.-% bevorzugt 0,01 bis 4 Gew.-% Lösungsmittel, wie z.B. Ethanol
0 - 1,0 Gew.-% Konservierungsmittel
0 - 10,0 Gew.-% Farbstoff
0 - 5,0 Gew.-% bevorzugt 0,01 bis 3 Gew.-% keimhemmende Mittel

Dabei versteht man beispielsweise erfindungsgemäß unter einem Polysaccharid z. B. ein Xanthan Gum oder ein Guar Gum oder Gemische aus Polysacchariden.

Xanthan wird aus einer Kette mit β-1,4-gebundener Glucose (Cellulose) mit Seitenketten gebildet. Die Struktur der Untergruppen besteht aus Glucose, Mannose, Glucuronsäure, Acetat und Pyruvat. Xanthan wird von Xanthomonas campestris unter aeroben Bedingungen mit einem Molekulargewicht von 2-15 X $10^6$ produziert. Xanthan wird u.a. in Batch-Kulturen hergestellt und nach Abtöten der Kultur und Fällen mit Propanol getrocknet und gemahlen. Andere geeignete Verfahren werden in der Literatur ebenfalls beschrieben.

Alkylpolyglycoside sind die bereits weiter oben erwähnten Tenside, die durch die Reaktion von Zuckern und Alkoholen nach den einschlägigen Verfahren der präparativen organischen Chemie erhalten werden können, wobei es je nach Art der Herstellung zu einem Gemisch monoalkylierter, oligomerer oder polymerer Zucker kommt. Bevorzugte Alkylpolyglykoside können Alkylpolyglucoside sein, wobei besonders bevorzugt der Alkohol ein langkettiger Fettalkohole ist mit Alkylkettenlängen zwischen C8 und C22 bevorzugt zwischen C8 und C16 und besonders bevorzugt zwischen C8 und C12 liegt oder ein Gemisch langkettiger Fettalkohole ist. Der Oligomerisierungsgrad der Zucker, der eine berechnete und damit in der Regel nicht ganzzahlige Größe ist, liegt zwischen 1 und 10, bevorzugt zwischen 1,1 und 5 und ganz besonders bevorzugt zwischen 1,2 und 3 und äußerst bevorzugt zwischen 1,3 und 2,5.

Anionische Co-Tenside gemäß der vorliegenden Erfindung können aliphatische Sulfate wie Fettalkoholsulfate, Fettalkoholethersulfate, Dialkylethersulfate, Monoglyceridsulfate und aliphatische Sulfonate wie Alkansulfonate, Olefinsulfonate, Ethersulfonate, n-Alkylethersulfonate, Estersulfonate, und Lingninsulfonate sein. Ebenfalls im Rahmen der vorliegenden Erfindung verwendbar, aber nicht bevorzugt sind Fettsäurecyanamide, Sulfobernsteinsäureester, Fettsäureisethionate, Acylaminoalkansulfonate (Fettsäuretauride), Fettsäuresarcosinate, Ethercarbonsäuren und Alkyl(ether)phosphate. Bevorzugt werden Fettalkoholsulfate und Fettalkoholethersulfate verwendet. Weniger gute Ergebnisse wurden bisher mit Alkylbenzolsulfonaten erzielt.

Aber auch nichtionische Co-Tenside können Verwendung finden. Nichtionische Tenside im Rahmen der vorliegenden Erfindung können alkoxylierte Alkohole sein, wie Polyglycolether, Fettalkoholpolygycolether, Alkylphenolpolyglycolether, endgruppenverschlossene Polyglycolether, Mischether und Hydroxymischether und Fettsäurepolyglycolester sein. Ebenfalls verwendbar sind Ethylenoxid, Propylenoxid, Blockpolymere und Fettsäurealkanolamide und Fettsäurepolyglycolether.

Dabei versteht der Fachmann allgemein unter alkoxylierten Alkoholen die Reaktionsprodukte von Alkylenoxid, bevorzugt Ethylenoxid, mit Alkoholen, bevorzugt längerkettigen Alkoholen. In der Regel entstehen aus n Molen Ethylenoxid und einem Mol Alkohol, abhängig von den Reaktionsbedingungen ein komplexes Gemisch von Additionsprodukten unterschiedlichen Ethoxylierungsgrades. Eine weitere Ausführungsform besteht im Einsatz von Gemischen der Alkylenoxide bevorzugt des Gemisches von Ethylenoxid und Propylenoxid. Auch kann man gewünschtenfalls durch eine abschließende Veretherung mit kurzkettigen Alkylgruppen, wie bevorzugt der Butylgruppe, zur Substanzklasse der "verschlossenen" Alkoholethoxylaten gelangen, die ebenfalls im Sinne der Erfindung eingesetzt werden kann. Ganz besonders bevorzugt im Sinne der vorliegenden Erfindung sind dabei hochethoxylierte Fettalkohole oder deren Gemische mit endgruppenverschlossenen Fettalkoholethoxylaten.

Die Formulierungen können vorzugsweise kalklösende Säuren wie die Zitronensäure, Essigsäure, Milchsäure oder deren wasserlöslichen Salze in einer Menge von 1 - 12 Gew.-% enthalten. Besonders bevorzugt sind Gehalte von 2 - 5 Gew.-%.

Die Gele enthalten vorzugsweise Farbstoff, entweder für die Farbgebung des Produktes oder für die Farbgebung der den Behälter umspielenden Flüssigkeit. Bevorzugt liegt der Gehalt an wasserlöslichen Farbstoffen < 1 Gew.-% und dient zur Verbesserung der Optik des Produktes. Wenn ein zusätzliches Farbsignal beim Einspülvorgang gewünscht ist, kann der Gehalt an wasserlöslichen Farbstoffen bis 5 Gew.-% betragen.

Obwohl die Gele bereits ohne diese Komponente bereits eine ausgezeichnete Reinigungswirkung besitzen, kann die hygienische Wirkung durch den Zusatz keimhemmender Mittel verstärkt werden. Die Menge dieser Mittel hängt stark von der Wirksamkeit der jeweiligen Verbindung ab und kann bis zu 5 Gew.-% betragen. Vorzugsweise werden mehr als 0,01 Gew.-% in die Gele eingearbeitet. Besonders bevorzugt ist der Bereich zwischen 0,01 Gew.-% und 3 Gew.-%. Geeignet sind insbesondere Isothiazolingemische, Natriumbenzoat oder Salicylsäure.

Zu den Parfümölen die in bevorzugten Gelen in Mengen von bis zu 15 Gew.-% und insbesondere bevorzugt von 2 bis 12 Gew.-% und ganz besonders bevorzugt von 3 bis 8 Gew.-% enthalten sein können, zählen die bereits weiter oben beschriebenen Verbindungen. Erfindungsgemäß enthalten diese Parfümöle die beschriebenen Kieselsäureester gemäß Formeln (I) und/oder (Ia). Dabei kann das Parfümöl vollständig aus erfindungsgemäßen Kieselsäureestern bestehen oder aber Kieselsäureester in Mischungen mit anderen Duftstoffen aufweisen.

Als Lösungsvermittler, etwa für Farbstoffe und Parfümöle können beispielsweise Alkanolamine, Polyole wie Ethylenglycol, Propylenglycol, 1,2 Glycerin und andere ein- und mehrwertige Alkohole, sowie Alkylbenzolsulfonate mit 1 bis 3 Kohlenstoffatomen im Alkylrest dienen. Besonders bevorzugt ist dabei die Gruppe der niederen Alkohole, ganz besonders Ethanol.

Zu den üblichen Verdickungsmitteln, die bei Bedarf zusätzlich ebenfalls einsetzbar wären, zählen Harnstoff, Natriumchlorid, Natriumsulfat, Magnesiumsulfat, Ammoniumchlorid und Magnesiumchlorid sowie die Kombination dieser Verdickungsmittel. Der Einsatz dieser zusätzlichen Verdickungsmittel ist jedoch nicht bevorzugt.

In den erfindungsgemäßen Gelen können ggf. wasserlösliche und wasserunlösliche Builder enthalten sein. Dabei sind dann wasserlösliche Builder bevorzugt, da sie auf harten Oberflächen in der Regel weniger dazu tendieren unlösliche Rückstände zu bilden. Übliche Builder, die im Rahmen der Erfindung zugegen sein können, sind die niedermolekularen Polycarbonsäuren und ihre Salze, die homopolymeren und copolymeren Polycarbonsäuren und ihre Salze, die Citronensäure und ihre Salze, die Carbonate, Phosphate und Silikate. Zu wasserunlöslichen Buildem zählen die Zeolithe, die ebenfalls verwendet werden können, ebenso wie Mischungen der vorgenannten Buildersubstanzen. Besonders bevorzugt ist die Gruppe der Citrate.

Weitere typische Reinigungsmittel, die die erfindungsgemäßen und/oder erfindungsgemäß zu verwendenden Kieselsäureester enthalten können, sind flüssige oder gelförmige Reiniger für harte Oberflächen, insbesondere sogenannte Allzweckreiniger, Glasreiniger, Boden- oder Badezimmerreiniger sowie spezielle Ausführungsformen derartiger Reiniger wozu saure oder alkalische Formen von Allzweckreinigern ebenso wie Glasreiniger mit sogenannter Anti-Rain-Wirkung gehören. Dabei können diese flüssigen Reinigungsmittel sowohl in einer als auch in mehreren Phasen vorliegen. In einer insbesondere bevorzugten Ausführungsform weisen die Reiniger 2 verschiedene Phasen auf.

Reiniger ist dabei im weitesten Sinne eine Bezeichnung für - zumeist Tensid-haltige - Formulierungen mit sehr weitem Einsatzbereich und davon abhängig sehr unterschiedlicher Zusammensetzung. Die wichtigsten Marktsegmente sind Haushalts-Reiniger, industrielle (technische) und institutionelle Reiniger. Nach dem pH-Wert unterscheidet man alkalische, neutrale und saure Reiniger, nach der Angebotsform flüssige und feste Reiniger (auch in Tablettenform). Die sogenannten Reiniger für harte Oberflächen sollen im Unterschied etwa zu Geschirrspülmitteln, die ebenfalls mit in die Produktgruppe der Reiniger eingeordnet werden, sowohl im konzentrierten Zustand als auch in verdünnter wäßriger Lösung. in Verbindung mit mechanischer Energie ein optimales Anwendungsprofil zeigen. Kältreiniger entfalten ihre Leistung ohne erhöhte Temperatur. Maßgebend für die Reinigungswirkung sind v.a. Tenside und/oder Alkaliträger, alternativ Säuren, ggf. auch Lösungsmittel wie Glykolether und niedere Alkohole. Im allgemeinen

sind in den Formulierungen darüber hinaus Builder, je nach Reiniger-Typ auch Bleichmittel, Enzyme, keimmindernde oder desinfizierende Zusätze sowie Parfümöle und Farbstoffe enthalten. Reiniger können auch als Mikroemulsionen formuliert sein. Der Reinigungserfolg hängt in hohem Maße von der - auch geographisch sehr unterschiedlichen - Schmutzart und den Eigenschaften der zu reinigenden Oberflächen ab.

Haushalts-Reiniger: können als Universal-Reiniger oder als Spezial-Reiniger für u. a. Keramik, Fliesen, Fenster, Kunststoffe, (Teppich-)Böden, Kochfelder, Backöfen, Mikrowellenherde, als Sanitär-Reiniger oder WC-Reiniger formuliert werden. Rohr-Reiniger sind alkalisch eingestellt und bestehen z. B. aus festem Natriumhydroxid und Aluminium-Pulver, bei dessen Auflösung der entstehende Wasserstoff für eine entsprechende Verwirbelung in den freizuspülenden Rohrsegmenten sorgt. Sanitär-Reiniger enthalten neben Tensid und Builder v. a. keimmindemde Wirkstoffe, wobei das früher verwendete Natriumhypochlorit teilweise durch Wasserstoffperoxid oder andere Persauerstoff-Verbindungen ersetzt ist. WC-Reiniger sind überwiegend sauer, manchmal auch alkalisch eingestellt, wobei im ersteren Fall die ursprünglich verwendete Phosphorsäure und das Natriumhydrogensulfat weitgehend durch organische Säuren, v. a. Citronensäure, ersetzt sind. Zu den Spezial-Reinigern. gehören im Do-it-yourself-Bereich auch Kfz-, Autoscheiben-, Felgen-, Motoren- und Farbauftragsgeräte-Reiniger.

Institutionelle Reiniger: dienen der betrieblichen Reinigung und Hygiene z. B. in Schulen, Bürogebäuden, Hotels, Gaststätten und Krankenhäusern, wobei im letzteren Fall eine sichere Flächendesinfektion eine besondere Anforderung an die Produkte stellt. Diese Reiniger werden in Großgebinden abgegeben (Großverbraucherware). Die Produkte und die zugehörige Dienstleistung unter Einsatz speziell entwickelter Reinigungsgeräte werden als Systemlösung angeboten.

Technische Reiniger: Werden v. a. in der Getränke-, Nahrungsgüter-, kosmetischen und pharmazeutischen Industrie, aber auch in der Metall-Industrie zum Metallentfetten eingesetzt. Die Produktgruppe umfaßt u. a. auch Reiniger für Kfz-Waschanlagen, Tankwagen- und Flugzeug-Reiniger. Im Hinblick auf die erforderliche Produktivität z. B. bei der Flaschenreinigung müssen diese Reiniger mit schaumarmen Tensiden formuliert sein, wofür sich spezielle nichtionische Tenside, wie Ethylenoxid-Propylenoxid-Blockcopolymere und sogenannte endgruppenverschlossene Alkylethoxylate, eignen.

Ein bevorzugter mehrphasiger Allzweckreiniger ist dabei ein wäßriges flüssiges mehrphasiges tensidhaltiges Reinigungsmittel mit wenigstens zwei kontinuierlichen Phasen, das mindestens eine untere wäßrige Phase I sowie eine mit dieser Phase nicht mischbare obere wäßrige Phase II aufweist und sich durch Schütteln temporär in eine Emulsion überführen läßt, und das 0 bis 5 Gew.-% Natriumhexametaphosphat enthält. Unter Natriumhexametaphosphat ist dabei eine Mischung kondensierter Orthophosphate der Formel I zu verstehen, wobei n für einen Mittelwert von etwa 12 steht.

Ein derartiger Reiniger ist in der älteren deutschen Patentanmeldung DE 198 113 86 beschrieben. Erfindungsgemäß enthält diese Reiniger-Duftstoffe, wobei mindestens ein Teil der enthaltenen Parfümöle in Form der erfindungsgemäßen und/oder erfindungsgemäß zu verwendenden Kieselsäureester vorliegt.

Im einfachsten Fall besteht ein derartiges Mittel aus einer unteren kontinuierlichen Phase, die aus der gesamten Phase I besteht, und einer oberen kontinuierlichen Phase, die aus der gesamten Phase II besteht. Eine oder mehrere kontinuierliche Phasen des Mittels können jedoch auch Teile einer anderen Phase in emulgierter Form enthalten, so daß in einem solchen Mittel beispielsweise Phase I zu einem Teil als kontinuierliche Phase I vorliegt, die die untere kontinuierliche Phase des Mittels darstellt, und zu einem anderen Teil als diskontinuierliche Phase I in der oberen kontinuierlichen Phase II emulgiert ist. Für Phase II und weitere kontinuierliche Phasen gilt analoges.

Unter temporär ist dabei zu verstehen, daß 90 % der Entmischung der durch Schütteln gebildeten Emulsion in die getrennten Phasen bei Temperaturen von etwa 20 °C bis ca. 40 °C innerhalb von 2 Minuten bis 10 Stunden erfolgt und die letzten 2 % der Entmischung in den Phasenzustand vor dem Schütteln innerhalb von weiteren 15 Minuten bis 50 Stunden erfolgen.

Derartige Mittel zeichnen sich durch eine ungewöhnlich gute Reinigungsleistung an hartnäckigem Fettschmutz bei unverdünnter Anwendung aus. Darüber hinaus zeigen die Mittel ein günstiges Rückstandsverhalten. Die einzelnen Phasen im Mittel sind über lange Zeit stabil, ohne daß sich z.B. Ablagerungen bildeten, und die Überführung in eine temporäre Emulsion bleibt auch nach häufigem Schütteln reversibel. Zudem kann die Trennung von Inhaltsstoffen in separate Phasen die chemische Stabilität des Mittels fördern.

In einer bevorzugten Ausführungsform sind die kontinuierlichen Phasen I und II durch eine scharfe Grenzfläche gegeneinander abgegrenzt.

In einer weiteren bevorzugten Ausführungsform enthalten eine oder beide der kontinuierlichen Phasen I und II Teile, vorzugsweise 0,1 bis 25 Vol.-%, insbesondere 0,2 bis 15 Vol.-%, bezogen auf das Volumen der jeweiligen kontinuierlichen Phase, der jeweils anderen Phase als Dispergens. Dabei ist dann die kontinuierliche Phase I bzw. II um den Volumenteil verringert, der als Dispergens in der jeweils anderen Phase verteilt ist. Besonders bevorzugt sind hierbei Mittel, in denen Phase I in Mengen von 0,1 bis 25 Vol.-%, bevorzugt 0,2 bis 15 Vol.-%, bezogen auf das Volumen der Phasen, in Phase II emulgiert ist.

In einer weiterhin bevorzugten Ausführungsform liegt neben den kontinuierlichen Phasen I und II ein Teil der

beiden Phasen als Emulsion einer der beiden Phasen in der anderen Phase vor, wobei diese Emulsion durch zwei scharfe Grenzflächen, eine obere und eine untere, gegenüber den nicht an der Emulsion beteiligten Teilen der Phasen I und II abgegrenzt ist.

Dabei enthalten die Mittel in einer besonders vorteilhaften Ausführungsform eine oder mehrere hydrophobe Komponenten. Geeignete Hydrophobkomponenten sind beispielsweise Dialkylether mit gleichen oder verschiedenen $C_4$- bis $C_{14}$-Alkylresten, insbesondere Dioctylether; Kohlenwasserstoffe mit einem Siedebereich von 100 bis 300 °C, insbesondere 140 bis 280 °C, z.B. aliphatische Kohlenwasserstoffe mit einem Siedebereich von 145 bis 200 °C, Isoparaffine mit einem Siedebereich von 200 bis 260 °C; etherische Öle, insbesondere Limonen und das aus Kiefernwurzeln und -stubben extrahierte Pine Oil; und auch Mischungen dieser Hydrophobkomponenten, insbesondere Mischungen von zwei oder drei der genannten Hydrophobkomponenten. Bevorzugte Gemische von Hydrophobkomponenten sind Gemische von verschiedenen Dialkylethern, von Dialkylethern und Kohlenwasserstoffen, von Dialkylethern und etherischen Ölen, von Kohlenwasserstoffen und etherischen Ölen, von Dialkylethern und Kohlenwasserstoffen und etherischen Ölen und von diesen Gemischen. Die Mittel enthalten Hydrophobkomponenten in Mengen, bezogen auf die Zusammensetzung, von 0 bis 20 Gew.-%, vorzugsweise 0,1 bis 14 Gew.-%, insbesondere 0,5 bis 10 Gew.-%, äußerst bevorzugt 0,8 bis 7 Gew.-%.

Die Mittel können darüberhinaus Phasentrennhilfsmittel enthalten. Geeignete Phasentrennhilfsmittel sind beispielsweise die Alkalimetall- und Erdalkalimetallchloride und -sulfate, insbesondere Natrium- und Kaliumchlorid und -sulfat, sowie Ammoniumchlorid und -sulfat bzw. deren Mischungen. Die genannten Salze unterstützen als starke Elektrolyte die Phasentrennung durch den Salzeffekt. Auch Buildersalze bewirken als Elektrolyte diesen Effekt und eignen sich dementsprechend ebenfalls als Phasentrennhilfsmittel. Die Mittel enthalten Phasentrennhilfsmittel in Mengen, bezogen auf die Zusammensetzung, von 0 bis 30 Gew.-%, vorzugsweise 1 bis 20 Gew.-%, insbesondere 3 bis 15 Gew.-%, äußerst bevorzugt 5 bis 12 Gew.-%.

Die Mittel können als Tensidkomponente anionische, nichtionische, amphotere oder kationische Tenside bzw. Tensidgemische aus einer, mehreren oder allen diesen Tensidklassen enthalten. Die Mittel enthalten Tenside in Mengen, bezogen auf die Zusammensetzung, von 0,01 bis 30 Gew.-%, vorzugsweise 0,1 bis 20 Gew.-%, insbesondere 1 bis 14 Gew.-%, äußerst bevorzugt 3 bis 10 Gew.-%.

Geeignete Niotenside in derartigen Allzweckreinigern sind beispielsweise $C_8$-$C_{18}$-Alkylalkoholpolyglykolether, Alkylpolyglykoside sowie stickstoffhaltige Tenside bzw. Mischungen davon, insbesondere der ersten beiden. Die Mittel enthalten nichtionische Tenside in Mengen, bezogen auf die Zusammensetzung, von 0 bis 30 Gew.-%, vorzugsweise 0,1 bis 20 Gew.-%, insbesondere 0,5 bis 14 Gew.-%, äußerst bevorzugt 1 bis 10 Gew.-%.

$C_8$-$C_{18}$-Alkylalkoholpolypropylenglykol/polyethylenglykolether stellen bekannte nichtionische Tenside dar. Sie können durch die Formel RO-$(CH_2CH(CH_3)O)_p(CH_2CH_2O)_e$-H, beschrieben werden, in der $R^i$ für einen linearen oder verzweigten, aliphatischen Alkyl- und/oder Alkenylrest mit 8 bis 18 Kohlenstoffatomen, *p* für 0 oder Zahlen von 1 bis 3 und *e* für Zahlen von 1 bis 20 steht. Die $C_8$-$C_{18}$-Alkylalkoholpolyglykolether kann man durch Anlagerung von Propylenoxid und/oder Ethylenoxid an Alkylalkohole, vorzugsweise an Fettalkohole, erhalten. Typische Beispiele sind Polyglykolether in der $R^i$ für einen Alkylrest mit 8 bis 18 Kohlenstoffatomen, *p* für 0 bis 2 und *e* für Zahlen von 2 bis 7 steht. Bevorzugte Vertreter sind beispielsweise $C_{10}$-$C_{14}$-Fettalkohol+1PO+6EO-ether (*p* = 1, *e* = 6) und $C_{12}$-$C_{18}$-Fettalkohol+7EO-ether *(p* = 0, *e* = 7) sowie deren Mischungen.

Es können auch endgruppenverschlossene $C_8$-$C_{18}$-Alkylalkoholpolyglykolether eingesetzt werden, d.h. Verbindungen in denen die freie OH-Gruppe in der Formel II verethert ist. Die endgruppenverschlossenen $C_8$-$C_{18}$-Alkylalkoholpolyglykolether können nach einschlägigen Methoden der präparativen organischen Chemie erhalten werden. Vorzugsweise werden $C_8$-$C_{18}$-Alkylalkohopolyglykolether in Gegenwart von Basen mit Alkylhalogeniden, insbesondere Butyl- oder Benzylchlorid, umgesetzt. Typische Beispiele sind Mischether bei denen $R^i$ für einen technischen Fettalkoholrest, vorzugsweise $C_{12/14}$-Kokosalkylrest, *p* für 0 und *e* für 5 bis 10 stehen, die mit einer Butylgruppe verschlossen sind.

Bevorzugte nichtionische Tenside sind weiterhin die bereits weiter oben beschriebenen Alkylpolyglykoside.

Als weitere nichtionische Tenside können stickstoffenthaltende Tenside enthalten sein, z.B. Fettsäurepolyhydroxyamide, beispielsweise Glucamide, und Ethoxylate von Alkylaminen, vicinalen Diolen und/oder Carbonsäureamiden, die Alkylgruppen mit 10 bis 22 C-Atomen, vorzugsweise 12 bis 18 C-Atomen, besitzen. Der Ethoxylierungsgrad dieser Verbindungen liegt dabei in der Regel zwischen 1 und 20, vorzugsweise zwischen 3 und 10. Bevorzugt sind Ethanolamid-Derivate von Alkansäuren mit 8 bis 22 C-Atomen, vorzugsweise 12 bis 16 C-Atomen. Zu den besonders geeigneten Verbindungen gehören die Laurinsäure-, Myristinsäure- und Palmitinsäuremonoethanolamide.

Für Allzweckreiniger geeignete Aniontenside sind $C_8$-$C_{18}$-Alkylsulfate, $C_8$-$C_{18}$-Alkylethersulfate, d.h. die Sulfatierungsprodukte der Alkoholether der Formel II,und/oder $C_8$-$C_{18}$-Alkylbenzolsulfonate, aber auch $C_8$-$C_{18}$-Alkansulfonate, $C_8$-$C_{18}$-$\alpha$-Olefinsulfonate, sulfonierte $C_8$-$C_{18}$-Fettsäuren, insbesondere Dodecylbenzolsulfonat, $C_8$-$C_{22}$-Carbonsäureamidethersulfate, Sulfonbernsteinsäuremono- und -di-$C_1$-$C_{12}$-Alkylester, $C_8$-$C_{18}$-Alkylpolyglykolethercarboxylate, $C_8$-$C_{18}$-N-Acyltauride, $C_8$-$C_{18}$-N-Sarkosinate und $C_8$-$C_{18}$-Alkylisethionate bzw. deren Mischungen. Sie werden in Form ihrer Alkalimetall- und Erdalkalimetallsalze, insbesondere Natrium-, Kalium- und Magnesiumsalze, wie auch Ammonium- und Mono-, Di-, Tri- bzw. Tetraalkylammoniumsalze sowie im Falle der Sulfonate auch in Form ihrer korrespondierende Säure, z.B. Dodecylbenzolsulfonsäure, eingesetzt. Die Mittel enthalten anionische Tenside in Mengen, bezogen auf die Zusammensetzung, von 0 bis 30 Gew.-%, vorzugsweise 0,1 bis 20 Gew.-%, insbesondere 1 bis 14 Gew.-%, äußerst bevorzugt 2 bis 10 Gew.-%.

Wegen ihrer schaumdämpfenden Eigenschaften können die Allzweckreiniger auch Seifen, d.h. Alkali- oder Ammoniumsalze gesättigter oder ungesättigter $C_6$-$C_{22}$-Fettsäuren, enthalten. Die Seifen können in einer Menge bis zu 5 Gew.-%, vorzugsweise von 0,1 bis 2 Gew.-%, eingesetzt werden.

Geeignete Amphotenside sind beispielsweise Betaine der Formel $(R^{ii})(R^{iii})(R^{iv})N^+CH_2COO^-$, in der $R^{ii}$ einen gegebenenfalls durch Heteroatome oder Heteroatomgruppen unterbrochenen Alkylrest mit 8 bis 25, vorzugsweise 10 bis 21 Kohlenstoffatomen und $R^{iii}$ sowie $R^{iv}$ gleichartige oder verschiedene Alkylreste mit 1 bis 3 Kohlenstoffatomen bedeuten, insbesondere $C_{10}$-$C_{18}$-Alkyl-dimethylcarboxymethylbetain und $C_{11}$-$C_{17}$-Alkylamidopropyl-dimethylcarboxymethylbetain. Die Mittel enthalten amphotere Tenside in Mengen, bezogen auf die Zusammensetzung, von 0 bis 15 Gew.-%, vorzugsweise 0,01 bis 10 Gew.-%, insbesondere 0,1 bis 5 Gew.-%.

Geeignete Kationtenside sind u.a. die quartären Ammoniumverbindungen der Formel $(R^v)(R^{vi})(R^{vii})(R^{viii})N^+X^-$, in der $R^v$ bis $R^{viii}$ für vier gleich- oder verschiedenartige, insbesondere zwei lang- und zwei kurzkettige, Alkylreste und $X^-$ für ein Anion, insbesondere ein Halogenidion, stehen, beispielsweise Didecyl-dimethyl-ammoniumchlorid, Alkylbenzyl-didecyl-ammoniumchlorid und deren Mischungen. Die Mittel enthalten kationische Tenside in Mengen, bezogen auf die Zusammensetzung, von 0 bis 10 Gew.-%, vorzugsweise 0,01 bis 5 Gew.-%, insbesondere 0,1 bis 3 Gew.-%.

In einer bevorzugten Ausführungsform enthalten die Reiniger anionische und nichtionische Tenside nebeneinander, vorzugsweise $C_8$-$C_{18}$-Alkylbenzolsulfonate, $C_8$-$C_{18}$-Alkylsulfate und/oder $C_8$-$C_{18}$-Alkylethersulfate neben $C_8$-$C_{18}$-Alkylalkoholpolyglykolethern und/oder Alkylpolyglykosiden, insbesondere $C_8$-$C_{18}$-Alkylbenzolsulfonate neben $C_8$-$C_{18}$-Alkylalkoholpolyglykolethern.

Weiterhin können die erfindungsgemäßen Mittel Builder enthalten. Geeignete Builder sind beispielsweise Alkalimetallgluconate, -citrate, -nitrilotriacetate, -carbonate und - bicarbonate, insbesondere Natriumgluconat, -citrat und -nitrilotriacetat sowie Natriumund Kaliumcarbonat und -bicarbonat, sowie Alkalimetall- und Erdalkalimetallhydroxide, insbesondere Natrium- und Kaliumhydroxid, Ammoniak und Amine, insbesondere Mono- und Triethanolamin, bzw. deren Mischungen. Hierzu zählen auch die Salze der Glutarsäure, Bernsteinsäure, Adipinsäure, Weinsäure und Benzolhexacarbonsäure sowie Phosphonate und Phosphate. Die Mittel enthalten Builder in Mengen, bezogen auf die Zusammensetzung, von 0 bis 20 Gew.-%, vorzugsweise 0,01 bis 12 Gew.-%, insbesondere 0,1 bis 8 Gew.-%, äußerst bevorzugt 0,3 bis 5 Gew.-%, wobei jedoch die Menge an Natriumhexametaphosphat — ausgenommen die verwendungsgemäßen Mittel — auf 0 bis 5 Gew.-% beschränkt ist. Als Elektrolyte sind die Buildersalze zugleich Phasentrennhilfsmittel.

Neben den genannten Komponenten können die erfindungsgemäßen Mittel weitere Hilfsund Zusatzstoffe enthalten, wie sie in derartigen Mitteln üblich sind. Hierzu zählen insbesondere Polymere, Soil-Release-Wirkstoffe, Lösungsmittel (z.B. Ethanol, Isopropanol, Glykolether), Lösungsvermittler, Hydrotrope (z.B. Cumolsulfonat, Octylsulfat, Butylglucosid, Butylglykol), Reinigungsverstärker, Viskositätsregler (z.B. synthetische Polymere wie Polysaccharide, Polyacrylate, in der Natur vorkommenden Polymere und deren Derivate wie Xanthangum, weitere Polysaccharide und/oder Gelatine), pH-Regulatoren (z.B. Citronensäure, Alkanolamine oder NaOH), Desinfektionsmittel, Antistatika, Konservierungsmittel, Bleichsysteme, Enzyme, Farbstoffe sowie Trübungsmittel oder auch Hautschutzmittel, wie sie in EP-A-522 556 beschrieben sind. Die Menge an derartigen Zusätzen liegt üblicherweise nicht über 12 Gew.-% im Reinigungsmittel. Die Untergrenze des Einsatzes hängt von der Art des Zusatzstoffes ab und kann beispielsweise bei Farbstoffen bis zu 0,001 Gew.-% und darunter betragen. Vorzugsweise liegt die Menge an Hilfsstoffen zwischen 0,01 und 7 Gew.-%, insbesondere 0,1 und 4 Gew.-%.

Der pH-Wert der Allzweckreiniger kann über einen weiten Bereich variiert werden, bevorzugt ist jedoch ein Bereich von 2,5 bis 12, insbesondere 5 bis 10,5. Unter dem pH-Wert ist dabei der vorliegenden Erfindung der pH-Wert des Mittels in Form der temporären Emulsion zu verstehen.

Derartige Allzweckreiniger-Rezepturen lassen sich für beliebige Zwecke modifizieren. Eine besondere Ausführungsform sind dabei die Glasreiniger. Wesentlich bei derartigen Reinigern ist, daß Flecken oder Ränder zurückbleiben. Insbesondere ist hierbei ein Problem, daß nach dem Reinigen Wasser auf diesen Oberflächen kondensiert und zu dem sogenannten Beschlageffekt führt. Ebenso ist es unerwünscht, wenn auf Glasscheiben die dem Regen ausgesetzt sind, sogenannte Regenflecken zurückbleiben. Dieser Effekt ist als Regeneffekt oder Anti-Rain-Effekt bekannt. Diesen Effekten kann durch geeignete Additive in Glasreinigern vorgebeugt werden.

Aus der WO 96/04358 sind Reinigungsmittel bekannt, die Glas reinigen können, ohne in einem störenden Ausmaß Flecken und/oder Filme zu hinterlassen, und eine wirksame Menge eines substantiven Polymers mit hydrophilen Gruppen enthalten, das das Glas mit einer lang anhaltenden höheren Hydrophilie versieht, so daß wenigstens bei den nächsten drei erneuten Benetzungen, beispielsweise durch Regen, das Wasser flächig abläuft und nach dem Trocknen weniger Flecken zurückbleiben. Substantive Polymere sind insbesondere Polycarboxylate wie Poly(vinylpyrrolidon-co-acrylsäure), aber auch Poly(styrolsulfonat), kationische Zucker- und Stärkederivate sowie aus Ethylenoxid und Propylenoxid aufgebaute Blockcopolymere, wobei gerade letztere Polyether weniger Substantivität besitzen.

Aus der WO 94/22800 sind epoxyverschlossene polyalkoxylierte Alkohole der Formel A bekannt,

$$R^{I}O[CH_2CH(CH)_3O]_x[CH_2CH_2O]_y[CH_2CH(R^{II})O]_zH \quad (A)$$

in der $R^I$ einen linearen, aliphatischen Kohlenwasserstoffrest mit etwa 4 bis etwa 18 Kohlenstoffatomen oder ein Gemisch verschiedener solcher Reste,
$R^{II}$ einen linearen, aliphatischen Kohlenwasserstoffrest mit etwa 2 bis etwa 26 Kohlenstoffatomen oder ein Gemisch verschiedener solcher Reste,
x eine Zahl von 1 bis etwa 3,
y eine Zahl von 5 bis etwa 30 und
z eine Zahl von 1 bis etwa 3

repräsentiert. Die Alkohole der Formel A können in pulverförmige und flüssige maschinelle Geschirrspülmittel oder Reinigungsmittel für harte Oberflächen eingearbeitet werden. In maschinellen Geschirrspülmitteln bewirken sie eine Verringerung der Fleckund Filmbildung.

Aus der WO 96/12001 sind epoxyverschlossene polyalkoxylierte Alkohole der Formel B bekannt,

$$R^{III}O[CH_2CH(CH)_3O]_u[CH_2CH(R^{IV})O]_v[CH_2CH(R^{V})O]_wH \quad (B)$$

in der $R^{III}$ einen linearen, aliphatischen Kohlenwasserstoffrest mit etwa 4 bis etwa 18 Kohlenstoffatomen oder ein Gemisch verschiedener solcher Reste,
$R^{IV}$ ein Wasserstoffatom oder einen niederen Alkylrest mit 1 bis 6 Kohlenstoffatomen,
$R^V$ einen linearen, aliphatischen Kohlenwasserstoffrest mit etwa 2 bis etwa 14 Kohlenstoffatomen oder ein Gemisch verschiedener solcher Reste,
u eine Zahl von 1 bis etwa 5,
v eine Zahl von 1 bis etwa 30 und
w eine Zahl von 1 bis etwa 3

repräsentiert. Die Alkohole der Formel B können allein oder zusammen mit Alkoholen der Formel A in pulverförmige und flüssige maschinelle Geschirrspülmittel oder Reinigungsmittel für harte Oberflächen wie Badezimmerkacheln eingearbeitet werden. Auch in maschinellen Geschirrspülmitteln bewirken sie eine Verringerung der Fleck- und Filmbildung.

In einer bevorzugten Ausführungsform werden die in der Patentanmeldung DE 198 565 29 beschriebenen endgruppenverschlossenen polyalkoxylierten Alkoholen der Formel I,

$$R^1O[CH_2CH(CH)_3O]_p[CH_2CH(R^2)O]_qR^3 \quad (I)$$

in der $R^1$ einen linearen, aliphatischen Kohlenwasserstoffrest mit 1 bis etwa 22 Kohlenstoffatomen oder ein Gemisch verschiedener solcher Reste,
$R^2$ ein Wasserstoffatom oder einen niederen Alkylrest mit 1 bis 6 Kohlenstoffatomen,
$R^3$ einen linearen oder verzweigten, gesättigten oder ungesättigten, aliphatischen, ggf. arylsubstitutierten, acyclischen oder cyclischen, Kohlenwasserstoffrest mit 1 bis etwa 78 Kohlenstoffatomen und optional ein oder mehreren Hydroxygruppen und/oder Ethergruppen -O- oder ein Gemisch verschiedener solcher Reste,
p eine Zahl von 0 bis etwa 15 und
q eine Zahl von 0 bis etwa 50

repräsentiert und die Summe von p und q mindestens 1 ist, in einem Reinigungsmittel für harte Oberflächen zur Verringerung des Regeneffekts und/oder des Beschlageffekts, eingesetzt.

Der Gehalt an einem oder mehreren endgruppenverschlossenen polyalkoxylierten Alkoholen der Formel I in dem Glasreiniger beträgt 0,001 bis 20 Gew.-%, vorzugsweise 0,01 bis 10 Gew.-%, insbesondere 0,05 bis 5 Gew.-%, besonders bevorzugt 0,1 bis 2,5 Gew.-% und äußerst bevorzugt 0,2 bis 2,0 Gew.-%.

Beispielhafte endgruppenverschlossene polyalkoxylierte Alkohole sind solche der Formel I, in der (a) $R^1$ = $C_{12-18}$- oder $C_{12/14}$-Fettalkylrest, $R^2$ = H, $R^3$ = Butylrest, p = 0, q = 10, (b) $R^1$ = $C_{12-18}$-Fettalkylrest, $R^2$ = H, $R^3$ =

Butylrest, p = 0, q = 5, (c) $R^1$ = $CH_3$, $R^2$ = H, $R^3$ = $C_{12/14}$-Fettalkylrest, p = 3, q = 5 oder (d) $R^1$ = $C_8$-Fettalkylrest, $R^2$ = H, $R^3$ = Butylrest, p = 0, q = 5.

Bevorzugte endgruppenverschlossene polyalkoxylierte Alkohole sind solche der Formel I, bei denen p und q beide mindestens 1 betragen und/oder der Rest $R^2$ ein Wasserstoffatom ist und/oder der Rest $R^3$ mindestens eine Hydroxygruppe, insbesondere in ω-Position, d.h. $R^3$ eine Gruppe -$CH_2CH(OH)$-R darstellt, enthält. Endgruppenverschlossene polyalkoxylierte Alkohole der Formel I, bei denen der Rest $R^3$ eine Gruppe - $CH_2CH(OH)$-R darstellt, sind beispielsweise aus DE 37 23 323 A1 bekannt.

Besonders bevorzugte endgruppenverschlossene polyalkoxylierte Alkohole sind epoxyverschlossene polyalkoxylierte Alkohole der Formel I, in der $R^1$ einen linearen, aliphatischen Kohlenwasserstoffrest mit etwa 4 bis etwa 18, vorzugsweise etwa 4 bis etwa 12, Kohlenstoffatomen, insbesondere einen Butyl-, Hexyl-, Octyl- oder Decylrest bzw. deren Mischungen, oder ein Gemisch verschiedener solcher Reste, $R^2$ ein Wasserstoffatom oder einen niederen Alkylrest mit 1 bis 6 Kohlenstoffatomen, vorzugsweise ein Wasserstoffatom, $R^3$ eine Gruppe $[CH_2CH(R^4)O]_rH$, in der $R^4$ für einen linearen, aliphatischen Kohlenwasserstoffrest mit etwa 2 bis etwa 26, vorzugsweise etwa 4 bis etwa 18, insbesondere etwa 6 bis etwa 14, Kohlenstoffatomen oder ein Gemisch verschiedener solcher Reste und r für eine Zahl von 1 bis etwa 3, vorzugsweise 1 bis etwa 2, insbesondere 1, steht, p eine Zahl von 1 bis etwa 5, vorzugsweise 1 bis etwa 2, insbesondere 1, und q eine Zahl von 1 bis etwa 30, vorzugsweise etwa 4 bis etwa 26, insbesondere etwa 10 bis etwa 24, repräsentiert, beispielsweise mit $R^1$ = $C_{8/10}$-Alkylrest, $R^2$ = H, $R^3$ = $[CH_2CH(R^4)O]_rH$ mit $R^4$ = $C_8$-Alkylrest und r = 1, u = 1 und v = 22.

Solche epoxyverschlossene polyalkoxylierten Alkohole und Methoden zur ihrer Herstellung sind beispielsweise aus WO 94/22800 und WO 96/12001 bekannt. Bevorzugte endgruppenverschlossene Alkohole sind beispielsweise unter den Handelsnamen Dehypon® von der Fa. Henkel KGaA oder unter dem Handelsnamen Poly Tergent® von der Fa. Olin Corporation erhältlich, beispielsweise Dehypon® LT 104, Dehypon® LS 104, Dehypon® LT 54, Dehypon® LS 531 oder Dehypon® O 54 bzw. Poly Tergent® SLF 18 B 48, Poly Tergent® SLF 18 B 45 oder Poly Tergent® SL 62.

In einer anderen bevorzugten Ausführungsform handelt es sich bei den Mitteln um pulverförmige oder granulatförmige Mittel. Die erfindungsgemäßen Mittel können dabei beliebige Schüttgewichte aufweisen. Die Palette der möglichen Schüttgewichte reicht von niedrigen Schüttgewichten unter 600 g/l, beispielsweise 300 g/l, über den Bereich mittlerer Schüttgewichte von 600 bis 750 g/l bis zum Bereich hoher Schüttgewichte von mindestens 750 g/l. In einer bevorzugten Variante der erfindungsgemäßen Mittel mit hohen Schüttgewichten liegt das Schüttgewicht jedoch sogar oberhalb von 800 g/l, wobei Schüttgewichte oberhalb 850 g/l besonders vorteilhaft sein können. Bei derartigen Superkompaktaten kommen die Vorteile des löslichen Buildersystems besonders zum Tragen, da derartige kompakte Mittel besondere Anforderungen an die Inhaltsstoffe stellen, um gut dispergierbar zu sein. Daneben führt das niederdosierte Buildersystem unabhängig vom Schüttgewicht zu zusätzlichen Vorteilen bei der Einsparung von Verpackungsvolumen und reduziert den Chemikalieneintrag pro Waschgang in die Umwelt.

Zur Herstellung solcher Mittel sind beliebige, aus dem Stand der Technik bekannte Verfahren, geeignet.

Bevorzugt werden die Mittel dadurch hergestellt, daß verschiedene teilchenförmige Komponenten, die Wasch- und/oder Reinigungsmittelinhaltsstoffe enthalten und zusammen mindestens 60 Gew.-% des gesamten Mittels bilden, miteinander vermischt werden.

Dabei können die teilchenförmigen Komponenten durch Sprühtrocknung, einfaches Mischen oder komplexe Granulationsverfahren, beispielsweise Wirbelschichtgranulation, hergestellt werden. Bevorzugt ist dabei insbesondere, daß mindestens eine tensidhaltige Komponente durch Wirbelschichtgranulation hergestellt wird.

Weiter kann es insbesondere bevorzugt sein, wenn wäßrige Zubereitungen von Buildersubstanzen gemeinsam mit anderen Wasch- und/oder Reinigungsmittelinhaltsstoffen in einer Trockeneinrichtung versprüht werden, wobei gleichzeitig mit der Trocknung eine Granulation stattfinden kann. Bei der Trockeneinrichtung, in die, die wäßrige Zubereitung versprüht wird, kann es sich um beliebige Trockenapparaturen handeln.

In einer bevorzugten Verfahrensführung wird die Trocknung als Sprühtrocknung in einem Trockenturm durchgeführt. Dabei werden die wäßrigen Zubereitungen in bekannter Weise einem Trocknungsgasstrom in feinverteilter Form ausgesetzt. Die Anmelderin beschreibt eine Ausführungsform der Sprühtrocknung mit überhitztem Wasserdampf in einer Reihe veröffentlichter Druckschriften. Das dort offenbarte Arbeitsprinzip wird hiermit ausdrücklich auch zum Gegenstand der vorliegenden Erfindungsoffenbarung gemacht. Verwiesen wird hier insbesondere auf die nachfolgenden Druckschriften: DE-A-40 30 688 sowie die weiterführenden Veröffentlichungen gemäß DE-A-42 04 035; DE-A-42 04 090; DE-A-42 06 050; DE-A-42 06 521; DE-A-42 06 495; DE-A-42 08 773; DE-A-42 09 432 und DE-A-42 34 376.

In einer anderen, insbesondere wenn Mittel hoher Schüttdichte erhalten werden sollen, bevorzugten Variante werden die Gemische anschließend einem Kompaktierungsschritt unterworfen, wobei weitere Inhaltsstoffe den Mitteln erst nach dem Kompaktierungsschritt zugemischt werden.

Die Kompaktierung der Inhaltsstoffe findet in einer bevorzugten Ausführungsform der Erfindung in einem Preßagglomerationsverfahren statt. Der Preßagglomerationsvorgang, dem das feste Vorgemisch unterworfen wird, kann dabei in verschiedenen Apparaten realisiert werden. Je nach dem Typ des verwendeten Agglomerators werden

unterschiedliche Preßagglomerationsverfahren unterschieden. Die vier häufigsten und im Rahmen der vorliegenden Erfindung bevorzugten Preßagglomerationsverfahren sind dabei die Extrusion, das Walzenpressen bzw. -kompaktieren, das Lochpressen (Pelletieren) und das Tablettieren, so daß im Rahmen der vorliegenden Erfindung bevorzugte Preßagglomerationsvorgänge Extrusions-, Walzenkompaktierungs-, Pelletierungs- oder Tablettierungsvorgänge sind.

Allen Verfahren ist gemeinsam, daß das Vorgemisch unter Druck verdichtet und plastifiziert wird und die einzelnen Partikel unter Verringerung der Porosität aneinandergedrückt werden und aneinander haften. Bei allen Verfahren (bei der Tablettierung mit Einschränkungen) lassen sich die Werkzeuge dabei auf höhere Temperaturen aufheizen oder zur Abführung der durch Scherkräfte entstehenden Wärme kühlen.

In allen Verfahren kann als Hilfsmittel zur Verdichtung ein Bindemittel eingesetzt werden. Im weiteren Verlauf der Beschreibung dieser Erfindung wird einfachheitshalber nur noch von einem oder dem Bindemittel die Rede sein. Dabei soll jedoch klargestellt sein, daß an sich immer auch der Einsatz von mehreren, verschiedenen Bindemitteln und Mischungen aus verschiedenen Bindemitteln möglich ist. In einer bevorzugten Ausführungsform der Erfindung wird ein Bindemittel eingesetzt, daß bei Temperaturen bis maximal 130°C, vorzugsweise bis maximal 100 °C und insbesondere bis 90 °C bereits vollständig als Schmelze vorliegt. Das Bindemittel muß also je nach Verfahren und Verfahrensbedingungen ausgewählt werden oder die Verfahrensbedingungen, insbesondere die Verfahrenstemperatur, müssen - falls ein bestimmtes Bindemittel gewünscht wird - an das Bindemittel angepaßt werden.

Der eigentliche Verdichtungsprozeß erfolgt dabei vorzugsweise bei Verarbeitungstemperaturen, die zumindest im Verdichtungsschritt mindestens der Temperatur des Erweichungspunkts, wenn nicht sogar der Temperatur des Schmelzpunkts des Bindemittels entsprechen. In einer bevorzugten Ausführungsform der Erfindung liegt die Verfahrenstemperatur signifikant über dem Schmelzpunkt bzw. oberhalb der Temperatur, bei der das Bindemittel als Schmelze vorliegt. Insbesondere ist es aber bevorzugt, daß die Verfahrenstemperatur im Verdichtungsschritt nicht mehr als 20°C über der Schmelztemperatur bzw. der oberen Grenze des Schmelzbereichs des Bindemittels liegt. Zwar ist es technisch durchaus möglich, auch noch höhere Temperaturen einzustellen; es hat sich aber gezeigt, daß eine Temperaturdifferenz zur Schmelztemperatur bzw. zur Erweichungstemperatur des Bindemittels von 20°C im allgemeinen durchaus ausreichend ist und noch höhere Temperaturen keine zusätzlichen Vorteile bewirken. Deshalb ist es - insbesondere auch aus energetischen Gründen - besonders bevorzugt, zwar oberhalb, jedoch so nah wie möglich am Schmelzpunkt bzw. an der oberen Temperaturgrenze des Schmelzbereichs des Bindemittels zu arbeiten. Eine derartige Temperaturführung besitzt den weiteren Vorteil, daß auch thermisch empfindliche Rohstoffe, beispielsweise Peroxybleichmittel wie Perborat und/oder Percarbonat, aber auch Enzyme, zunehmend ohne gravierende Aktivsubstanzverluste verarbeitet werden können. Die Möglichkeit der genauen Temperatursteuerung des Binders insbesondere im entscheidenden Schritt der Verdichtung, also zwischen der Vermischung/Homogenisierung des Vorgemisches und der Formgebung, erlaubt eine energetisch sehr günstige und für die temperaturempfindlichen Bestandteile des Vorgemisches extrem schonende Verfahrensführung, da das Vorgemisch nur für kurze Zeit den höheren Temperaturen ausgesetzt ist. In bevorzugten Preßagglomerationsverfahren weisen die Arbeitswerkzeuge des Preßagglomerators (die Schnecke(n) des Extruders, die Walze(n) des Walzenkompaktors sowie die Preßwalze(n) der Pelletpresse) eine Temperatur von maximal 150°C, vorzugsweise maximal 100°C uns insbesondere maximal 75°C auf und die Verfahrenstemperatur liegt 30°C und insbesondere maximal 20°C oberhalb der Schmelztemperatur bzw. der oberen Temperaturgrenze des Schmelzbereichs des Bindemittels. Vorzugsweise beträgt die Dauer der Temperatureinwirkung im Kompressionsbereich der Preßagglomeratoren maximal 2 Minuten und liegt insbesondere in einem Bereich zwischen 30 Sekunden und 1 Minute.

Bevorzugte Bindemittel, die allein oder in Mischung mit anderen Bindemitteln eingesetzt werden können, sind Polyethylenglykole, 1,2-Polypropylenglykole sowie modifizierte Polyethylenglykole und Polypropylenglykole. Zu den modifizierten Polyalkylenglykolen zählen insbesondere die Sulfate und/oder die Disulfate von Polyethylenglykolen oder Polypropylenglykolen mit einer relativen Molekülmasse zwischen 600 und 12000 und insbesondere zwischen 1000 und 4000. Eine weitere Gruppe besteht aus Mono- und/oder Disuccinaten der Polyalkylenglykole, welche wiederum relative Molekülmassen zwischen 600 und 6000, vorzugsweise zwischen 1000 und 4000 aufweisen. Für eine genauere Beschreibung der modifizierten Polyalkylenglykolether wird auf die Offenbarung der internationalen Patentanmeldung WO-A-93/02176 verwiesen. Im Rahmen dieser Erfindung zählen zu Polyethylenglykolen solche Polymere, bei deren Herstellung neben Ethylenglykol ebenso $C_3$-$C_5$-Glykole sowie Glycerin und Mischungen aus diesen als Startmoleküle eingesetzt werden. Ferner werden auch ethoxylierte Derivate wie Trimethylolpropan mit 5 bis 30 EO umfaßt. Die vorzugsweise eingesetzten Polyethylenglykole können eine lineare oder verzweigte Struktur aufweisen, wobei insbesondere lineare Polyethylenglykole bevorzugt sind. Zu den insbesondere bevorzugten Polyethylenglykolen gehören solche mit relativen Molekülmassen zwischen 2000 und 12000, vorteilhafterweise um 4000, wobei Polyethylen-glykole mit relativen Molekülmassen unterhalb 3500 und oberhalb 5000 insbesondere in Kombination mit Polyethylenglykolen mit einer relativen Molekülmasse um 4000 eingesetzt werden können und derartige Kombinationen vorteilhafterweise zu mehr als 50 Gew.-%, bezogen auf die gesamte Menge der Polyethylenglykole, Polyethylenglykole mit einer relativen Molekülmasse zwischen 3500 und 5000 aufweisen. Als Bindemittel können jedoch auch Polyethylenglykole eingesetzt werden, welche an sich bei Raumtemperatur und einem Druck von 1 bar in flüssigem Stand vorliegen; hier ist vor allem

von Polyethylenglykol mit einer relativen Molekülmasse von 200, 400 und 600 die Rede. Allerdings sollten diese an sich flüssigen Polyethylenglykole nur in einer Mischung mit mindestens einem weiteren Bindemittel eingesetzt werden, wobei diese Mischung wieder den erfindungsgemäßen Anforderungen genügen muß, also einen Schmelzpunkt bzw. Erweichungspunkt von mindestens oberhalb 45 °C aufweisen muß.

Ebenso eignen sich als Bindemittel niedermolekulare Polyvinylpyrrolidone und Derivate von diesen mit relativen Molekülmassen bis maximal 30000. Bevorzugt sind hierbei relative Molekülmassenbereiche zwischen 3000 und 30000, beispielsweise um 10000. Polyvinylpyrrolidone werden vorzugsweise nicht als alleinige Bindemittel, sondern in Kombination mit anderen, insbesondere in Kombination mit Polyethylenglykolen, eingesetzt.

Als geeignete weitere Bindemittel haben sich Rohstoffe erwiesen, welche Rohstoffe wasch- oder reinigungsaktiven Eigenschaften aufweisen, also beispielsweise nichtionische Tenside mit Schmelzpunkten von mindestens 45 °C oder Mischungen aus nichtionischen Tensiden und anderen Bindemitteln. Zu den bevorzugten nichtionischen Tensiden gehören alkoxylierte Fett- oder Oxoalkohole, insbesondere $C_{12}$-$C_{18}$-Alkohole. Dabei haben sich Alkoxylierungsgrade, insbesondere Ethoxylierungsgrade von durchschnittlich 18 bis 80 AO, insbesondere EO pro Mol Alkohol und Mischungen aus diesen als besonders vorteilhaft erwiesen. Vor allem Fettalkohole mit durchschnittlich 18 bis 35 EO, insbesondere mit durchschnittlich 20 bis 25 EO, zeigen vorteilhafte Bindereigenschaften im Sinne der vorliegenden Erfindung. Gegebenenfalls können in Bindemittelmischungen auch ethoxylierte Alkohole mit durchschnittlich weniger EO-Einheiten pro Mol Alkohol enthalten sein, beispielsweise Talgfettalkohol mit 14 EO. Allerdings ist es bevorzugt, diese relativ niedrig ethoxylierten Alkohole nur in Mischung mit höher ethoxylierten Alkoholen einzusetzen. Vorteilhafterweise beträgt der Gehalt der Bindemittel an diesen relativ niedrig ethoxylierten Alkoholen weniger als 50 Gew.-%, insbesondere weniger als 40 Gew.-%, bezogen auf die Gesamtmenge an eingesetztem Bindemittel. Vor allem üblicherweise in Wasch- oder Reinigungsmitteln eingesetzte nichtionische Tenside wie $C_{12}$-$C_{18}$-Alkohole mit durchschnittlich 3 bis 7 EO, welche bei Raumtemperatur an sich flüssig vorliegen, sind vorzugsweise in den Bindemittelmischungen nur in den Mengen vorhanden, daß dadurch weniger als 2 Gew.-% dieser nichtionischen Tenside, bezogen auf das Verfahrensendprodukt, bereitgestellt werden. Wie bereits oben beschrieben ist es allerdings weniger bevorzugt, in den Bindemittelmischungen bei Raumtemperatur flüssige nichtionische Tenside einzusetzen. In einer besonders vorteilhaften Ausführungsform sind derartige nichtionische Tenside aber kein Bestandteil der Bindemittelmischung, da diese nicht nur den Erweichungspunkt der Mischung herabsetzen, sondern auch zur Klebrigkeit des Endprodukts beitragen können und außerdem durch ihre Neigung, beim Kontakt mit Wasser zu Vergelungen zu führen, auch dem Erfordernis der schnellen Auflösung des Bindemittels/der Trennwand im Endprodukt nicht im gewünschten Umfang genügen. Ebenso ist es nicht bevorzugt, daß übliche in Wasch- oder Reinigungsmitteln eingesetzte Aniontenside oder deren Vorstufen, die Aniontensidsäuren, in der Bindemittelmischung enthalten sind. Andere nichtionische Tenside, die als Bindemittel geeignet sind, stellen die nicht zu Vergelungen neigenden Fettsäuremethylesterethoxylate, insbesondere solche mit durchschnittlich 10 bis 25 EO dar (genauere Beschreibung dieser Stoffgruppe siehe unten). Besonders bevorzugte Vertreter dieser Stoffgruppe sind überwiegend auf $C_{16}$-$C_{18}$-Fettsäuren basierende Methylester, beispielsweise gehärteter Rindertalgmethylester mit durchschnittlich 12 EO oder mit durchschnittlich 20 EO. In einer bevorzugten Ausführungsform der Erfindung wird als Bindemittel eine Mischung eingesetzt, welche $C_{12}$-$C_{18}$-Fettalkohol auf Basis Kokos oder Talg mit durchschnittlich 20 EO und Polyethylenglykol mit einer relativen Molekülmasse von 400 bis 4000 eingesetzt. In einer weiteren bevorzugten Ausführungsform der Erfindung wird als Bindemittel eine Mischung eingesetzt, welche überwiegend auf $C_{16}$-$C_{18}$-Fettsäuren basierende Methylester mit durchschnittlich 10 bis 25 EO, insbesondere gehärteten Rindertalgmethylester mit durchschnittlich 12 EO oder durchschnittlich 20 EO, und einem $C_{12}$-$C_{18}$-Fettalkohol auf Basis Kokos oder Talg mit durchschnittlich 20 EO und/oder Polyethylenglykol mit einer relativen Molekülmasse von 400 bis 4000 enthält.

Als besonders vorteilhafte Ausführungsformen der Erfindung haben sich Bindemittel erwiesen, die entweder allein auf Polyethylenglykolen mit einer relativen Molekülmasse um 4000 oder auf einer Mischung aus $C_{12}$-$C_{18}$-Fettalkohol auf Basis Kokos oder Talg mit durchschnittlich 20 EO und einem der oben beschriebenen Fettsäuremethylesterethoxylate oder auf einer Mischung aus $C_{12}$-$C_{18}$-Fettalkohol auf Basis Kokos oder Talg mit durchschnittlich 20 EO, einem der oben beschriebenen Fettsäuremethylesterethoxylate und einem Polyethylenglykol, insbesondere mit einer relativen Molekülmasse um 4000, basieren.

In geringen Mengen können neben den hier genannten Substanzen noch weitere geeignete Substanzen in dem Bindemittel enthalten sein.

Das verdichtete Gut weist direkt nach dem Austritt aus dem Herstellungsapparat vorzugsweise Temperaturen nicht oberhalb von 90°C auf, wobei Temperaturen zwischen 35 und 85°C besonders bevorzugt sind. Es hat sich herausgestellt, daß Austrittstemperaturen - vor allem im Extrusionsverfahren - von 40 bis 80°C, beispielsweise bis 70°C, besonders vorteilhaft sind.

In einer bevorzugten Ausführungsform der Erfindung wird das erfindungsgemäße Verfahren mittels einer Extrusion durchgeführt, wie sie beispielsweise in dem europäischen Patent EP-B-486592 oder den internationalen Patentanmeldungen WO 93/02176 und WO 94/09111 bzw. WO 98/12299 beschrieben werden. Dabei wird ein festes Vorgemisch unter Druck strangförmig verpreßt und der Strang nach Austritt aus der Lochform mittels einer Schneidevorrichtung auf die vorbestimmbare Granulatdimension zugeschnitten. Das homogene und feste Vorgemisch enthält ein Plastifizier- und/oder Gleitmittel, welches bewirkt, daß das Vorgemisch unter dem Druck bzw. unter dem Eintrag spezifischer Arbeit plastisch erweicht und extrudierbar wird. Bevorzugte Plastifizier- und/oder Gleitmittel sind Tenside und/oder Polymere.

Zur Erläuterung des eigentlichen Extrusionsverfahrens wird hiermit ausdrücklich auf die obengenannten Patente und Patentanmeldungen verwiesen. In einer bevorzugten Ausführungsform der Erfindung wird dabei das Vorgemisch vorzugsweise kontinuierlich einem Planetwalzenextruder oder einem 2-Wellen-Extruder bzw. 2-Schnecken-Extruder mit gleichlaufender oder gegenlaufender Schneckenführung zugeführt, dessen Gehäuse und dessen Extruder-Granulierkopf auf die vorbestimmte Extrudiertemperatur aufgeheizt sein können. Unter der Schereinwirkung der Extruderschnecken wird das Vorgemisch unter Druck, der vorzugsweise mindestens 25 bar beträgt, bei extrem hohen Durchsätzen in Abhängigkeit von dem eingesetzten Apparat aber auch darunter liegen kann, verdichtet, plastifiziert, in Form feiner Stränge durch die Lochdüsenplatte im Extruderkopf extrudiert und schließlich das Extrudat mittels eines rotierenden Abschlagmessers vorzugsweise zu etwa kugelförmigen bis zylindrischen Granulatkömern verkleinert. Der Lochdurchmesser der Lochdüsenplatte und die Strangschnittlänge werden dabei auf die gewählte Granulatdimension abgestimmt. In dieser Ausführungsform gelingt die Herstellung von Granulaten einer im wesentlichen gleichmäßig vorherbestimmbaren Teilchengröße, wobei im einzelnen die absoluten Teilchengrößen dem beabsichtigten Einsatzzweck angepaßt sein können. Im allgemeinen werden Teilchendurchmesser bis höchstens 0,8 cm bevorzugt. Wichtige Ausführungsformen sehen hier die Herstellung von einheitlichen Granulaten im Millimeterbereich, beispielsweise im Bereich von 0,5 bis 5 mm und insbesondere im Bereich von etwa 0,8 bis 3 mm vor. Das Länge/Durchmesser-Verhältnis der abgeschlagenen primären Granulate liegt dabei in einer wichtigen Ausführungsform im Bereich von etwa 1:1 bis etwa 3:1. Weiterhin ist es bevorzugt, das noch plastische Primärgranulat einem weiteren formgebenden Verarbeitungsschritt zuzuführen; dabei werden am Rohextrudat vorliegende Kanten abgerundet, so daß letztlich kugelförmig bis annähernd kugelförmige Extrudatkömer erhalten werden können. Falls gewünscht können in dieser Stufe geringe Mengen an Trockenpulver, beispielsweise Zeolithpulver wie Zeolith NaA-Pulver, mitverwendet werden. Diese Formgebung kann in marktgängigen Rondiergeräten erfolgen. Dabei ist darauf zu achten, daß in dieser Stufe nur geringe Mengen an Feinkornanteil entstehen. Eine Trocknung, welche in den obengenannten Dokumenten des Standes der Technik als bevorzugte Ausführungsform beschrieben wird, ist anschließend möglich, erfindungsgemäß aber nicht zwingend erforderlich. Es kann gerade bevorzugt sein, nach dem Kompaktierungsschritt keine Trocknung mehr durchzuführen.

Alternativ können Extrusionen/Verpressungen auch in Niedrigdruckextrudern, in der Kahl-Presse (Fa. Amandus Kahl) oder im Bextruder der Fa. Bepex durchgeführt werden.

In einer besonders bevorzugten Ausführungsform sieht die Erfindung nun vor, daß die Temperaturführung im Übergangsbereich der Schnecke, des Vorverteilers und der Düsenplatte derart gestaltet ist, daß die Schmelztemperatur des Bindemittels bzw. die obere Grenze des Schmelzbereichs des Bindemittels zumindest erreicht, vorzugsweise aber überschritten wird. Dabei liegt die Dauer der Temperatureinwirkung im Kompressionsbereich der Extrusion vorzugsweise unterhalb von 2 Minuten und insbesondere in einem Bereich zwischen 30 Sekunden und 1 Minute.

In einer weiteren bevorzugten Ausführungsform der vorliegenden Erfindung wird das erfindungsgemäße Verfahren mittels einer Walzenkompaktierung durchgeführt. Hierbei wird das Vorgemisch gezielt zwischen zwei glatte oder mit Vertiefungen von definierter Form versehene Walzen eindosiert und zwischen den beiden Walzen unter Druck zu einem blattförmigen Kompaktat, der sogenannten Schülpe, ausgewalzt. Die Walzen üben auf das Vorgemisch einen hohen Liniendruck aus und können je nach Bedarf zusätzlich geheizt bzw. gekühlt werden. Bei der Verwendung von Glattwalzen erhält man glatte, unstrukturierte Schülpenbänder, während durch die Verwendung strukturierter Walzen entsprechend strukturierte Schülpen erzeugt werden können, in denen beispielsweise bestimmte Formen der späteren Wasch- oder Reinigungsmittelteilchen vorgegeben werden können. Das Schülpenband wird nachfolgend durch eine Abschlag- und Zerkleinerungsvorgang in kleinere Stücke gebrochen und kann auf diese Weise zu Granulatkörnern verarbeitet werden, die durch weitere an sich bekannte Oberflächenbehandlungsverfahren veredelt, insbesondere in annähernd kugelförmige Gestalt gebracht werden können.

Auch bei der Walzenkompaktierung liegt die Temperatur der pressenden Werkzeuge, also der Walzen, bevorzugt bei maximal 150°C, vorzugsweise bei maximal 100°C und insbesondere bei maximal 75°C. Besonders bevorzugte Herstellungsverfahren arbeiten bei der Walzenkompaktierung mit Verfahrenstemperaturen, die 10°C, insbesondere maximal 5°C oberhalb der Schmelztemperatur bzw. der oberen Temperaturgrenze des Schmelzbereichs des Bindemittels liegen. Hierbei ist es weiter bevorzugt, daß die Dauer der Temperatureinwirkung im Kompressionsbereich der glatten oder mit Vertiefungen von definierter Form versehenen Walzen maximal 2 Minuten beträgt und insbesondere in einem Bereich zwischen 30 Sekunden und 1 Minute liegt.

In einer weiteren bevorzugten Ausführungsform der vorliegenden Erfindung wird das erfindungsgemäße Verfahren mittels einer Pelletierung durchgeführt. Hierbei wird das Vorgemisch auf eine perforierte Fläche aufgebracht und mittels eines druckgebenden Körpers unter Plastifizierung durch die Löcher gedrückt. Bei üblichen Ausführungsformen von Pelletpressen wird das Vorgemisch unter Druck verdichtet, plastifiziert, mittels einer rotierenden Walze in

Form feiner Stränge durch eine perforierte Fläche gedrückt und schließlich mit einer Abschlagvorrichtung zu Granulatkörnern zerkleinert. Hierbei sind die unterschiedlichsten Ausgestaltungen von Druckwalze und perforierter Matrize denkbar. So finden beispielsweise flache perforierte Teller ebenso Anwendung wie konkave oder konvexe Ringmatrizen, durch die das Material mittels einer oder mehrerer Druckwalzen hindurchgepreßt wird. Die Preßrollen können bei den Tellergeräten auch konisch geformt sein, in den ringförmigen Geräten können Matrizen und Preßrolle(n) gleichläufigen oder gegenläufigen Drehsinn besitzen. Ein zur Durchführung des erfindungsgemäßen Verfahrens geeigneter Apparat wird beispielsweise in der deutschen Offenlegungsschrift DE 38 16 842 beschrieben. Die in dieser Schrift offenbarte Ringmatrizenpresse besteht aus einer rotierenden, von Preßkanälen durchsetzten Ringmatrize und wenigstens einer mit deren Innenfläche in Wirkverbindung stehenden Preßrolle, die das dem Matrizenraum zugeführte Material durch die Preßkanäle in einen Materialaustrag preßt. Hierbei sind Ringmatrize und Preßrolle gleichsinnig antreibbar, wodurch eine verringerte Scherbelastung und damit geringere Temperaturerhöhung des Vorgemischs realisierbar ist. Selbstverständlich kann aber auch bei der Pelletierung mit heiz- oder kühlbaren Walzen gearbeitet werden, um eine gewünschte Temperatur des Vorgemischs einzustellen.

Auch bei der Pelletierung liegt die Temperatur der pressenden Werkzeuge, also der Druckwalzen oder Preßrollen, bevorzugt bei maximal 150°C, vorzugsweise bei maximal 100°C und insbesondere bei maximal 75°C. Besonders bevorzugte Herstellungsverfahren arbeiten bei der Walzenkompaktierung mit Verfahrenstemperaturen, die 10°C, insbesondere maximal 5°C oberhalb der Schmelztemperatur bzw. der oberen Temperaturgrenze des Schmelzbereichs des Bindemittels liegen.

Ein weiteres Preßagglomerationsverfahren, das erfindungsgemäß eingesetzt werden kann, ist die Tablettierung. In diesem Verfahren werden Wasch- oder Reinigungsmittelformkörper hergestellt. Dementsprechend liegen die Wasch- oder Reinigungsmittel in einer weiteren bevorzugten Ausführungsform der Erfindung in Form von Formkörpern vor, wobei es sich vorzugsweise um Tabletten handelt, die aus einer einzigen oder aber aus mehren, insbesondere zwei oder drei verschiedenen Phasen bestehen können.

Um den Zerfall hochverdichteter Formkörper zu erleichtern, ist es möglich, Desintegrationshilfsmittel, sogenannte Tablettensprengmittel, in diese einzuarbeiten, um die Zerfallszeiten zu verkürzen. Unter Tablettensprengmitteln bzw. Zerfallsbeschleunigern werden gemäß Römpp (9. Auflage, Bd. 6, S. 4440) und Voigt "*Lehrbuch der pharmazeutischen Technologie*" (6. Auflage, 1987, S. 182-184) Hilfsstoffe verstanden, die für den raschen Zerfall von Tabletten in Wasser oder Magensaft und für die Freisetzung der Pharmaka in resorbierbarer Form sorgen.

Diese Stoffe, die auch aufgrund ihrer Wirkung als "Spreng"mittel bezeichnet werden, vergrößern bei Wasserzutritt ihr Volumen, wobei einerseits das Eigenvolumen vergrößert (Quellung), andererseits auch über die Freisetzung von Gasen ein Druck erzeugt werden kann, der die Tablette in kleinere Partikel zerfallen läßt. Altbekannte Desintegrationshilfsmittel sind beispielsweise Carbonat/Citronensäure-Systeme, wobei auch andere organische Säuren eingesetzt werden können. Quellende Desintegrationshilfsmittel sind beispielsweise synthetische Polymere wie Polyvinylpyrrolidon (PVP) oder natürliche Polymere bzw. modifizierte Naturstoffe wie Cellulose und Stärke und ihre Derivate, Alginate oder Casein-Derivate.

In einer bevorzugten Variante enthalten die Wasch- und Reinigungsmittelformkörper 0,5 bis 10 Gew.-%, vorzugsweise 3 bis 7 Gew.-% und insbesondere 4 bis 6 Gew.-% eines oder mehrerer Desintegrationshilfsmittel, jeweils bezogen auf das Formkörpergewicht.

Als bevorzugte Desintegrationsmittel werden im Rahmen der vorliegenden Erfindung Desintegrationsmittel auf Cellulosebasis eingesetzt, so daß bevorzugte Wasch- und Reinigungsmittelformkörper ein solches Desintegrationsmittel auf Cellulosebasis in Mengen von 0,5 bis 10 Gew.-%, vorzugsweise 3 bis 7 Gew.-% und insbesondere 4 bis 6 Gew.-% enthalten. Reine Cellulose weist die formale Bruttozusammensetzung $(C_6H_{10}O_5)_n$ auf und stellt formal betrachtet ein β-1,4-Polyacetal von Cellobiose dar, die ihrerseits aus zwei Molekülen Glucose aufgebaut ist. Geeignete Cellulosen bestehen dabei aus ca. 500 bis 5000 Glucose-Einheiten und haben demzufolge durchschnittliche Molmassen von 50.000 bis 500.000. Als Desintegrationsmittel auf Cellulosebasis verwendbar sind im Rahmen der vorliegenden Erfindung auch Cellulose-Derivate, die durch polymeranaloge Reaktionen aus Cellulose erhältlich sind. Solche chemisch modifizierten Cellulosen umfassen dabei beispielsweise Produkte aus Veresterungen bzw. Veretherungen, in denen Hydroxy-Wasserstoffatome substituiert wurden. Aber auch Cellulosen, in denen die Hydroxy-Gruppen gegen funktionelle Gruppen, die nicht über ein Sauerstoffatom gebunden sind, ersetzt wurden, lassen sich als Cellulose-Derivate einsetzen. In die Gruppe der Cellulose-Derivate fallen beispielsweise Alkalicellulosen, Carboxymethylcellulose (CMC), Celluloseester und -ether sowie Aminocellulosen. Die genannten Cellulosederivate werden vorzugsweise nicht allein als Desintegrationsmittel auf Cellulosebasis eingesetzt, sondern in Mischung mit Cellulose verwendet. Der Gehalt dieser Mischungen an Cellulosederivaten beträgt vorzugsweise unterhalb 50 Gew.-%, besonders bevorzugt unterhalb 20 Gew.-%, bezogen auf das Desintegrationsmittel auf Cellulosebasis. Besonders bevorzugt wird als Desintegrationsmittel auf Cellulosebasis reine Cellulose eingesetzt, die frei von Cellulosederivaten ist.

Die als Desintegrationshilfsmittel eingesetzte Cellulose wird vorzugsweise nicht in feinteiliger Form eingesetzt, sondern vor dem Zumischen zu den zu verpressenden Vorgemischen in eine gröbere Form überführt, beispielsweise granuliert oder kompaktiert. Wasch- und Reinigungsmittelformkörper, die Sprengmittel in granularer oder gegebenenfalls cogranulierter Form enthalten, werden in den deutschen Patentanmeldungen DE 197 09 991 und DE 197 10 254 sowie der internationalen Patentanmeldung WO98/40463 beschrieben. Diesen Schriften sind auch nähere Angaben zur Herstellung granulierter, kompaktierter oder cogranulierter Cellulosesprengmittel zu entnehmen. Die Teilchengrößen solcher Desintegrationsmittel liegen zumeist oberhalb 200 µm, vorzugsweise zu mindestens 90 Gew.-% zwischen 300 und 1600 µm und insbesondere zu mindestens 90 Gew.-% zwischen 400 und 1200 µm. Die vorstehend genannten und in den zitierten Schriften näher beschriebenen gröberen Desintegrationshilfsmittel auf Cellulosebasis sind im Rahmen der vorliegenden Erfindung bevorzugt als Desintegrationshilfsmittel einzusetzen und im Handel beispielsweise unter der Bezeichnung Arbocel® TF-30-HG von der Firma Rettenmaier erhältlich.

Als weiteres Desintegrationsmittel auf Cellulosebasis oder als Bestandteil dieser Komponente kann mikrokristalline Cellulose verwendet werden. Diese mikrokristalline Cellulose wird durch partielle Hydrolyse von Cellulosen unter solchen Bedingungen erhalten, die nur die amorphen Bereiche (ca. 30% der Gesamt-Cellulosemasse) der Cellulosen angreifen und vollständig auflösen, die kristallinen Bereiche (ca. 70%) aber unbeschadet lassen. Eine nachfolgende Desaggregation der durch die Hydrolyse entstehenden mikrofeinen Cellulosen liefert die mikrokristallinen Cellulosen, die Primärteilchengrößen von ca. 5 µm aufweisen und beispielsweise zu Granulaten mit einer mittleren Teilchengröße von 200 µm kompaktierbar sind.

Im Rahmen der vorliegenden Erfindung bevorzugte Wasch- und Reinigungsmittelformkörper enthalten zusätzlich ein Desintegrationshilfsmittel, vorzugsweise ein Desintegrationshilfsmittel auf Cellulosebasis, vorzugsweise in granularer, cogranulierter oder kompaktierter Form, in Mengen von 0,5 bis 10 Gew.-%, vorzugsweise von 3 bis 7 Gew.-% und insbesondere von 4 bis 6 Gew.-%, jeweils bezogen auf das Formkörpergewicht.

Die Herstellung der erfindungsgemäßen Formkörper erfolgt zunächst durch das trockene Vermischen der Bestandteile, die ganz oder teilweise vorgranuliert sein können, und anschließendes Informbringen, insbesondere Verpressen zu Tabletten, wobei auf herkömmliche Verfahren zurückgegriffen werden kann. Zur Herstellung der erfindungsgemäßen Formkörper wird das Vorgemisch in einer sogenannten Matrize zwischen zwei Stempeln zu einem festen Komprimat verdichtet. Dieser Vorgang, der im folgenden kurz als Tablettierung bezeichnet wird, gliedert sich in vier Abschnitte: Dosierung, Verdichtung (elastische Verformung), plastische Verformung und Ausstoßen.

Zunächst wird das Vorgemisch in die Matrize eingebracht, wobei die Füllmenge und damit das Gewicht und die Form des entstehenden Formkörpers durch die Stellung des unteren Stempels und die Form des Preßwerkzeugs bestimmt werden. Die gleichbleibende Dosierung auch bei hohen Formkörperdurchsätzen wird vorzugsweise über eine volumetrische Dosierung des Vorgemischs erreicht. Im weiteren Verlauf der Tablettierung berührt der Oberstempel das Vorgemisch und senkt sich weiter in Richtung des Unterstempels ab. Bei dieser Verdichtung werden die Partikel des Vorgemisches näher aneinander gedrückt, wobei das Hohlraumvolumen innerhalb der Füllung zwischen den Stempeln kontinuierlich abnimmt. Ab einer bestimmten Position des Oberstempels (und damit ab einem bestimmten Druck auf das Vorgemisch) beginnt die plastische Verformung, bei der die Partikel zusammenfließen und es zur Ausbildung des Formkörpers kommt. Je nach den physikalischen Eigenschaften des Vorgemisches wird auch ein Teil der Vorgemischpartikel zerdrückt und es kommt bei noch höheren Drücken zu einer Sinterung des Vorgemischs. Bei steigender Preßgeschwindigkeit, also hohen Durchsatzmengen, wird die Phase der elastischen Verformung immer weiter verkürzt, so daß die entstehenden Formkörper mehr oder minder große Hohlräume aufweisen können. Im letzten Schritt der Tablettierung wird der fertige Formkörper durch den Unterstempel aus der Matrize herausgedrückt und durch nachfolgende Transporteinrichtungen wegbefördert. Zu diesem Zeitpunkt ist lediglich das Gewicht des Formkörpers endgültig festgelegt, da die Preßlinge aufgrund physikalischer Prozesse (Rückdehnung, kristallographische Effekte, Abkühlung etc.) ihre Form und Größe noch ändern können.

Die Tablettierung erfolgt in handelsüblichen Tablettenpressen, die prinzipiell mit Einfach- oder Zweifachstempeln ausgerüstet sein können. Im letzteren Fall wird nicht nur der Oberstempel zum Druckaufbau verwendet, auch der Unterstempel bewegt sich während des Preßvorgangs auf den Oberstempel zu, während der Oberstempel nach unten drückt. Für kleine Produktionsmengen werden vorzugsweise Exzentertablettenpressen verwendet, bei denen der oder die Stempel an einer Exzenterscheibe befestigt sind, die ihrerseits an einer Achse mit einer bestimmten Umlaufgeschwindigkeit montiert ist. Die Bewegung dieser Preßstempel ist mit der Arbeitsweise eines üblichen Viertaktmotors vergleichbar. Die Verpressung kann mit je einem Ober- und Unterstempel erfolgen, es können aber auch mehrere Stempel an einer Exzenterscheibe befestigt sein, wobei die Anzahl der Matrizenbohrungen entsprechend erweitert ist. Die Durchsätze von Exzenterpressen variieren ja nach Typ von einigen hundert bis maximal 3000 Tabletten pro Stunde.

Für größere Durchsätze wählt man Rundlauftablettenpressen, bei denen auf einem sogenannten Matrizentisch eine größere Anzahl von Matrizen kreisförmig angeordnet ist. Die Zahl der Matrizen variiert je nach Modell zwischen 6 und 55, wobei auch größere Matrizen im Handel erhältlich sind. Jeder Matrize auf dem Matrizentisch ist ein Oberund Unterstempel zugeordnet, wobei wiederum der Preßdruck aktiv nur durch den Ober- bzw. Unterstempel, aber auch durch beide Stempel aufgebaut werden kann. Der Matrizentisch und die Stempel bewegen sich um eine gemeinsame senkrecht stehende Achse, wobei die Stempel mit Hilfe schienenartiger Kurvenbahnen während des Umlaufs in die Positionen für Befüllung, Verdichtung, plastische Verformung und Ausstoß gebracht werden. An den Stellen, an denen eine besonders gravierende Anhebung bzw. Absenkung der Stempel erforderlich ist (Befüllen, Verdichten, Ausstoßen), werden diese Kurvenbahnen durch zusätzliche Niederdruckstücke, Nierderzugschienen und Aushebebahnen unterstützt. Die Befüllung der Matrize erfolgt über eine starr angeordnete Zufuhreinrichtung, den sogenannten Füllschuh, der mit einem Vorratsbehälter für das Vorgemisch verbunden ist. Der Preßdruck auf das Vorgemisch ist über die Preßwege für Ober- und Unterstempel individuell einstellbar, wobei der Druckaufbau durch das Vorbeirollen der Stempelschaftköpfe an verstellbaren Druckrollen geschieht.

Rundlaufpressen können zur Erhöhung des Durchsatzes auch mit zwei Füllschuhen versehen werden, wobei zur Herstellung einer Tablette nur noch ein Halbkreis durchlaufen werden muß. Zur Herstellung zwei- und mehrschichtiger Formkörper werden mehrere Füllschuhe hintereinander angeordnet, ohne daß die leicht angepreßte erste Schicht vor der weiteren Befüllung ausgestoßen wird. Durch geeignete Prozeßführung sind auf diese Weise auch Mantel- und Punkttabletten herstellbar, die einen zwiebelschalenartigen Aufbau haben, wobei im Falle der Punkttabletten die Oberseite des Kerns bzw. der Kernschichten nicht überdeckt wird und somit sichtbar bleibt. Auch Rundlauftablettenpressen sind mit Einfach- oder Mehrfachwerkzeugen ausrüstbar, so daß beispielsweise ein äußerer Kreis mit 50 und ein innerer Kreis mit 35 Bohrungen gleichzeitig zum Verpressen benutzt werden. Die Durchsätze moderner Rundlauftablettenpressen betragen über eine Million Formkörper pro Stunde.

Im Rahmen der vorliegenden Erfindung geeignete Tablettiermaschinen sind beispielsweise erhältlich bei den Firmen Apparatebau Holzwarth GbR, Asperg, Wilhelm Fette GmbH, Schwarzenbek, Hofer GmbH, Weil, KILIAN, Köln, KOMAGE, Kell am See, KORSCH Pressen GmbH, Berlin, Mapag Maschinenbau AG, Bern (CH) sowie Courtoy N.V., Halle (BE/LU). Besonders geeignet ist beispielsweise die Hydraulische Doppeldruckpresse HPF 630 der Firma LAEIS, D.

Die Formkörper können dabei in vorbestimmter Raumform und vorbestimmter Größe gefertigt werden. Als Raumform kommen praktisch alle sinnvoll handhabbaren Ausgestaltungen in Betracht, beispielsweise also die Ausbildung als Tafel, die Stab- bzw. Barrenform, Würfel, Quader und entsprechende Raumelemente mit ebenen Seitenflächen sowie insbesondere zylinderförmige Ausgestaltungen mit kreisförmigem oder ovalem Querschnitt. Diese letzte Ausgestaltung erfaßt dabei die Darbietungsform von der Tablette bis zu kompakten Zylinderstücken mit einem Verhältnis von Höhe zu Durchmesser oberhalb 1.

Die portionierten Preßlinge können dabei jeweils als voneinander getrennte Einzelelemente ausgebildet sein, die der vorbestimmten Dosiermenge der Waschund/oder Reinigungsmittel entspricht. Ebenso ist es aber möglich, Preßlinge auszubilden, die eine Mehrzahl solcher Masseneinheiten in einem Preßling verbinden, wobei insbesondere durch vorgegebene Sollbruchstellen die leichte Abtrennbarkeit portionierter kleinerer Einheiten vorgesehen ist. Für den Einsatz von Textilwaschmitteln in Maschinen des in Europa üblichen Typs mit horizontal angeordneter Mechanik kann die Ausbildung der portionierten Preßlinge als Tabletten, in Zylinder- oder Quaderform zweckmäßig sein, wobei ein Durchmesser/Höhe-Verhältnis im Bereich von etwa 0,5 : 2 bis 2 : 0,5 bevorzugt ist. Handelsübliche Hydraulikpressen, Exzenterpressen oder Rundläuferpressen sind geeignete Vorrichtungen insbesondere zur Herstellung derartiger Preßlinge.

Die Raumform einer anderen Ausführungsform der Formkörper ist in ihren Dimensionen der Einspülkammer von handelsüblichen Haushaltswaschmaschinen angepaßt, so daß die Formkörper ohne Dosierhilfe direkt in die Einspülkammer eindosiert werden können, wo sie sich während des Einspülvorgangs auflöst. Selbstverständlich ist aber auch ein Einsatz der Waschmittelformkörper über eine Dosierhilfe problemlos möglich und im Rahmen der vorliegenden Erfindung bevorzugt.

Ein weiterer bevorzugter Formkörper, der hergestellt werden kann, hat eine platten- oder tafelartige Struktur mit abwechselnd dicken langen und dünnen kurzen Segmenten, so daß einzelne Segmente von diesem "Riegel" an den Sollbruchstellen, die die kurzen dünnen Segmente darstellen, abgebrochen und in die Maschine eingegeben werden können. Dieses Prinzip des "riegelförmigen" Formkörperwaschmittels kann auch in anderen geometrischen Formen, beispielsweise senkrecht stehenden Dreiecken, die lediglich an einer ihrer Seiten längsseits miteinander verbunden sind, verwirklicht werden.

Möglich ist es aber auch, daß die verschiedenen Komponenten nicht zu einer einheitlichen Tablette verpreßt werden, sondern daß Formkörper erhalten werden, die mehrere Phasen oder Schichten, also mindestens zwei Phasen oder Schichten, aufweisen. Dabei ist es auch möglich, daß diese verschiedenen Schichten unterschiedliche Lösegeschwindigkeiten aufweisen. Hieraus können vorteilhafte anwendungstechnische Eigenschaften der Formkörper resultieren. Falls beispielsweise Komponenten in den Formkörpern enthalten sind, die sich wechselseitig negativ beeinflussen, so ist es möglich, die eine Komponente in der schneller löslichen Schicht zu integrieren und die andere Komponente in eine langsamer lösliche Schicht einzuarbeiten, so daß die erste Komponente bereits abreagiert hat, wenn die zweite in Lösung geht. Bevorzugt sind auch Wasch- oder Reinigungsmittelformkörper, bei denen mindestens 2 Phasen den selben Wirkstoff in unterschiedlichen Mengen enthalten. Der Begriff "unterschiedliche Mengen" bezieht sich dabei nicht auf die absolute Menge des Inhaltsstoff in der Phase, sondern auf die Relativmenge, bezogen auf das Phasengewicht, stellt also eine Gewichtsprozent-Angabe, bezogen auf die einzelnen Phasen, dar. Im Rahmen der vorliegenden Erfindung kann es insbesondere bevorzugt sein, wenn die erfindungsgemäßen und/oder erfindungsgemäß zu verwendenden Kieselsäureester, und vorzugsweise auch das gesamte Parfümgemisch, sich in anderen Phasen der Formkörper befinden, als in diesen Formkörpern enthaltene Bleichmittel. Ebenso ist es bevorzugt, wenn Alkaliträger, wie Alkalimetallhydroxyde, Alkalimetallcarbonate, Alkalimetallhydrogencarbonate, Alkalimetallsequicarbonate, Alkalisilikate, Alkalimethasilikate und Mischungen dieser Stoffe, sich zumindest zum größten Anteil, vorzugsweise jedoch vollständig, in anderen Phasen der Formkörper befinden, als die erfindungsgemäßen und/oder erfindungsgemäß zu verwendenden Kieselsäureester, bzw. besonders bevorzugt das gesamte Parfümgemisch. Der Schichtaufbau der Formkörper kann dabei sowohl stapelartig erfolgen, wobei ein Lösungsvorgang der inneren Schicht(en) an den Kanten des Formkörpers bereits dann erfolgt, wenn die äußeren Schichten noch nicht vollständig gelöst sind, es kann aber auch eine vollständige Umhüllung der inneren Schicht(en) durch die jeweils weiter außen liegende(n) Schicht(en) erreicht werden, was zu einer Verhinderung der frühzeitigen Lösung von Bestandteilen der inneren Schicht(en) führt.

In einer weiter bevorzugten Ausführungsform der Erfindung besteht ein Formkörper aus mindestens drei Schichten, also zwei äußeren und mindestens einer inneren Schicht, wobei mindestens in einer der inneren Schichten ein Peroxy-Bleichmittel enthalten ist, während beim stapelförmigen Formkörper die beiden Deckschichten und beim hüllenförmigen Formkörper die äußersten Schichten jedoch frei von Peroxy-Bleichmittel sind. Weiterhin ist es auch möglich, Peroxy-Bleichmittel und gegebenenfalls vorhandene Bleichaktivatoren und/oder Enzyme räumlich in einem Formkörper voneinander zu trennen. Derartige mehrschichtige Formkörper weisen den Vorteil auf, daß sie nicht nur über eine Einspülkammer oder über eine Dosiervorrichtung, welche in die Waschflotte gegeben wird, eingesetzt werden können; vielmehr ist es in solchen Fällen auch möglich, den Formkörper im direkten Kontakt zu den Textilien in die Maschine zu geben, ohne daß Verfleckungen durch Bleichmittel und dergleichen zu befürchten wären.

Ähnliche Effekte lassen sich auch durch Beschichtung ("coating") einzelner Bestandteile der zu verpressenden Wasch- und Reinigungsmittelzusammensetzung oder des gesamten Formkörpers erreichen. Hierzu können die zu beschichtenden Körper beispielsweise mit wäßrigen Lösungen oder Emulsionen bedüst werden, oder aber über das Verfahren der Schmelzbeschichtung einen Überzug erhalten.

Nach dem Verpressen weisen die Wasch- und Reinigungsmittelformkörper eine hohe Stabilität auf. Die Bruchfestigkeit zylinderförmiger Formkörper kann über die Meßgröße der diametralen Bruchbeanspruchung erfaßt werden. Diese ist bestimmbar nach

$$\sigma = \frac{2P}{\pi D t}$$

Hierin steht σ für die diametrale Bruchbeanspruchung (diametral fracture stress, DFS) in Pa, P ist die Kraft in N, die zu dem auf den Formkörper ausgeübten Druck führt, der den Bruch des Formkörpers verursacht, D ist der Formkörperdurchmesser in Meter und t ist die Höhe der Formkörper.

Ein weiterer Gegenstand der vorliegenden Erfindung sind kosmetische Mittel zur Haaroder Hautbehändlung, die Duftstoffmischungen oder Parfümzusammensetzungen, enthaltend Kieselsäureester der Formel I

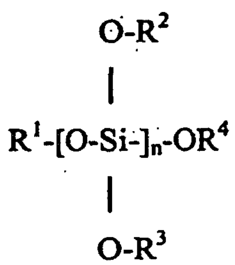

(I),

in der $R^1$ und $R^4$ unabhängig voneinander ausgewählt sind aus der Gruppe der geradkettigen oder verzweigten, gesättigten oder ungesättigten, substituierten oder unsubstituierten $C_{1-6}$-Kohlenwasserstoffreste und der Duftstoffalkoholreste, jedes $R^2$ und $R^3$ unabhängig voneinander ausgewählt ist aus der Gruppe der Duftstoffalkoholreste und n Werte zwischen 2 und 20 annimmt, enthalten. Dabei enthalten die Mittel, wie bereits oben beschrieben wurde, neben diesen Kieselsäureestern noch weitere Duftstoffe.

Vorzugsweise enthalten diese kosmetischen Mittel Kieselsäureester I in Mengen von 0,001 bis 10 Gew.-%, vorzugsweise von 0,01 bis 5 Gew.-%, besonders bevorzugt von 0,02 bis 3 und insbesondere in Mengen von 0,05 bis 2 Gew.-%, jeweils bezogen auf das Wasch- und Reinigungsmittel, enthalten sind.

In einer bevorzugten Ausführungsform handelt es sich bei den kosmetischen Mitteln um wäßrige Zubereitungen, die oberflächenaktive Wirkstoffe enthalten und die sich insbesondere zur Behandlung von Keratinfasern, insbesondere menschlichen Haaren, oder zur Behandlung von Haut eignen.

Bei den angesprochenen Haarbehandlungsmitteln handelt es sich dabei insbesondere um Mittel zur Behandlung von menschlichem Kopfhaar. Die gebräuchlichsten Mittel dieser Kategorie lassen sich einteilen in Haarwaschmittel, Haarpflegemittel, Haarverfestigungsund Haarverformungsmittel sowie Haarfärbemittel und Haarentfernungsmittel. Zu den erfindungsgemäß bevorzugten, oberflächenaktive Wirkstoffe enthaltenden Mittel zählen insbesondere Haarwasch- und Pflegemittel. Ein derartiges Haarwaschmittel oder Haarshampoo besteht aus 10 bis 20, in Einzelfällen bis zu 30 Rezepturbestandteilen. Diese wäßrigen Zubereitungen liegen meist in flüssig bis pastöser Form vor.

Für die wichtigste Inhaltsstoff-Gruppe, die oberflächenaktiven Wirkstoffe oder Waschaktivstoffe werden überwiegend Fettalkoholpolyglykolethersulfate (Ethersulfate, Alkylethersulfate) eingesetzt, zum Teil in Kombination mit anderen meist anionischen Tensiden. Shampoo-Tenside sollen außer guter Reinigungskraft und Unempfindlichkeit gegen Wasserhärte Haut- und Schleimhautverträglichkeit aufweisen. Entsprechend den gesetzlichen Regelungen muß gute biologische Abbaubarkeit gegeben sein. Neben den Alkylethersulfaten können bevorzugte Mittel zusätzlich weitere Tenside, wie Alkylsulfate, Alkylethercarboxylate, vorzugsweise mit Ethoxylierungsgraden von 4 bis 10, sowie tensidische Eiweiß-Fettsäure-Kondensate enthalten. Hier ist insbesondere das Eiweiß-Abitinsäure-Kondensat zu erwähnen. Auch Sulfobernsteinsäureester, Amidopropylbetaine, Amphoacetate und Amphodiacetate sowie Alkylpolyglykoside sind in Haarshampoos bevorzugt eingesetzte Tenside. Eine weitere Gruppe von Inhaltsstoffen wird unter dem Begriff Hilfsstoffe zusammengefaßt und ist sehr vielfältig: z. B. erhöhen Zusätze von nichtionischen Tensiden wie ethoxylierten Sorbitanestem oder von Eiweiß-Hydrolysaten die Verträglichkeit bzw. wirken reizmindernd, z. B. in Baby-Shampoos; als Rückfetter zur Vorbeugung zu starker Entfettung bei der Haarwäsche dienen z. B. natürliche Öle oder synthetische Fettsäureester; als Feuchthaltemittel dienen Glycerin, Sorbit, Propylenglykol (s. Propandiole), Polyethylenglykole u. a. Polyole. Zur Verbesserung der Naßkämmbarkeit und Verminderung elektrostatischer Aufladung der Haare nach dem Trocknen können den Shampoos Kationtenside wie z. B. quartäre Ammonium-Verbindungen zugesetzt werden. Für ein farbiges, brillantes Erscheinungsbild werden Farbstoffe bzw. Perlglanzpigmente zugesetzt. Zur Einstellung der gewünschten Viskosität können Verdickungsmittel verschiedener Stoffklassen verwendet werden, eine pH-Stabilität wird durch Puffer z. B. auf der Basis von Citrat, Lactat oder Phosphat erzielt. Um eine ausreichende Haltbarkeit und Lagerfähigkeit zu gewährleisten, werden Konservierungsmittel wie z. B. 4-Hydroxybenzoesäureester zugesetzt; oxidationsempfindliche Inhaltsstoffe können durch Zusatz von Antioxidantien wie Ascorbinsäure, Butylmethoxyphenol oder Tocopherol geschützt werden.

Eine dritte Gruppe von Inhaltsstoffen bilden spezielle Wirkstoffe für Spezial-Shampoos, z. B. Öle, Kräuterextrakte, Proteine, Vitamine und Lecithine in Shampoos für schnell fettendes, für besonders trockenes, für strapaziertes oder geschädigtes Haar. Wirkstoffe in Shampoos zur Bekämpfung von Schuppen haben meist eine breite wachstumshemmende Wirkung gegen Pilze und Bakterien. Insbesondere die fungistatischen Eigenschaften z. B. von Pyrithion-Salzen konnten als Ursache guter Antischuppen-Wirkung nachgewiesen werden. Zur Erzeugung einer angenehmen Duftnote enthalten die Haarshampoos Parfümöle. Dabei können die Shampoos ausschließlich die erfindungsgemäßen Kieselsäureester enthalten, es ist jedoch ebenfalls bevorzugt wenn die Haarshampoos nicht nur diese, sondern auch andere Duftstoffe enthalten. Dabei können alle üblichen, und in Haarshampoos zugelassenen Duftstoffe eingesetzt werden.

Haarpflegemittel haben zum Ziel den Naturzustand des frisch nachgewachsenen Haares möglichst lange zu erhalten und bei Schädigung wiederherzustellen. Merkmale die diesen Naturzustand charakterisieren sind seidiger Glanz, geringe Porosität, spannkräftige und dabei weiche Fülle und angenehm glattes Gefühl. Eine wichtige Voraussetzung hierfür ist eine saubere, schuppenfreie und nicht überfettete Kopfhaut. Zu den Haarpflegemitteln zählt man heute eine Vielzahl verschiedener Produkte, deren wichtigste Vertreter als Vorbehandlungsmittel, Haarwässer, Frisierhilfsmittel, Haarspülungen und Kurpackungen bezeichnet werden, und deren Zusammensetzung wie bei den Haarwaschmitteln grob in Grundstoffe, Hilfsstoffe und spezielle Wirkstoffe aufgegliedert wird.

Als Grundstoffe dienen Fettalkohole, v.a. Cetylalkohol (1-Hexadecanol) und Stearylalkohol (1-Octadecanol), Wachse wie Bienenwachs, Wollwachs (Lanolin), Walrat und synthetische Wachse, Paraffine, Vaseline, Paraffinöl sowie als Lsm. v. a. Ethanol, 2-Propanol und Wasser. Hilfsstoffe sind Emulgatoren, Verdickungsmittel, Konservierungsmittel, Antioxidantien, Farbstoffe und Parfüm-Öle. Die heute wichtigste Gruppe spezieller Wirkstoffe in den Haarpflegemitteln sind die quartären Ammonium-Verbindungen. Man unterscheidet monomere (z. B.: Alkyltrimethylammoniumhalogenid mit v. a. der Lauryl-, Cetyl- oder Stearyl-Gruppe als Alkyl-Rest) und polymere quartäre Ammonium-Verbindungen [z. B.: quartäre Celluloseether-Derivate oder Poly(N,N-dimethyl-3,4-methylenpyrrolidiniumchlorid)]. Ihre Wirkung in Haarpflegemitteln beruht darauf, daß die positive Ladung der Stickstoff-Atome dieser Verbindung sich an die negativen Ladungen des Haar-Keratins anlagern kann; geschädigte Haare enthalten wegen ihres höheren Cysteinsäure-Gehalts mehr negativ geladene Säure-Gruppen und können daher mehr quartäre Ammonium-Verbindungen aufnehmen. Diese, wegen ihres kationaktiven Charakters auch als "kationaktive Pflegestoffe" bezeichnet, wirken glättend auf das Haar, verbessern die Kämmbarkeit, vermindern die elektrostatische Aufladung, verbessern Griff und Glanz. Die polymeren quartären Ammonium-Verbindungen haften so gut am Haar, daß ihre Wirkung noch nach mehreren Wäschen nachgewiesen werden kann. Organische Säuren wie Citronensäure, Weinsäure oder Milchsäure werden häufig zur Einstellung eines sauren Milieus eingesetzt. Die wasserlöslichen Eiweiß-Hydrolysate ziehen wegen ihrer engen chemischen Verwandtschaft gut auf das Haar-Keratin auf. Die größte Gruppe spezieller Wirkstoffe in Haarpflegemitteln bilden diverse Pflanzenextrakte und Pflanzenöle, die meist bereits seit langem verwendet werden, ohne daß ihre Wirksamkeit auf den ausgelobten Effekt in allen Fällen wissenschaftlich einwandfrei nachgewiesen wurde. Genauso ist die Wirksamkeit von in Haarpflegemitteln verwendeten Vitaminen nur in Einzelfällen nachgewiesen. Zur Vermeidung einer zu schnellen Rückfettung enthalten einige Haarwässer Substanzen wie gewisse Teer-Inhaltsstoffe, Cysteinsäure-Derivate oder Glycyrrhizin; die beabsichtigte Verminderung der Talgdrüsenproduktion ist ebenfalls noch nicht eindeutig bewiesen. Dagegen ist die Wirksamkeit von Antischuppen-Wirkstoffen einwandfrei belegt. Sie werden daher in entsprechenden Haarwässern u. a. Pflegemitteln eingesetzt.

Bei den wäßrigen Zubereitungen zur Behandlung von Haut handelt es sich insbesondere um Zubereitungen zur Pflege menschlicher Haut. Diese Pflege beginnt mit der Reinigung für die in erster Linie Seifen benutzt werden. Hier unterscheidet man feste, meist stückige und flüssige Seife. Dementsprechend liegen die kosmetischen Mittel in einer bevorzugten Ausführungsform als Formkörper vor, die oberflächenaktive Inhaltsstoffe enthalten. Wichtigste Inhaltsstoffe derartiger Formkörper sind in einer bevorzugten Ausführungsform die Alkalisalze der Fettsäuren natürlicher Öle und Fette, vorzugsweise mit Ketten von 12-18 C-Atomen. Da Laurinsäure-Seifen besonders gut schäumen, sind die Laurinsäure-reichen Kokos- und Palmkern-Öle bevorzugte Rohstoffe für die Feinseifen-Herstellung. Die Na-Salze der Fettsäure-Gemische sind fest, die K-Salze weich-pastös. Zur Verseifung wird die verdünnte Natron- oder Kali-Lauge den Fett-Rohstoffen im stöchiometrichem Verhältnis so zugesetzt, daß in der fertigen Seife ein Laugenüberschuß von max. 0,05% vorhanden ist. Vielfach werden die Seifen heute nicht mehr direkt aus den Fetten, sondern aus den durch Fettspaltung gewonnenen Fettsäuren hergestellt. Übliche Seifen-Zusätze sind Fettsäuren, Fettalkohole, Lanolin, Lecithin, pflanzliche Öle, Partialglyceride u. a. fettähnliche Substanzen zur Rückfettung der gereinigten Haut, Antioxidantien wie Ascorbyl-Palmitat oder Tocopherol zur Verhinderung der Autoxidation der Seife (Ranzigkeit), Komplexierungsmittel wie Nitrilotriacetat zur Bindung von Schwermetall-Spuren, die den autoxidativen Verderb katalysieren könnten, Parfüm-Öle zur Erzielung der gewünschten Duftnoten, Farbstoffe zur Einfärbung der Seifenstücke und ggf. spezielle Zusätze.

Wichtigste Typen der Feinseifen sind:

- Toilettenseifen mit 20-50% Kokosöl im Fettansatz, bis 5% Rückfetter-Anteil und 0,5-2% Parfümöl, sie bilden den größten Anteil der Feinseifen;
- Luxusseifen mit bis zu 5% z. T. besonders kostbarer Parfümöle;
- Deoseifen mit Zusätzen desodorierender Wirkstoffe, wie z. B. 3,4,4'-Trichlorcarbanilid (Triclocarban);
- Cremeseifen mit besonders hohen Anteilen rückfettender und die Haut cremender Substanzen;
- Babyseifen mit guter Rückfettung und zusätzlich pflegenden Anteilen wie z. B. Kamille-Extrakten, allenfalls sehr schwach parfümiert;
- Hautschutzseifen mit hohen Anteilen rückfettender Substanzen sowie weiteren pflegenden und schützenden Zusätzen, wie z. B. Proteinen;
- Transparentseifen mit Zusätzen von Glycerin, Zucker u.a., welche die Kristallisation der Fettsäure-Salze in der erstarrten Seifenschmelze verhindern und so ein transparentes Aussehen bewirken;
- Schwimmseifen mit D. < 1, hervorgerufen durch bei der Herstellung kontrolliert eingearbeitete Luftbläschen.

Seifen können auch mit abrasiven Zusätzen zur Reinigung stark verschmutzter Hände versehen sein. Beim Waschen mit Seife stellt sich in der Waschlauge ein pH-Wert von 8-10 ein. Diese Alkalität neutralisiert den natürlichen Säuremantel der Haut (pH-Wert 5-6). Dieser wird bei normaler Haut zwar relativ schnell rückgebildet, bei empfindlicher oder vorgeschädigter Haut kann es jedoch zu Irritationen kommen. Ein weiterer Nachteil der Seifen ist die Bildung unlöslicher Kalkseifen in hartem Wasser. Diese Nachteile liegen nicht vor bei Syndet-Seifen. Ihre Basis sind synthetische Aniontenside, die mit Gerüstsubstanzen, Rückfettem und weiteren Zusätzen zu Seifen-ähnlichen Stücken verarbeitet werden können. Ihr pH-Wert ist in weiten Grenzen variierbar und wird meist neutral auf pH 7 oder dem Säuremantel der Haut angepaßt auf pH 5,5 eingestellt. Sie haben hervorragende Reinigungskraft, schäumen in jeder Wasserhärte, sogar in Meerwasser, der Anteil rückfettender Zusätze muß wegen ihrer intensiven Reinigungsund Entfettungswirkung deutlich höher als bei normalen Seifen sein. Ihr Nachteil ist der relativ hohe Preis.

Flüssige Seifen basieren sowohl auf K-Salzen natürlicher Fettsäuren als auch auf synthetischen Aniontensiden. Sie enthalten in wäßriger. Lösung. weniger waschaktive Substanzen als feste Seifen, haben die üblichen Zusätze, ggf. mit viskositätsregulierenden Bestandteilen sowie Perlglanz-Additiven. Wegen ihrer bequemen und hygienischen Anwendung aus Spendern werden sie vorzugsweise in öffentlichen Waschräumen und dergleichen verwendet. Wasch-Lotionen für besonders empfindliche Haut basieren auf mild wirkenden synthetischen Tensiden mit Zusätzen hautpflegender Substanzen, pHneutral oder schwach sauer (pH 5,5) eingestellt.

Zur Reinigung vornehmlich der Gesichtshaut gibt es eine Reihe weitere Präparate, wie Gesichtswässer, Reinigungs-Lotionen, -Milche, -Cremes, -Pasten; Gesichtspackungen dienen z. T. der Reinigung, überwiegend jedoch

der Erfrischung und Pflege der Gesichtshaut. Gesichtswässer sind meist wässrige-alkoholische Lösungen mit geringen Tensid-Anteilen sowie weiteren hautpflegenden Substanzen. Reinigungs-Lotionen, - Milche, -Cremes und -Pasten basieren meist auf O/W-Emulsionen mit relativ geringen Gehalten an Fettkomponenten mit reinigenden und pflegenden Zusätzen. Sogenannte Scruffing- und Peeling-Präparate enthalten mild keratolytisch wirkende Substanzen zur Entfernung der obersten abgestorbenen Haut-Horn-Schichten, z. T. mit Zusätzen abrasiv wirkender Pulver. Die seit langem als mildes Hautreinigungsmittel verwendete Mandelkleie ist auch heute noch vielfach Bestandteil solcher Präparate. In Mitteln zur reinigenden Behandlung unreiner Haut sind außerdem antibakterielle und entzündungshemmende Substanzen enthalten, da die Talgansammlungen in Komedonen (Mitessern) Nährböden für bakterielle Infektionen darstellen und zu Entzündungen neigen. Die angebotene breite Palette verschiedener Hautreinigungs-Produkte variiert in Zusammensetzung und Gehalt an diversen Wirkstoffen, abgestimmt auf die verschiedenen Hauttypen u. auf spezielle Behandlungsziele.

Die für die Hautreinigung im Wannen- oder Duschbad angebotenen Badezusätze haben breite Anwendung gefunden. Badesalze und Badetabletten sollen das Badewasser enthärten, färben und parfümieren und enthalten in der Regel keine waschaktiven Substanzen. Durch die Enthärtung des Badewassers fördern sie die Reinigungskraft von Seifen, sollen jedoch in erster Linie erfrischend wirken und das Badeerlebnis verstärken. Größere Bedeutung haben die Schaumbäder. Bei einem höheren Gehalt an rückfettenden und hautpflegenden Substanzen spricht man auch von Creme-Bädern.

Seit etwa 1970 haben sich die Duschbäder neben den Schaumbädern auf dem Markt durchgesetzt und diese seit 1986 im Produktionsvolumen wertmäßig überflügelt. Sie sind ähnlich zusammengesetzt wie flüssige Haarwaschmittel, enthalten jedoch anstelle haarpflegender besondere hautpflegende Wirkstoffe. Neuerdings sind auch Kombipräparate, geeignet für Haut und Haar, auf dem Markt.

Die der Reinigung folgende Hautpflege hat zwei wesentliche Ziele: Zum einen soll sie der Haut die bei der Wäsche unkontrolliert entzogenen Inhaltsstoffe wie Hornzellen, Hautfettlipide, Säurebildner und Wasser zurückführen in den natürlichen Gleichgewichtszustand, zum anderen u. v. a. soll sie dem natürlichen Alterungsprozeß der Haut sowie den möglichen Schädigungen durch Witterungs- und Umwelteinflüsse möglichst weitgehend entgegenwirken. Präparate zur Hautpflege und zum Hautschutz werden in großer Zahl und in vielen Zubereitungsformen angeboten. Die wichtigsten sind Haut-Cremes, -Lotionen, -Öle und -Gele. Basis der Cremes und Lotionen sind Emulsionen in O/W- (Öl in Wasser) od. W/O- (Wasser in Öl) Form. Die Hauptbestandteile der Öl- bzw. Fett- oder Lipid-Phase sind Fettalkohole, Fettsäuren, Fettsäureester, Wachse, Vaseline, Paraffine sowie weitere Fett- und Ölkomponenten hauptsächlich natürlichen Ursprungs. In der wäßrigen Phase sind neben Wasser hauptsächlich feuchtigkeitsregulierende und feuchtigkeitsbewahrende Substanzen als wesentliche Hautpflege-Wirkstoffe enthalten, ferner konsistenz- bzw. viskositätsregulierende Mittel. Weitere Zusätze wie Konservierungsmittel, Antioxidantien, Komplexbildner, Parfüm-Öle, Färbemittel sowie spezielle Wirkstoffe werden je nach ihrer Löslichkeit und ihren Stabilitätseigenschaften einer der beiden vorgenannten Phasen beigegeben. Wesentlich für den Emulsionstyp und seine Eigenschaften ist die Auswahl des Emulgator-Systems. Seine Auswahl kann nach dem HLB-System erfolgen.

Nach ihrem Anwendungs-Bereich kann man die Cremes bzw. Lotionen einteilen in "Tagescremes" und "Nachtcremes". Tagescremes sind meist als O/W-Emulsionen aufgebaut, sie ziehen schnell in die Haut ein, ohne einen Fettglanz zu hinterlassen; man bezeichnet sie daher z. T. auch als Trockencremes, Mattcremes oder Vanishing Creams. Nachtcremes sind meist W/O-Emulsionen, sie werden von der Haut langsamer aufgenommen und enthalten oft spezielle Wirkstoffe, die während der Nachtruhe eine Regeneration der Haut bewirken sollen. Manche dieser Präparate werden auch als "Nährcremes" bezeichnet, obgleich eine "Ernährung" des Zellstoffwechsels in der Haut nur über den Blutkreislauf erfolgen kann; der Begriff "Nährcreme" ist daher umstritten. Sogenannte Cold Creams sind Mischemulsionen vom O/W- und W/O-Typ, wobei die Ölphase mengenmäßig überwiegt. Bei der klassischen Cold Cream wurde beim Auftragen das z. T. nur instabil emulgierte Wasser frei und erzeugte durch Verdunsten einen Kühleffekt, der dieser Zubereitungsform ihren Namen verlieh.

Auf die Vielzahl der in den Hautpflegemitteln eingesetzten speziellen Wirkstoffe und die ihnen zugeschriebenen Wirkungen kann hier nicht im einzelnen eingegangen werden. Erwähnt seien Milcheiweißprodukte, Eigelb, Lecithine, Lipoide, Phosphatide, Getreidekeimöle, Vitamine - insbesondere Vitamin F und das früher als Hautvitamin (Vitamin H) bezeichnete Biotin sowie hormonfreie Placenta-Extrakte. Früher manchmal eingesetzte Hormone werden nicht mehr verwendet, da sie als Arzneimittel-Wirkstoffe eingestuft sind und in kosmetischen Mitteln nicht verwendet werden dürfen.

Hautöle gehören zu den ältesten Produktformen der Hautpflege und werden noch heute verwendet. Basis sind nichttrocknende Pflanzenöle wie Mandelöl oder Olivenöl, mit Zusätzen natürlicher Vitaminöle wie Weizenkeimöl oder Avocadoöl sowie öligen Pflanzenextrakten aus z. B. Johanniskraut, Kamille u. ä. Der Zusatz von Antioxidantien gegen Ranzigkeit ist unerläßlich, gewünschte Duftnoten werden durch Parfüm- oder ether. Öle erzielt, ein Zusatz von Paraffinöl oder flüssigen Fettsäureestern dient zur Optimierung der Anwendungs.-Eigenschaften.

Hautgele sind halbfeste transparente Produkte, die durch entsprechende Gelbildner stabilisiert werden. Man unterscheidet Oleogele (wasserfrei), Hydrogele (ölfrei) und Öl/Wasser-Gele. Die Typenauswahl richtet sich nach dem

gewünschten AnwendungsZweck. Die Öl/Wasser-Gele enthalten hohe Emulgator-Anteile und weisen gegenüber Emulsionen gewisse Vorteile auf sowohl unter ästhetischen als auch unter Anwendungs.-Gesichtspunkten.

Fußbäder sollen gut reinigend, erfrischend, durchblutungsfördernd und belebend sowie desodorierend und homhauterweichend wirken. Fußbadzusätze gibt es als Badesalze und Schaumbäder. Sie bestehen z. B. aus Basismischungen von Na-Carbonat, Na-Hydrogencarbonat und Na-Perborat oder Na-Hexametaphosphat (s. kondensierte Phosphate), Na-Sulfat, Na-Perborat und 1% Na-Laurylsulfat als Schaumkomponente mit antihidrot., desodorierenden, ggf. bakteriziden und/oder fungiziden Zusätzen sowie Duftund Farbstoffen. Fußpuder sollen, nach der Fußwäsche angewendet und/oder eingestreut in Strümpfe und Schuhe, hautglättend, kühlend, feuchtigkeitsaufsaugend, schweißhemmend, antiseptisch, desodorierend und ggf. homhauterweichend wirken. Sie bestehen in der Regel zu 85% aus Talkum (s. Talk) mit Zusätzen von Kieselsäure-Pulver, Aluminiumhydroxychlorid, Salicylsäure sowie ggf. Bakteriziden, Fungiziden, Desodorantien und Duftstoffen. Fußcremes bzw. Fußbalsame werden zur Hautpflege sowie zur Massage der Fuß- und Unterschenkel-Muskulatur verwendet. Fußcremes sind in der Regel O/W-Emulsionen aus z. B. 30%Isopropyl-Myristat, 10% Polysorbat, 4,2% Aluminiummetahydroxid und 55,8% Wasser als Basisrezeptur; Fußbalsame sind meist wasserfrei und. enthalten z. B. 85% Vaseline 5% Paraffin, 3% Lanolin, 3% Methylsalicylat, 2% Campher, 1% Menthol und 1% Eucalyptusöl. Hornhautbeseitigende Mittel wie z. B. "Rubbelcremes" werden so lange auf der Haut verrieben, bis die Hornhaut krümelförmig abgetragen wird. Eine Rahmenrezeptur besteht aus 25% Paraffin, 2% Stearinsäure, 2% Bienenwachs, 2% Walrat, 2% Glycerinmonostearat, 0,5% 2,2',2"-Nitrilotriethanol, 1% Parfümöl, 0,2% 4-Hydroxybenzoesäure und 65,3% Wasser. Nagelfalz-Tinkturen dienen zur Erweichung von Verhornungen in den Nagelfalzen und zum Weichhalten der Nagelränder bei einwachsenden Zehennägeln, hauptsächlich an den Großzehen. Eine Rahmenrezeptur ist aus 10% 2,2',2"-Nitrilotriethanol, 15% Harnstoff, 0,5% Fettalkoholpolyglykolether und 74,5% Wasser aufgebaut

Weitere erfindungsgemäß bevorzugte kosmetische Mittel sind Mittel zur Beeinflussung des Körpergeruchs. Insbesondere sind hier deodorierende Mittel gemeint. Derartige Deodorantien können Gerüche überdecken, entfernen oder zerstören. Unangenehme Körpergerüche entstehen bei bakterieller Zersetzung des Schweißes, insbesondere in den feuchtwarmen Achselhöhlen, wo Mikroorganismen gute Lebensbedingungen finden. Dementsprechend sind die wichtigsten Inhaltsstoffe von Deodorantien keimhemmende Substanzen. Insbesondere sind solche keimhemmenden Substanzen bevorzugt, die eine weitgehende selektive Wirksamkeit gegenüber den für den Körpergeruch verantwortlichen Bakterien besitzen. Bevorzugte Wirkstoffe haben dabei jedoch lediglich eine bakteriostatische Wirkung und töten die Bakterienflora keinesfalls ganz ab. Zu dem keimhemmenden Mitteln können generell alle geeigneten Konservierungsmittel mit spezifischer Wirkung gegen grampositiver Bakterien gerichtet werden. Beispielsweise sind dies Irgasan DP 300 (Trichlosan, 2,4,4'-Trichlor-2'-Hydroxydiphenylether), Chlorhexidin (1,1'-Hexamethylenbis(5-(4'-chlorphenyl)-biguanid) sowie 3,4,4'-Trichlorcarbanilid. Auch quartäre Ammonium-Verbindungen sind prinzipiell ebenfalls geeignet. Aufgrund ihrer hohen antimikrobierenden Wirksamkeit werden all diese Stoffe bevorzugt nur in geringen Konzentrationen von etwa 0,1 bis 0,3 Gew.-% eingesetzt. Weiterhin haben auch zahlreiche Riechstoffe antimikrobielle Eigenschaften. Dementsprechend werden derartige Riechstoffe mit antimikrobiellen Eigenschaften in Deodorantien bevorzugt eingesetzt. Insbesondere sind hier Farnesol und Phenoxyethanol zu nennen. Daher ist es insbesondere bevorzugt, wenn die erfindungsgemäßen Deodorantien solche selbst bakteriostatisch wirksamen Riechstoffe enthalten. Dabei können die Riechstoffe vorzugsweise wieder in Form von Kieselsäureestern enthalten sein. Es ist jedoch auch möglich, daß gerade diese antibakteriell wirksamen Riechstoffe nicht in Form von Kieselsäureestern eingesetzt werden und dann in Mischungen mit anderen Riechstoffen, die als Kieselsäureester vorliegen, eingesetzt sind. Eine weitere Gruppe wesentlicher Inhaltsstoffe von Deodorantien sind Enzyminhibitoren, die die Zersetzung des Schweißes durch Enzyme hemmen, wie beispielsweise Citronensäuretriethylester oder Zinkglycinat. Wesentliche Inhaltsstoffe von Deodorantien sind weiterhin auch Antioxidantien, die eine Oxidation der Schweißbestandteile verhindern sollen.

In einer weiteren ebenfalls bevorzugten Ausführungsform der Erfindung handelt es sich bei dem kosmetischen Mittel um ein Haarfestlegemittel, das zur Festigung Polymere enthält. Besonders bevorzugt ist es dabei, wenn unter den Polymeren mindestens ein Polyurethan enthalten ist.

Dabei können die erfindungsgemäßen Mittel in einer bevorzugten Ausführungsform wasserlösliche Polymere aus der Gruppe der nichtionischen, anionischen, amphoteren und zwitterionischen Polymeren enthalten.

Als wasserlösliche Polymere sind dabei solche Polymeren zu verstehen, die bei Raumtemperatur in Wasser zu mehr als 2,5 Gew.-% löslich sind.

Erfindungsgemäß bevorzugte wasserlösliche Polymere sind nichtionisch. Geeignete nichtionogene Polymere sind beispielsweise:

- Polyvinylpyrrolidone, wie sie beispielsweise unter der Bezeichnung Luviskol$^{R}$ (BASF) vertrieben werden. Polyvinylpyrrolidone sind bevorzugte nichtionische Polymere im Rahmen der Erfindung.

- Vinylpyrrolidon/Vinylester-Copolymere, wie sie beispielsweise unter dem Warenzeichen Luviskol$^{R}$ (BASF) vertrieben werden. Luviskol$^{R}$ VA 64 und Luviskol$^{R}$ VA 73, jeweils Vinylpyrrolidon/Vinylacetat-Copolymere, sind besonders

bevorzugte nichtionische Polymere.

- Celluloseether, wie Hydroxypropylcellulose, Hydroxyethylcellulose und Methylhydroxypropylcellulose, wie sie beispielsweise unter den Warenzeichen Culminal$^R$ und Benecel$^R$ (AQUALON) vertrieben werden.

Geeignete amphotere Polymere sind beispielsweise die unter den Bezeichnungen Amphomer$^R$ und Amphomer$^R$ LV-71 (DELFT NATIONAL) erhältlichen Octylacrylamid/Methylmethacrylat/tert. Butylaminoethylmethacrylat/2-Hydroxypropylmethacrylat-Copolymere.

Geeignete zwitterionische Polymere sind beispielsweise die in den deutschen Patentanmeldungen DE 39 29 973, DE 21 50 557, DE 28 17 369 und DE 37 08 451 offenbarten Polymerisate. Acrylamidopropyltrimethylammoniumchlorid/Acrylsäurebzw. -Methacrylsäure-Copolymerisate und deren Alkali- und Ammoniumsalze sind bevorzugte zwitterionische Polymere. Weiterhin geeignete zwitterionische Polymere sind Methacroylethylbetain/Methacrylat-Copolymere, die unter der Bezeichnung Amersette$^R$ (AMERCHOL) im Handel erhältlich sind.

Erfindungsgemäß geeignete anionische Polymere sind u. a.:

- Vinylacetat/Crotonsäure-Copolymere, wie sie beispielsweise unter den Bezeichnungen Resyn$^R$ (NATIONAL STARCH), Luviset$^R$ (BASF) und Gafset$^R$ (GAF) im Handel sind.

- Vinylpyrrolidon/Vinylacrylat-Copolymere, erhältlich beispielsweise unter dem Warenzeichen Luviflex$^R$ (BASF). Ein bevorzugtes Polymer ist das unter der Bezeichnung Luviflex$^R$ VBM-35 (BASF) erhältliche Vinylpyrrolidon/Acrylat-Terpolymere.

- Acrylsäure/Ethylacrylat/N-tert.Butylacrylamid-Terpolymere, die beispielsweise unter der Bezeichnung Ultrahold$^R$ strong (BASF) vertrieben werden.

In den Fällen, in denen das Polyurethan ionische Gruppen enthält, hat es sich als zweckmäßig erwiesen, wenn weitere wasserlösliche Polymere nichtionogen oder von gleicher Ionogenität sind.

Die erfindungsgemäßen Haarbehandlungsmittel enthalten wasserlösliche Polymere in Abhängigkeit vom Typ des Haarbehandlungsmittels, der keinen Einschränkungen unterliegt, bevorzugt in Mengen von 0,01 bis 20 Gew.-%, insbesondere 0,1 bis 10 Gew.-%, bezogen auf das gesamte Mittel.

Die Polyurethane und die wasserlöslichen Polymere sind bevorzugt in einem Mengenverhältnis von 1:10 bis 10:1 in den erfindungsgemäßen Mitteln enthalten. Ein Mengenverhältnis von 2:1 bis 1:1 hat sich in vielen Fällen als besonders geeignet erwiesen.

Bei den erfindungsgemäßen Haarfestlegemitteln handelt es sich insbesondere um Haarfestiger, Haarsprays und Fönwellen. Haarsprays sind eine besonders bevorzugte Ausführungsform der erfindungsgemäßen Haarfestlegemittel.

Die erfindungsgemäßen Mittel können weiterhin in einer ebenfalls bevorzugten Ausführungsform mit Hilfe eines Treibmittels auch als Schaumaerosol formuliert sein.

Weitere Bestandteile der erfindungsgemäßen Mittel können beispielsweise sein:

- anionische Tenside, wie beispielsweise Fettalkylsulfate und -ethersulfate,
- kationische Tenside, wie beispielsweise quartäre Ammoniumverbindungen,
- zwitterionische Tenside, wie beispielsweise Betaine,
- ampholytische Tenside,
- nichtionogene Tenside, wie beispielsweise Alkylpolyglycoside und ethoxylierte Fettalkohole,
- Strukturanten wie Glucose und Maleinsäure,
- haarkonditionierende Verbindungen wie Phospholipide, beispielsweise Sojalecithin, Ei-Lecithin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, die die erfindungsgemäßen und/oder erfindungsgemäß zu verwendenden Kieselsäureester enthalten,
- Lösungsvermittler, wie Ethylenglykol, Propylenglykol, Glycerin und Diethylenglykol,
- Farbstoffe,
- Antischuppenwirkstoffe wie Piroctone Olamine und Zink Omadine,
- weitere Substanzen zur Einstellung des pH-Wertes,
- Wirkstoffe wie Panthenol, Allantoin, Pyrrolidoncarbonsäuren und deren Salze, Pflanzenextrakte und Vitamine,
- Lichtschutzmittel,
- Konsistenzgeber wie Zuckerester, Polyolester oder Polyolalkylether,

- Fette und Wachse, wie Walrat, Bienenwachs, Montanwachs, Paraffine und Fettalkohole,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Treibmittel wie Propan-Butan-Gemische, $N_2O$, Dimethylether, $CO_2$ und Luft sowie
- Antioxidantien.

Vorzugsweise enthalten die erfindungsgemäßen Haarfestlegemittel wie bereits erwähnt, auch Polymere aus der Klasse der Polyurethane. Die Polyurethane bestehen aus mindestens zwei verschiedenen Monomertypen,

- einer Verbindung (A) mit mindestens 2 aktiven Wasserstoffatomen pro Molekül und
- einem Di- oder Polyisocyanat (B).

Bei den Verbindungen (A) kann es sich beispielsweise um Diole, Triole, Diamine, Triamine, Polyetherole und Polyesterole handeln. Dabei werden die Verbindungen mit mehr als 2 aktiven Wasserstoffatomen üblicherweise nur in geringen Mengen in Kombination mit einem großen Überschuß an Verbindungen mit 2 aktiven Wasserstoffatomen eingesetzt.

Beispiele für Verbindungen (A) sind Ethylenglykol, 1,2- und 1,3-Propylenglykol, Butylenglykole, Di-, Tri-, Tetra- und Poly-Ethylen- und -Propylenglykole, Copolymere von niederen Alkylenoxiden wie Ethylenoxid, Propylenoxid und Butylenoxid, Ethylendiamin, Propylendiamin, 1,4-Diaminobutan, Hexamethylendiamin und $\alpha,\omega$-Diamine auf Basis von langkettigen Alkanen oder Polyalkylenoxiden.

Polyurethane, bei denen die Verbindungen (A) Diole, Triole und Polyetherole sind, können erfindungsgemäß bevorzugt sein. Insbesondere Polyethylenglykole und Polypropylenglykole mit Molmassen zwischen 200 und 3000, insbesondere zwischen 1600 und 2500, haben sich in einzelnen Fällen als besonders geeignet erwiesen.

Polyesterole werden üblicherweise durch Modifizierung der Verbindung (A) mit Dicarbonsäuren wie Phthalsäure, Isophthalsäure und Adipinsäure erhalten.

Als Verbindungen (B) werden überwiegend Hexamethylendiisocyanat, 2,4- und 2,6-Toluoldiisocyanat, 4,4'-Methylendi(phenylisocyanat) und insbesondere Isophorondiisocyanat eingesetzt.

Weiterhin können die erfindungsgemäß verwendeten Polyurethane noch Bausteine wie beispielsweise Diamine als Kettenverlängerer und Hydroxycarbonsäuren enthalten. Dialkylolcarbonsäuren wie beispielsweise Dimethylolpropionsäure sind besonders geeignete Hydroxycarbonsäuren. Hinsichtlich der weiteren Bausteine besteht keine grundsätzliche Beschränkung dahingehend, ob es sich um nichtionische, anionischen oder kationische Bausteine handelt.

Bezüglich weiterer Informationen über den Aufbau und die Herstellung der Polyurethane wird ausdrücklich auf die Artikel in den einschlägigen Übersichtswerken wie Römpps Chemie-Lexikon und Ullmanns Enzyklopädie der technischen Chemie Bezug genommen.

Als in vielen Fälle erfindungsgemäß besonders geeignet haben sich Polyurethane erwiesen, die wie folgt charakterisiert werden können:

- ausschließlich aliphatische Gruppen im Molekül
- keine freien Isocyanatgruppen im Molekül
- Polyether- und Polyesterpolyurethane
- anionische Gruppen im Molekül.

Es hat sich ebenfalls in einigen Fällen als vorteilhaft erwiesen, wenn das Polyurethan in dem System nicht gelöst, sondern stabil dispergiert ist.

Weiterhin hat es sich als für die Herstellung der erfindungsgemäßen Mittel als vorteilhaft erwiesen, wenn die Polyurethane nicht direkt mit den weiteren Komponenten gemischt, sondern in Form von wäßrigen Dispersionen eingebracht wurden. Solche Dispersionen weisen üblicherweise einen Feststoffgehalt von ca. 20-50 %, insbesondere etwa 35-45% auf und sind auch kommerziell erhältlich.

Die erfindungsgemäßen Haarfestlegemittel enthalten das Polyurethan vorzugsweise in Mengen von 0,1 bis 15 Gew.-%, insbesondere 0,5 bis 10 Gew.-%, bezogen auf das gesamte Mittel.

**Beispiele:**

**A Wäschebehandlungsmittel/Waschmittel:**

Es wurden 15 Parfdmöle mit den in den Tabellen 1 bis 3 angegebenen Zusammensetzungen hergestellt. Die Parfümöle 1a, 2a und 3a enthielten dabei drei Duftstoffalkohole in Form ihrer erfindungsgemäßen Kieselsäureester, während die Vergleichsbeispiele 1b, 2b und 3b gleiche Mengen an den freien Duftstoffalkoholen enthielten, also frei von den erfindungsgemäßen Verbindungen waren. Die Beispiele 1c, 1d, 1e, 2c, 2d, 2e, 3c, 3d und 3e enthielten jeweils zwei Duftstoffalkohole und einen erfindungsgemäßen Duftstoffalkohol-Kieselsäureester. Die Zusammensetzung der Parfümöle (‰, bezogen auf gesamtes Parfümöl) ist in den Tabellen 1 bis 3 angegeben:

Tabelle 1:

| Parfümöle mit blumig, ozoniger Duftnote (Anteile in ‰) | | | | | |
|---|---|---|---|---|---|
| **Einsatzstoff** | **Formel 1a** | **Formel 1b** | **Formel 1c** | **Formel 1d** | **Formel 1e** |
| Ethylenbrassilat | 180 | 180 | 180 | 180 | 180 |
| ISO E Super | 135 | 135 | 135 | 135 | 135 |
| Hedione | 130 | 130 | 130 | 130 | 130 |
| Cyclohexylsalicylat Henkel | 100 | 100 | 100 | 100 | 100 |
| Lilial | 80 | 80 | 80 | 80 | 80 |
| Dihydro-Beta-Jonon | 60 | 60 | 60 | 60 | 60 |
| Troenan Henkel | 60 | 60 | 60 | 60 | 60 |
| **Phenylethylkieselsäureester** | **45** | **-** | **-** | **-** | **45** |
| **Phenylethylalkohol** | **-** | **45** | **45** | **45** | **-** |
| **Geranylkieselsäureester** | **40** | **-** | **-** | **40** | **-** |
| **Geraniol** | **-** | **40** | **40** | **-** | **40** |
| **Citronellylkieselsäureester** | **40** | **-** | **40** | **-** | **-** |
| **Citronellol** | **-** | **40** | **-** | **40** | **40** |
| Linalool | 37 | 37 | 37 | 37 | 37 |
| Helional | 34 | 34 | 34 | 34 | 34 |
| Eugenol rein | 10 | 10 | 10 | 10 | 10 |
| Canthoxal | 8 | 8 | 8 | 8 | 8 |
| Calone | 7 | 7 | 7 | 7 | 7 |
| Cyclovertal Henkel | 6 | 6 | 6 | 6 | 6 |
| Dimetol | 5 | 5 | 5 | 5 | 5 |
| Methylanthranilat 10% in DPG | 5 | 5 | 5 | 5 | 5 |
| Decalacton Gamma | 4 | 4 | 4 | 4 | 4 |
| Phenylessigsäure | 3 | 3 | 3 | 3 | 3 |
| Damascenone 10% in DPG | 3 | 3 | 3 | 3 | 3 |
| Neroli Phase Oil | 3 | 3 | 3 | 3 | 3 |
| Cyclogalbanat | 2 | 2 | 2 | 2 | 2 |
| Indol | 1 | 1 | 1 | 1 | 1 |
| Isoeugenolmethylether | 1 | 1 | 1 | 1 | 1 |
| Ambroxan Henkel | 1 | 1 | 1 | 1 | 1 |

Tabelle 2:

| Parfümöle mit frischer, rosiger Duftnote (Anteile in ‰) | | | | | |
|---|---|---|---|---|---|
| **Einsatzstoff** | **Formel 2a** | **Formel 2b** | **Formel 2c** | **Formel 2d** | **Formel 2e** |
| Hexylzimtaldehyd (Alpha) | 170 | 170 | 170 | 170 | 170 |
| Lilial | 170 | 170 | 170 | 170 | 170 |

Tabelle 2: (fortgesetzt)

| Parfümöle mit frischer, rosiger Duftnote (Anteile in ‰) | | | | | |
|---|---|---|---|---|---|
| **Einsatzstoff** | **Formel 2a** | **Formel 2b** | **Formel 2c** | **Formel 2d** | **Formel 2e** |
| Ethyllinalool | 152 | 152 | 152 | 152 | 152 |
| **Citronellylkieselsäureester** | **106** | **-** | **-** | **-** | **106** |
| **Citronellol** | **-** | **106** | **106** | **106** | **-** |
| Ethylenbrassilat | 80 | 80 | 80 | 80 | 80 |
| Benzylacetat | 41 | 41 | 41 | 41 | 41 |
| Cyclohexylsalicylat Henkel | 40 | 40 | 40 | 40 | 40 |
| Citronellylacetat | 40 | 40 | 40 | 40 | 40 |
| Acetessigester | 34 | 34 | 34 | 34 | 34 |
| **Geranylkieselsäureester** | **30** | **-** | **-** | **30** | **-** |
| **Geraniol** | **-** | **30** | **30** | **-** | **30** |
| **Phenylethylkieselsäureester** | **28** | **-** | **28** | **-** | **-** |
| **Phenylethylalkohol** | **-** | **28** | **-** | **28** | **28** |
| Geraniumöl Bourbon | 20 | 20 | 20 | 20 | 20 |
| Linalool | 14 | 14 | 14 | 14 | 14 |
| Isoraldein 70 | 10 | 10 | 10 | 10 | 10 |
| Indoflor | 5 | 5 | 5 | 5 | 5 |
| Ethylvanillin 10 % in DPG | 5 | 5 | 5 | 5 | 5 |
| Rosenoxid R 10% in DPG | 5 | 5 | 5 | 5 | 5 |
| Muguet-Aldehyd 100 % | 5 | 5 | 5 | 5 | 5 |
| Styrolylacetat | 5 | 5 | 5 | 5 | 5 |
| Cuminaldehyd 10 % in DPG | 5 | 5 | 5 | 5 | 5 |
| Calone 10 % in DPG | 5 | 5 | 5 | 5 | 5 |
| Phenylacetalaldehyddimethylacetal | 5 | 5 | 5 | 5 | 5 |
| Cyclovertal 10 % in DPG Henkel | 5 | 5 | 5 | 5 | 5 |
| Petitgrainöl Parag. | 5 | 5 | 5 | 5 | 5 |
| Ethylphenylacetat | 4 | 4 | 4 | 4 | 4 |
| Hexenylacetat | 3 | 3 | 3 | 3 | 3 |
| Hexenol (Beta Gamma) | 3 | 3 | 3 | 3 | 3 |
| Hydratropaald. Dim. Acetal | 2 | 2 | 2 | 2 | 2 |
| Phenylethylphenylacetat | 1 | 1 | 1 | 1 | 1 |
| Cyclogalbanat | 1 | 1 | 1 | 1 | 1 |
| Ambroxan Henkel | 1 | 1 | 1 | 1 | 1 |

Tabelle 3:

| Parfümöle mit frischer, grüner Duftnote (Anteile in ‰) | | | | | |
|---|---|---|---|---|---|
| **Einsatzstoff** | **Formel 3a** | **Formel 3b** | **Formel 3c** | **Formel 3d** | **Formel 3e** |
| Hexylzimtaldehyd (Alpha) | 274 | 274 | 274 | 274 | 274 |
| Dipropylenglycol | 200 | 200 | 200 | 200 | 200 |
| **Pheaylethylkieselsäureester** | **103** | **-** | **-** | **-** | **103** |
| **Phenylethylalkohol** | **-** | **103** | **103** | **103** | **-** |
| Bergamotteöl Berg.-frei | 100 | 100 | 100 | 100 | 100 |
| Cyclohexylsalicylat Henkel | 98,5 | 98,5 | 98,5 | 98,5 | 98,5 |
| Orangenöl süss. ital. | 42 | 42 | 42 | 42 | 42 |
| Ethylenbrassilat | 41 | 41 | 41 | 41 | 41 |
| **Geranylkieselsäureester** | **35** | **-** | **-** | **35** | **-** |

Tabelle 3: (fortgesetzt)

| Parfümöle mit frischer, grüner Duftnote (Anteile in ‰) | | | | | |
|---|---|---|---|---|---|
| **Einsatzstoff** | **Formel 3a** | **Formel 3b** | **Formel 3c** | **Formel 3d** | **Formel 3e** |
| **Geraniol** | **-** | **35** | **35** | **-** | **35** |
| **Citronellylkieselsäureester** | **24** | **-** | **24** | **-** | **-** |
| **Citronellol** | **-** | **24** | **-** | **24** | **24** |
| Linalylacetat | 20 | 20 | 20 | 20 | 20 |
| Litsea-Cubeba-Öl | 15 | 15 | 15 | 15 | 15 |
| Linalool | 12 | 12 | 12 | 12 | 12 |
| Pinen Beta P&F | 10 | 10 | 10 | 10 | 10 |
| Jonon Beta Synth. | 8 | 8 | 8 | 8 | 8 |
| Petitgrainöl Parag. | 7 | 7 | 7 | 7 | 7 |
| Muskateller Salbeiöl | 3 | 3 | 3 | 3 | 3 |
| Cyclovertal Henkel | 3 | 3 | 3 | 3 | 3 |
| Damascone Beta | 2 | 2 | 2 | 2 | 2 |
| Allyljonon (Cetone V) | 1 | 1 | 1 | 1 | 1 |
| Cedernblätteröl | 1 | 1 | 1 | 1 | 1 |
| Eucalyptol | 0,5 | 0,5 | 0,5 | 0,5 | 0,5 |

Die Parfümöle 1a und 1b wurden zu 1% in ein Weichspülerkonzentrat mit 15 % Esterquat eingearbeitet, dessen Zusammensetzung in Tabelle 4 angegeben ist. Die mit parfümfreiem Universalwaschmittel gewaschene Wäsche wurde mit jeweils 40g dieser Weichspüler im letzten Spülgang behandelt. Nach dem Schleudern wurde der Duft der feuchten Wäsche beurteilt, danach die Wäsche zum Trocknen auf die Leine gehängt. Der Duft der trockenen Wäsche wurde nach dem Abnehmen von der Leine beurteilt und jeweils nach 3 bzw. 7 Tagen, wobei die Wäsche in Plastikbeuteln gelagert wurde.

Beurteilt wurden die Wäschepaare hinsichtlich Intensität = I und Gefallen = G von 8 Fachleuten (Parfümeuren) im direkten Vergleich. Die Ergebnisse dieser Präferenztests sind in Tabelle 5 angegeben. Analoge Versuche wurden für den Vergleich der Parfümöle 1b/1d sowie 2b/2e durchgeführt. Die Ergebnisse dieser Untersuchungen zeigen die Tabellen 6 und 7.

Tabelle 4:

| Zusammensetzung des Weichspüler-Konzentrats [Gew.-%]: | |
|---|---|
| Dehyquart® AU 46 | 15 |
| Parfümöl | 1 |
| Wasser,Salze | Rest |
| Dehyquart® AU46 : Dipalmitoleyloxyethyl-hydroxyethyl-methylammonium-methoxy-sulfat, 90 %-ig in Isopropanol, Handelsprodukt der Firma Henkel, Düsseldorf | |

Tabelle 5:

| Ergebnisse der Geruchstests weichgespülter Wäsche (Präferenz der Intensität: I, Präferenz des Gefallens: G), Panel aus 8 Parfümeuren, Parfümöl 1a/1b | | | | | | | | |
|---|---|---|---|---|---|---|---|---|
| | **feuchte Wäsche** | | **trockene Wäsche** | | | | | |
| | | | **1.Tag** | | **3.Tag** | | **7.Tag** | |
| | **I** | **G** | **I** | **G** | **I** | **G** | **I** | **G** |
| 1a | 4 | 6 | 5 | 7 | 7 | 6 | 8 | 8 |
| 1b | 4 | 2 | 3 | 1 | 1 | 2 | 0 | 0 |

Tabelle 6:

| Ergebnisse der Geruchstests weichgespülter Wäsche (Präferenz der Intensität: I, Präferenz des Gefallens: G), Panel aus 8 Parfümeuren, Parfümöl 1d/1b | | | | | | | | |
|---|---|---|---|---|---|---|---|---|
| | **feuchte Wäsche** | | **trockene Wäsche** | | | | | |
| | | | **1.Tag** | | **3.Tag** | | **7.Tag** | |
| | **I** | **G** | **I** | **G** | **I** | **G** | **I** | **G** |
| 1d | 3 | 4 | 6 | 6 | 8 | 5 | 8 | 6 |
| 1b | 5 | 4 | 2 | 2 | 0 | 3 | 0 | 2 |

Tabelle 7:

| Ergebnisse der Geruchstests weichgespülter Wäsche (Präferenz der Intensität: I, Präferenz des Gefallens: G), Panel aus 8 Parfümeuren, Parfümöl 2e/2b | | | | | | | | |
|---|---|---|---|---|---|---|---|---|
| | **feuchte Wäsche** | | **trockene Wäsche** | | | | | |
| | | | **1.Tag** | | **3.Tag** | | **7.Tag** | |
| | **I** | **G** | **I** | **G** | **I** | **G** | **I** | **G** |
| 2e | 5 | 6 | 4 | 5 | 7 | 7 | 8 | 7 |
| 2b | 3 | 2 | 4 | 3 | 1 | 1 | 0 | 1 |

Ein handelsübliches parfümfreies Waschmittel wurde mit 0,25 Gew.-% der Parfümöle 3a und 3b versetzt. Mit jeweils 150g dieser Pulver wurde die Wäsche bei 60° gewaschen und 3x mit klarem Wasser nachgespült. Nach dem Schleudern wurde der Duft der feuchten Wäsche beurteilt, danach die Wäsche auf der Leine getrocknet. Der Duft der trockenen Wäsche wurde sofort, nach 3 sowie nach 7 Tagen beurteilt, wobei die Wäsche in Plastikbeuteln gelagert wurde.

Beurteilt wurden die Wäschepaare im direkten Vergleich hinsichtlich Duftintensität = I und Duftgefallen = G von 7 Fachleuten (Parfümeuren). Die Ergebnisse dieser Tests zeigt Tabelle 8, analoge Verleiche der Parfümöle 3e/3b sind in Tabelle 9 aufgeführt.

Tabelle 8:

| Ergebnisse der Geruchstests der behandelten Wäsche (Präferenz der Intensität: I, Präferenz des Gefallens: G), Panel aus 7 Parfümeuren, Parfümöl 3a/3b | | | | | | | | |
|---|---|---|---|---|---|---|---|---|
| | **feuchte Wäsche** | | **trockene Wäsche** | | | | | |
| | | | **1.Tag** | | **3.Tag** | | **7.Tag** | |
| | **I** | **G** | **I** | **G** | **I** | **G** | **I** | **G** |
| 3a | 6 | 5 | 4 | 5 | 6 | 7 | 6 | 6 |
| 3b | 1 | 2 | 3 | 2 | 1 | 0 | 1 | 1 |

Tabelle 9:

| Ergebnisse der Geruchstests der behandelten Wäsche (Präferenz der Intensität: I, Präferenz des Gefallens: G), Panel aus 7 Parfümeuren, Parfümöl 3e/3b | | | | | | | | |
|---|---|---|---|---|---|---|---|---|
| | **feuchte Wäsche** | | **trockene Wäsche** | | | | | |
| | | | **1.Tag** | | **3.Tag** | | **7.Tag** | |
| | **I** | **G** | **I** | **G** | **I** | **G** | **I** | **G** |
| 3e | 5 | 5 | 4 | 3 | 6 | 5 | 7 | 6 |
| 3b | 2 | 2 | 3 | 4 | 1 | 2 | 0 | 1 |

Die Ergebnisse zeigen deutlich, daß schon ein Teilersatz der Duftstoffalkohole durch die erfindungsgemäßen Kieselsäureester zu deutlich verbesserten Geruchsbewertungen führt.

**B Reinigungsmittel:**

**B1 Saurer Reiniger**

Es wurden Parfümöle mit Zusammensetzungen gemäß Tabelle 10 hergestellt. Dabei handelt es sich bei 10A um ein erfindungsgemäßes Parfümöl, bei 10B um ein Vergleichsbeispiel.

Tabelle 10:

| Parfümöle für einen sauren Reiniger mit einem Apfelduft | | |
|---|---|---|
| Einsatzstoff | 10A Anteile in ‰ | 10B Anteile in ‰ |
| Dynascone 10 | 5,0 | 5,0 |
| Cyclovertal | 7,5 | 7,5 |
| Hexylacetat | 35,0 | 35,0 |
| Allylheptanoat | 200,0 | 200,0 |
| Amylbutyrat | 5,0 | 5,0 |
| Prenylacetate | 10,0 | 10,0 |
| Aldehyd C 14 SOG | 70,0 | 70,0 |
| Manzanate | 15,0 | 15,0 |
| Melusat | 30,0 | 30,0 |
| Ortho tert Butylcyclohexylacetat | 200,0 | 200,0 |
| Zimtaldehyd | 5,0 | 5,0 |
| Isobornylacetat | 10,0 | 10,0 |
| Dihydrofloriffone TD | 2,5 | 2,5 |
| Floramat | 100,0 | 100,0 |
| Phenylethylalkohol | 30,0 | 30,0 |
| **Geranylkieselsäureester** | **105,0** | **-** |
| **Geraniol** | **-** | **105,0** |
| Cyclohexylsalicylat | 150,0 | 150, |
| Citronellol | 20,0 | 20,0 |

Diese Parfümöle wurden in ein saures Reinigungsmittel mit einer Rezeptur gemäß Tabelle 11 eingearbeitet. Mit dem parfümhaltigen Mittel wurde dann ein Badezimmer (Badewanne und Waschbecken) gereinigt. Anschließend wurde der Dufteindruck der gereinigten Oberfläche von 8 Duftexperten (Parfümeuren) hinsichtlich Intensität und Gefallen nach folgender Skala beurteilt:

| Intensität | Gefallen |
|---|---|
| 1= kein Duft<br>2= etwas Duft<br>3= mittelstarker Duft<br>4= starker Duft<br>5= sehr starker Duft | 1= sehr schlecht<br>2= schlecht<br>3= mäßig<br>4= mittel<br>5= gut<br>6= sehr gut<br>7= hervorragend |

Tabelle 11:

| Rezeptur des sauren Reinigers [Gew.-%] | |
|---|---|
| ABS-Säure | 4,0 |
| Fettalkoholethoxylat (EO=7) | 2,0 |
| KOH | 5,5 |
| Citronensäure x 1H20 | 10,5 |
| Soda kalz. | 0,05 |
| Glutaraldehyd | 1,5 |
| Parfüm | 1,5 |
| VE-Wasser entkeimt | Rest |
| pH-Wert | 4,4 |

In Tabelle 12 sind die Ergebnisse der Geruchstests angegeben. Direkt danach und auch noch nach 3 Stunden liefern beide Parfümöle vergleichbare Eindrücke. Bereits nach 6 Stunden und erst recht nach 24 Stunden war beim erfindungsgemäßen Mittel 10A ein deutlich intensiverer Duft wahrnehmbar, der zusätzlich auch angenehmer auf die Duftexperten wirkte.

Tabelle 12:

| Ergebnis des Geruchstests nach Reinigung von Badewannen / Waschbecken | | | | |
|---|---|---|---|---|
| | Parfümöl 10 A | | Parfümöl 10 B | |
| | Intensität | Gefallen | Intensität | Gefallen |
| frisch | 4,5 | 4,6 | 4,7 | 4,5 |
| nach 3 Stunden | 4,6 | 4,5 | 4,4 | 4,5 |
| nach 6 Stunden | 4,4 | 4,6 | 3,8 | 4,3 |
| nach 24 Stunden | 3,1 | 3,7 | 1,8 | 3,1 |

**B2 Allzweckreiniger**

In einem Allzweckreiniger mit einer Rezeptur gemäß Tabelle 13, bei dem es sich um einen 2-Phasen-Reiniger handelte, wurden Parfümöle mit Rezepturen gemäß Tabelle 14 eingearbeitet. Bei 14A handelt es sich um das erfindungsgemäße Beispiel bei 14B um das Vergleichsbeispiel. Mit diesen Reinigern wurde ein Fußboden gereinigt und anschließend der Dufteindruck ebenso wie bei B1 von 8 Parfümeuren beurteilt. Die Ergebnisse in Tabelle 15 zeigen auch hier direkt nach dem Reinigen und 3 Stunden danach noch vergleichbare Eindrücke, nach 6 Stunden jedoch bereits beim erfindungsgemäßen Beispiel die höhere Intensität und auch einen angenehmeren Eindruck. Nach 24 Stunden werden die Vorteile des erfindungsgemäßen Mittels dann besonders deutlich.

Tabelle 13:

| Rezepturen des Allzweckreinigers (2-Phasen-Reiniger); Angaben in [Gew.-%] | |
|---|---|
| ABS-Säure | 4 |
| Fettalkoholethoxylat (EO=7) | 2 |
| KOH | 4,5 |
| Citronensäure x1H2O | 4,75 |
| Cetiol OE | 4,5 |
| Soda kalz. | 0,2 |
| Glutaraldehyd | 0,05 |

Tabelle 13: (fortgesetzt)

| Rezepturen des Allzweckreinigers (2-Phasen-Reiniger); Angaben in [Gew.-%] | |
|---|---|
| Parfüm | 1,6 |
| VE-Wasser entkeimt | Rest |
| pH-Wert | 10 |

Tabelle 14:

| Parfümöle für einen Allzweckreiniger mit einer Zitronenduftnote | | |
|---|---|---|
| Einsatzstoff | 14A Anteile in ‰ | 14B Anteile in ‰ |
| Dihydromyrcenol | 30,0 | 30,0 |
| Aldinyle 3881 | 5,0 | 5,0 |
| Citral AR | 15,0 | 15,0 |
| Geranonitril | 56,0 | 56,0 |
| Tridecene-2-Nitril 10% in DPG | 3,0 | 3,0 |
| Methylpamplemousse | 5,0 | 5,0 |
| Vertacetal | 2,0 | 2,0 |
| Citronellal | 10,0 | 10,0 |
| Citrathal | 10,0 | 10,0 |
| Orangenoel destilliert weiss | 480,0 | 480,0 |
| Methylnaphthylketon kristallisiert | 5,0 | 5,0 |
| Aldehyd C 08 | 10,0 | 10,0 |
| Aldehyd C 09 | 6,0 | 6,0 |
| Aldehyd C 10 | 15,0 | 15,0 |
| Cyclovertal | 7,0 | 7,0 |
| Hexenol (Beta Gamma) | 1,0 | 1,0 |
| Hexylacetat | 6,0 | 6,0 |
| Campher synthetisch | 11,0 | 11,0 |
| Pineoel französisch 70 | 30,0 | 30,0 |
| Carvon L | 1,0 | 1,0 |
| Styrolylacetat | 6,0 | 6,0 |
| Linalool | 30,0 | 30,0 |
| Phenylethylalkohol | 20,0 | 20,0 |
| Citronellol | 25,0 | 25,0 |
| Geranylacetat | 3,0 | 3,0 |
| **Geranylkieselsäureester** | **127,0** | **-** |
| **Geraniol** | **-** | **127,0** |
| Nerylacetat | 1,0 | 1,0 |
| Cyclohexylsalicylat | 70,0 | 70,0 |
| Sandelice | 5,0 | 5,0 |
| Boisambrene Forte | 3,0 | 3,0 |

Tabelle 14: (fortgesetzt)

| Parfümöle für einen Allzweckreiniger mit einer Zitronenduftnote | | |
|---|---|---|
| Einsatzstoff | 14A Anteile in ‰ | 14B Anteile in ‰ |
| Ethylvanillin 10% in DPG | 2,0 | 2,0 |

Tabelle 15:

| Ergebnis des Geruchstests nach Reinigung von Fußböden | | | | |
|---|---|---|---|---|
| | Parfümöl 14 A | | Parfümöl 14 B | |
| | Intensität | Gefallen | Intensität | Gefallen |
| frisch | 4,2 | 4,3 | 4,3 | 4,3 |
| nach 3 Stunden | 4,1 | 4,1 | 4,0 | 4,2 |
| nach 6 Stunden | 3,6 | 4,0 | 3,2 | 3,6 |
| nach 24 Stunden | 2,9 | 3,1 | 1,2 | 2,2 |

**C Duschbad**

Als Beispiel für ein kosmetisches Hautbehandlungsmittel dient im folgenden ein Duschbad. In Tabelle 16 ist die Parfümölzusammensetzung angegeben, die in dem Duschbad mit einer Rezeptur gemäß Tabelle 17 eingesetzt wurde. Bei 16A handelt es sich um die erfindungsgemäße Rezeptur, bei 16B um das Vergleichsbeispiel. Mit dem Duschbad wurden die Unterarme eines Probanden gereinigt und der Duft von 8 Duftexperten nach der unter B1 angegebenen Skala beurteilt. Wie auch bei den Reinigungsmitteln beobachtet, ist der Dufteindruck mit dem erfindungsgemäßen Mittel wesentlich länger anhaltend und nach längerer Zeit auch angenehmer in der Wahrnehmung.

Tabelle 16:

| Parfümöle mit einer frischen, blumigen Duftnote | | |
|---|---|---|
| Einsatzstoff | 16A Anteile in ‰ | 16B Anteile in ‰ |
| Bergamotteöl | 250,0 | 250,0 |
| Citronenöl Messina | 50,0 | 50,0 |
| Citronellal | 2,0 | 2,0 |
| Orangenöl süss | 50,0 | 50,0 |
| Lavendelöl | 50,0 | 50,0 |
| Terpineol | 50,0 | 50,0 |
| Lilial | 100,0 | 100,0 |
| Phenylethylalkohol | 80,0 | 80,0 |
| **Citronellylkieselsäureester** | **100,0** | **-** |
| **Citronellol** | **-** | **100,0** |
| Geraniol | 20,0 | 20,0 |
| Benzylacetat | 60,0 | 60,0 |
| Isoraldein 70 | 50,0 | 50,0 |
| Ylang | 30,0 | 30,0 |
| Ambroxan 10% in IPM | 1,0 | 1,0 |
| Heliotropin | 47,0 | 47,0 |
| Habanolide | 60,0 | 60,0 |

Tabelle 17:

| Zusammensetzung des Duschbads | |
|---|---|
| **Inhaltsstoffe** | **[Gew.-%]** |
| $C_{12-14}$-Fettalkohol 2 EO-sulfat | 8,0 |
| $C_{12-14}$-Fettalkoholsulfosuccinat | 4,0 |
| Kokosamidbetain | 2,0 |
| Kokosfettsäuremonoglycerid 7 EO | 3,0 |
| Glycerinmonolaurat | 2,0 |
| Eiweißhydrolysat | 1,0 |
| Ethylenglykoldistearat | 1,0 |
| Parfümöl | 1,0 |
| Wasser | Rest |

Tabelle 18:

| Ergebnis des Geruchstests nach Reinigung der Unterarme mit dem Duschbad | | | | |
|---|---|---|---|---|
| | Parfümöl 16 A | | Parfümöl 16 B | |
| | Intensität | Gefallen | Intensität | Gefallen |
| frisch | 4,2 | 5,1 | 4,4 | 5,2 |
| nach 2 Stunden | 3,8 | 4,8 | 3,9 | 4,9 |
| nach 5 Stunden | 3,5 | 4,5 | 3,3 | 4,2 |
| nach 8 Stunden | 2,5 | 4,0 | 1,8 | 3,5 |

**D Seife**

Ein Stück Seife wurde mit einer Zusammensetzung entsprechend Tabelle 20 hergestellt. Sie enthielt im erfindungsgemäßen Beispiel die Duftzusammensetzung 19A, im Vergleichsbeispiel die Duftzusammensetzung 19B (Tabelle 19). Nach Reinigung der Unterarme eines Probanden wurde auch hier der Duft von 8 Parfümeuren beurteilt. Es wurden wiederum Intensität des Dufteindrucks und Gefallen des Dufteindrucks individuell beurteilt. Die gemittelten Urteile sind in Tabelle 21 angegeben. Auch in diesem Fall war der Dufteindruck direkt nach dem Waschen bzw. 2 Stunden danach noch vergleichbar, nach 5 Stunden und erst recht nach 8 Stunden zeigte sich jedoch wiederum die Überlegenheit des erfindungsgemäßen Beispiels.

Tabelle 19:

| Parfümöle mit einer frischen, blumigen Duftnote | | |
|---|---|---|
| Einsatzstoff | 19A Anteile in ‰ | 19B Anteile in ‰ |
| Bergamotteöl | 250,0 | 250,0 |
| Citronenöl Messina | 50,0 | 50,0 |
| Citronellal | 2,0 | 2,0 |
| Orangenöl süss | 50,0 | 50,0 |
| Lavendelöl | 50,0 | 50,0 |
| Terpineol | 50,0 | 50,0 |
| Lilial | 100,0 | 100,0 |
| **Phenylethylalkohol** | **-** | **80,0** |

Tabelle 19: (fortgesetzt)

| Parfümöle mit einer frischen, blumigen Duftnote | | |
|---|---|---|
| Einsatzstoff | 19A Anteile in ‰ | 19B Anteile in ‰ |
| **Phenylethylkieselsäureester** | **80,0** | **-** |
| Citronellol | 100,0 | 100,0 |
| Geraniol | 20,0 | 20,0 |
| Benzylacetat | 60,0 | 60,0 |
| Isoraldein 70 | 50,0 | 50,0 |
| Ylang | 30,0 | 30,0 |
| Ambroxan 10% in IPM | 1,0 | 1,0 |
| Heliotropin | 47,0 | 47,0 |
| Habanolide | 60,0 | 60,0 |

Tabelle 20:

| Zusammensetzung der Stückseife [Gew.-%] | |
|---|---|
| Einsatzstoff | [Gew.-%] |
| Talgfettsäureseife | 60 |
| Kokosfettsäureseife | 27 |
| Galycerin | 2 |
| Parfümöl | 3 |
| Wasser | Rest |

Tabelle 21:

| Ergebnis des Geruchstests nach Reinigung der Unterarme mit der Seife aus Tabelle 20 | | | | |
|---|---|---|---|---|
| | Parfümöl 19A | | Parfümöl 19B | |
| | Intensität | Gefallen | Intensität | Gefallen |
| frisch | 4,1 | 4,7 | 4,2 | 4,7 |
| nach 2 Stunden | 3,6 | 4,3 | 3,5 | 4,1 |
| nach 5 Stunden | 3,1 | 3,8 | 2,7 | 3,4 |
| nach 8 Stunden | 2,4 | 3,2 | 1,6 | 2,6 |

**E Deodorant**

Eine Parfümölzusammensetzung für ein Deospray ist in Tabelle 22 angegeben, wobei in A wiederum das erfindungsgemäße und in B das Vergleichsbeispiel angegeben ist. Zur Durchführung des Geruchstest wurde das Deodorant auf einem Papiervlies aufgebracht, das zur Simulation von Bedingungen in einer Achselhöhle in einem Becher bei 37 °C gelagert wurde. Der Dufteindruck wird wiederum von 8 Parfümeuren hinsichtlich Intensität und Gefallen beurteilt. Die Ergebnisse sind in Tabelle 24 wiedergegeben. Wie bei allen vorangegangenen Versuchen zeigt sich, daß nach längerer Zeit (4, 5 bzw. 8 Stunden) die erfindungsgemäßen Parfümöle deutliche Vorteile hinsichtlich Gefallen des Dufts und der Intensität des verbleibenden Dufteindrucks aufweisen.

Tabelle 22:

| Parfümöle für ein Deospray mit blumigem Duft, der an Pfingstrosen erinnert | | |
|---|---|---|
| Einsatzstoff | 22A Anteile in ‰ | 22B Anteile in ‰ |
| Petitgrainöl Paraguay | 5,0 | 5,0 |
| Cyclogalbanat | 1,0 | 1,0 |
| Hexenol (Beta Gamma) | 3,0 | 3,0 |
| Hexenylacetat | 3,0 | 3,0 |
| Cyclovertal 10% in DPG | 5,0 | 5,0 |
| Phenylacetaldehyddimethylacetal | 5,0 | 5,0 |
| Calone 10% in DPG | 5,0 | 5,0 |
| Acetessigester | 35,0 | 35,0 |
| Cuminaldehyd 10% in DPG | 5,0 | 5,0 |
| Styrolylacetat | 5,0 | 5,0 |
| Bourgeonal | 6,0 | 6,0 |
| Cyclamenaldehyd extra L.G. | 4,0 | 4,0 |
| Florol 943160 | 62,0 | 62,0 |
| Hydroxycitronellal rein | 12,0 | 12,0 |
| Lilial | 37,0 | 37,0 |
| Lyral | 61,0 | 61,0 |
| Nerolidol | 4,0 | 4,0 |
| Ethyllinalool | 152,0 | 152,0 |
| Linalool | 14,0 | 14,0 |
| Rosenöl Türkei | 2,0 | 2,0 |
| Rose Wardia | 10,0 | 10,0 |
| Phenylethylalkohol | 28,0 | 28,0 |
| Citronellylacetat | 40,0 | 40,0 |
| **Citronellylkieselsäureester** | **106,0** | **-** |
| **Citronellol** | **-** | **106,0** |
| Geraniol | 10,0 | 10,0 |
| Phenylethylacetat | 1,0 | 1,0 |
| Rosenoxid R 10% in DPG | 5,0 | 5,0 |
| Geraniumöl Bourbon | 20,0 | 20,0 |
| Benzylacetat | 41,0 | 41,0 |
| Veloutone | 1,0 | 1,0 |
| Hedione | 100,0 | 100,0 |
| Hexylzimtaldehyd (Alpha) | 70,0 | 70,0 |
| Hydratropaaldehyd dim.acetal | 2,0 | 2,0 |
| Isoraldein 70 | 9,0 | 9,0 |
| Cyclohexylsalicylat | 75,0 | 75,0 |

Tabelle 22: (fortgesetzt)

| Parfümöle für ein Deospray mit blumigem Duft, der an Pfingstrosen erinnert | | |
|---|---|---|
| Einsatzstoff | 22A Anteile in ‰ | 22B Anteile in ‰ |
| Hexenylsalicylat (CIS-3) | 21,0 | 21,0 |
| Ethylvanillin 10% in DPG | 5,0 | 5,0 |
| Ethylphenylacetat | 4,0 | 4,0 |
| Phenylethylphenylacetat | 1,0 | 1,0 |
| Ambrettolide | 3,0 | 3,0 |
| Cyclopentadecanolide | 2,0 | 2,0 |
| Ethylenbrassilat | 15,0 | 15,0 |
| Indoflor | 5,0 | 5,0 |

Tabelle 23:

| Rezeptur des Deodorants [Gew.-%] | |
|---|---|
| APG 600 | 0,050 Gew.-% |
| APG 220 | 0,150 Gew.-% |
| Cetiol OE | 0,030 Gew.-% |
| Eutanol G | 0,007 Gew.-% |
| Citronensäure (krist.) | 0,005 Gew.-% |
| Parfümöl | 0,300 Gew.-% |
| Aluminiumhydroxychlorid | 20,000 Gew.-% |
| Böhmit (20%ig) | 4,000 Gew.-% |
| Wasser | 75,460 Gew.-% |
| APG 600: Plantacare 1200 UP (Henkel KGaA) Aktivsubstanz : 50 - 53 Gew.-% Alkyl-$C_{12}$-$C_{16}$-oligo(1,4)-glucosid<br>APG 220: Plantacare 220 UP (Henkel KGaA) Aktivsubstanz : 62 - 65 Gew.-% Alkyl-$C_8$-$C_{10}$-oligo(1,5)-glucosid<br>Cetiol OE: Dioctylether (Henkel KGaA)<br>Eutanol G: 2-Octyl-dodecanol (Henkel KGaA) | |

Tabelle 24:

| Ergebnis des Geruchstests nach dem Sprühen auf Papiervlies, das im Becher bei 37°C gelagert wurde. | | | | |
|---|---|---|---|---|
| | Parfümöl 22 A | | Parfümöl 22 B | |
| | Intensität | Gefallen | Intensität | Gefallen |
| frisch | 4,8 | 5,1 | 4,9 | 5,3 |
| nach 2 Stunden | 4,3 | 5,0 | 4,3 | 4,9 |
| nach 5 Stunden | 3,2 | 4,5 | 2,9 | 4,2 |
| nach 8 Stunden | 2,8 | 4,1 | 1,8 | 3,1 |

**F Haarspray**

Die Zusammensetzung 25A gibt ein erfindungsgemäßes Parfümöl für ein Haarspray wieder, 25B ein Vergleichsbeispiel dazu (Tabelle 25). Diese Duftzusammensetzungen wurden in ein Haarspray gemäß der in Tabelle 26

wiedergegebenen Rezeptur eingearbeitet und der Dufteindruck von Haarsträhnen, die mit diesem Spray besprüht wurden, von 8 Parfümeuren beurteilt. Die Ergebnisse sind in Tabelle 27 wiedergegeben, auch hier zeigt es sich, daß über längere Zeiträume die Duftintensität bei den erfindungsgemäßen Mitteln wesentlich höher bleibt als bei Verwendung von Mitteln aus dem Stand der Technik.

Tabelle 25:

| Parfümöle für ein Haarspray mit einer blumigen, frischen, würzigen Duftnote | | |
|---|---|---|
| Einsatzstoff | 25A Anteile in ‰ | 25B Anteile in ‰ |
| Linalylacetat | 28,0 | 28,0 |
| Citronenöl Messina | 44,0 | 44,0 |
| Methylpamplemousse | 0,5 | 0,5 |
| Cyclocalbanat | 0,5 | 0,5 |
| Cyclovertal | 1,0 | 1,0 |
| Hexenol (Beta Gamma) | 2,0 | 2,0 |
| Hexenylacetat | 1,0 | 1,0 |
| Precyclemone B | 5,0 | 5,0 |
| Peranat | 5,0 | 5,0 |
| Terpineol | 3,0 | 3,0 |
| Styrolylacetat | 2,0 | 2,0 |
| Lilial | 50,0 | 50,0 |
| Troenan | 27,0 | 27,0 |
| Ethyllinalool | 106,0 | 106,0 |
| Linalool | 36,0 | 36,0 |
| Helional | 40,0 | 40,0 |
| Phenylethylalkohol | 10,0 | 10,0 |
| Citronellylacetat | 5,0 | 5,0 |
| Citronellol | 35,0 | 35,0 |
| **Geranylkieselsäureester** | **100,0** | **-** |
| **Geräniol** | **-** | **100,0** |
| Geraniumöl Bourbon | 10,0 | 10,0 |
| Jasmon-Cis | 2,0 | 2,0 |
| Hedione | 157,0 | 157,0 |
| Hexyzimtaldehyd (Alpha) | 41,0 | 41,0 |
| Isoraldein 70 | 16,0 | 16,0 |
| Jonon Beta synthetisch | 14,0 | 14,0 |
| Liffarome | 1,0 | 1,0 |
| Cyclohexylsalicylat | 6,0 | 6,0 |
| Patchoulyöl | 3,0 | 3,0 |
| Sandelice | 26,0 | 26,0 |
| Iso E Super | 109,0 | 109,0 |
| Ambroxan | 1,0 | 1,0 |

Tabelle 25: (fortgesetzt)

| Parfümöle für ein Haarspray mit einer blumigen, frischen, würzigen Duftnote | | |
|---|---|---|
| Einsatzstoff | 25A Anteile in ‰ | 25B Anteile in ‰ |
| Vanillin | 2,0 | 2,0 |
| Ethylenbrassilat | 110,5 | 110,5 |
| Indol | 0,5 | 0,5 |

Tabelle 26:

| Rezeptur des Haarsprays [Gew.-%] | |
|---|---|
| Alberdingk U®500 | 5,0 |
| Luviskol®VA64 | 3,0 |
| Panthenol | 0,5 |
| Parfümöl | 1,0 |
| Dimethylether | 40,0 |
| Wasser | ad 100 |

Alberdingk U 500: anionische Polyether-Polyurethan-Dispersion (40% in Wasser) (Fa. ALBERDINGK BOLEY)

Luviskol VA64: Vinylacetat-Vinylpyrrolidon-Copolymer (Fa. BASF)

Tabelle 27:

| Ergebnis des Geruchstests nach dem Sprühen auf Haarsträhnen mit dem Haarspray aus Tabelle 25 | | | | |
|---|---|---|---|---|
| | Parfümöl 25A | | Parfümöl 25B | |
| | Intensität | Gefallen | Intensität | Gefallen |
| frisch | 4,8 | 4,8 | 4,9 | 4,7 |
| nach 2 Stunden | 4,0 | 4,4 | 3,9 | 4,4 |
| nach 5 Stunden | 3,2 | 3,8 | 2,9 | 3,6 |
| nach 8 Stunden | 2,4 | 3,2 | 1,8 | 2,8 |

**Patentansprüche**

1. Verwendung von Kieselsäureestern der allgemeinen Formel I

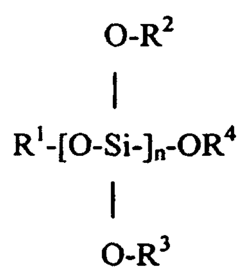

(I)

in der $R^1$ und $R^4$ unabhängig voneinander ausgewählt sind aus der Gruppe der geradkettigen oder verzweigten, gesättigten oder ungesättigten, substituierten oder unsubstituierten $C_{1-6}$-Kohlenwasserstoffreste und der Duftstoffalkoholreste, jedes $R^2$ und $R^3$ unabhängig voneinander ausgewählt ist aus der Gruppe der Duftstoffalkoholreste und n Werte zwischen 2 und 20 annimmt, zur Verlängerung der Duftwirkung von anderen Duftstoffen.

**2.** Verwendung der Kieselsäureester der Formel I nach Anspruch 1, **dadurch gekennzeichnet, daß** $R^1$ und $R^4$ unabhängig voneinander ausgewählt sind aus der Gruppe Methyl, Ethyl, *n*-Propyl, *iso*-Propyl, *n*-Butyl, *iso*-Butyl und *tert*-Butyl.

**3.** Verwendung der Kieselsäureester nach einem der Ansprüche 1 oder 2, **dadurch gekennzeichnet, daß** jedes $R^2$ und $R^3$ unabhängig voneinander ausgewählt ist aus der Gruppe der Reste folgender Duftstoffalkohole: 10-Undecen-1-ol., 2,6-Dimethylheptan-2-ol, 2-Methylbutanol, 2-Methylpentanol, 2-Phenoxyethanol, 2-Phenylpropanol, 2-tert-Butycyclohexanol, 3,5,5-Trimethylcyclohexanol, 3-Hexanol, 3-Methyl-5-phenyl pentanol, 3-Octanol, 3-Phenylpropanol, 4-Heptenol, 4-Isopropyl cyclohexanol, 4-tert-Butycyclohexanol, 6,8-Dimethyl-2-nonanol, 6-Nonen-1-ol, 9-Decen-1-ol, alpha-Methylbenzylalkohol, alpha-Terpineol, Amylsalicylat, Benzylalkohol, Benzylsalicylat, beta-Terpineol, Butylsalicylat, Citronellol, Cyclohexylsalicylat, Decanol, Dihydromyrcenol, Dimethylbenzylcarbinol, Dimethylheptanol, Dimethyloctanol, Ethylsalicylat, Ethylvanilin, Eugenol, Geraniol, Heptanol, Hexylsalicylat, Isoborneol, Isoeugenol, Isopulegol, Linalool, Menthol, Myrtenol, n-Hexanol, Nerol, Nonanol, Octanol, para-Menthan-7-ol, Phenylethylalkohol, Phenol, Phenylsalicylat, Tetrahydrogeraniol, Tetrahydrolinalool, Thymol, trans-2-cis-6-Nonadicnol, trans-2-Nonen-1-ol, trans-2-Octenol, Undecanol, Vanillin, Zimtalkohol.

**4.** Verwendung der Kieselsäureester nach einem der Ansprüche 1 bis 3, **dadurch gekennzeichnet, daß** jeder Rest $R^1$ bis $R^4$ unabhängig voneinander ausgewählt ist aus der Gruppe der Reste folgender Duftstoffalkohole: 10-Undecen-1-ol., 2,6-Dimethylheptan-2-ol, 2-Methylbutanol, 2-Methylpentanol, 2-Phenoxyethanol, 2-Phenylpropanol, 2-tert-Butycyclohexanol, 3,5,5-Trimethylcyclohexanol, 3-Hexanol, 3-Methyl-5-phenyl pentanol, 3-Octanol, 3-Phenylpropanol, 4-Heptenol, 4-Isopropyl cyclohexanol, 4-tert-Butycyclohexanol, 6,8-Dimethyl-2-nonanol, 6-Nonen-1-ol, 9-Decen-1-ol, alpha-Methylbenzylalkohol, alpha-Terpineol, Amylsalicylat, Benzylalkohol, Benzylsalicylat, beta-Terpineol, Butylsalicylat, Citronellol, Cyclohexylsalicylat, Decanol, Dihydromyrcenol, Dimethylbenzylcarbinol, Dimethylheptanol, Dimethyloctanol, Ethylsalicylat, Ethylvanilin, Eugenol, Geraniol, Heptanol, Hexylsalicylat, Isoborneol, Isoeugenol, Isopulegol, Linalool, Menthol, Myrtenol, n-Hexanol, Nerol, Nonanol, Octanol, para-Menthan-7-ol, Phenylethylalkohol, Phenol, Phenylsalicylat, Tetrahydrogeraniol, Tetrahydrolinalool, Thymol, trans-2-cis-6-Nonadicnol, trans-2-Nonen-1-ol, trans-2-Octenol, Undecanol, Vanillin, Zimtalkohol.

**5.** Verwendung der Kieselsäureester nach einem der Ansprüche 1 bis 4, **dadurch gekennzeichnet, daß** n Werte zwischen 2 und 15, vorzugsweise zwischen 2 und 12 und insbesondere zwischen 3 und 10 unter besonderer Bevorzugung der Werte 4, 5, 6, 7 und 8, annimmt.

**6.** Verwendung der Kieselsäureester nach einem der Ansprüche 1 bis 5, **dadurch gekennzeichnet, daß** die Reste $R^1$ bis $R^4$ identisch sind.

**7.** Verwendung der Kieselsäureester nach einem der Ansprüche 1 bis 6 zusammen mit anderen Duftstoffen in flüssigen oder festen Wasch- und Reinigungsmitteln.

**8.** Verwendung der Kieselsäureester nach einem der Ansprüche 1 bis 6 zusammen mit anderen Duftstoffen in kosmetischen Mitteln zur Haut- oder Haarbehandlung.

**9.** Verwendung nach einem der Ansprüche 7 oder 8, **dadurch gekennzeichnet, daß** die Kieselsäureester der Formel I in Mengen von 0,001 bis 10 Gew.-%, vorzugsweise von 0,01 bis 5 Gew.-%, besonders bevorzugt von 0,02 bis 3 und insbesondere in Mengen von 0,05 bis 2 Gew.-%, jeweils bezogen auf das Mittel, eingesetzt werden.

**10.** Duftstoffmischungen oder Parfümzusammensetzungen, enthaltend Kieselsäureester der Formel I

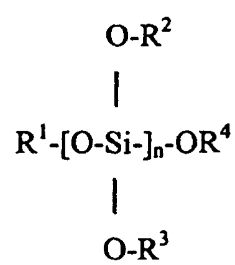

(I)

in der $R^1$ und $R^4$ unabhängig voneinander ausgewählt sind aus der Gruppe der geradkettigen oder verzweigten, gesättigten oder ungesättigten, substituierten oder unsubstituierten $C_{1-6}$-Kohlenwasserstoffreste und der Duftstoffalkoholreste, jedes $R^2$ und $R^3$ unabhängig voneinander ausgewählt ist aus der Gruppe der Duftstoffalkoholreste und n Werte zwischen 2 und 20 annimmt, sowie andere Duftstoffe.

**11.** Wasch- oder Reinigungsmittel, enthaltend Mittel nach Anspruch 10, wobei die Kieselsäureester der Formel I in Mengen von 0,001 bis 10 Gew.-%, vorzugsweise von 0,01 bis 5 Gew.-%, besonders bevorzugt von 0,02 bis 3 und insbesondere in Mengen von 0,05 bis 2 Gew.-%, jeweils bezogen auf das Wasch- und Reinigungsmittel, enthalten sind.

**12.** Wasch- oder Reinigungsmittel nach Anspruch 11, **dadurch gekennzeichnet, daß** es sich um ein flüssiges oder gelförmiges Mittel, insbesondere ein Flüssigwaschmittel oder ein flüssiges Geschirrspülmittel oder ein Reinigungsgel, insbesondere ein gelförmiges Reinigungsmittel für Spültoiletten handelt.

**13.** Wasch- oder Reinigungsmittel nach einem der Ansprüche 11 oder 12, **dadurch gekennzeichnet, daß** es sich um Reiniger aus der Gruppe flüssige oder gelförmige Reiniger für harte Oberflächen, insbesondere sogenannte Allzweckreiniger, Glasreiniger, Boden- oder Badezimmerreiniger, sowie spezielle Ausführungsformen derartiger Reiniger wozu saure oder alkalische Formen von Allzweckreinigern ebenso wie Glasreiniger mit sogenannter Anti-Rain-Wirkung gehören, handelt.

**14.** Wasch- oder Reinigungsmittel nach einem der Ansprüche 11 bis 13, **dadurch gekennzeichnet, daß** es sich um flüssige Reinigungsmittel handelt, die in mehreren Phasen, insbesondere in zwei Phasen, vorliegen.

**15.** Wasch- oder Reinigungsmittel nach Anspruch 11, **dadurch gekennzeichnet, daß** es sich um ein pulverförmiges oder granulatförmiges Mittel handelt.

**16.** Wasch- oder Reinigungsmittel nach Anspruch 11, **dadurch gekennzeichnet, daß** das Mittel in Form von Formkörpern vorliegt, wobei es sich vorzugsweise um Tabletten handelt, die aus einer einzigen oder aber aus mehreren insbesondere 2 oder 3 verschiedenen Phasen bestehen können.

**17.** Kosmetisches Mittel zur Haar- oder Hautbehandlung, enthaltend Mittel nach Anspruch 10, wobei die Kieselsäureester der Formel I vorteilhafterweise in Mengen von 0,001 bis 10 Gew.-%, vorzugsweise von 0,01 bis 5 Gew.-%, besonders bevorzugt von 0,02 bis 3 und insbesondere in Mengen von 0,05 bis 2 Gew.-%, jeweils bezogen auf das kosmetische Mittel, enthalten sind.

**18.** Kosmetisches Mittel nach Anspruch 17, **dadurch gekennzeichnet, daß** es sich um eine wäßrige Zubereitung, die oberflächenaktive Wirkstoffe enthält und sich insbesondere zur Behandlung von Keratinfasern, insbesondere menschlichen Haaren, oder zur Behandlung von Haut eignet, handelt.

**19.** Kosmetisches Mittel nach Anspruch 17, **dadurch gekennzeichnet, daß** es sich um einen Formkörper handelt, der oberflächenaktive Inhaltsstoffe enthält.

**20.** Kosmetisches Mittel nach einem der Ansprüche 17 oder 19, **dadurch gekennzeichnet, daß** es sich um ein Mittel zur Beeinflussung des Körpergeruchs, insbesondere um ein deodorierendes Mittel handelt.

**21.** Kosmetisches Mittel nach Anspruch 17, **dadurch gekennzeichnet, daß** es sich um ein Haarfestlegemittel handelt, das zur Festigung Polymere, vorzugsweise insbesondere mindestens ein Polyurethan, enthält.

**22.** Kieselsäureester der Formel Ia

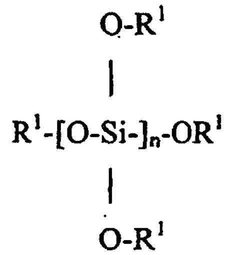 (Ia)

in der $R^1$ ausgewählt ist aus der Gruppe der Duftstoffalkoholreste, insbesondere aus der Gruppe der Reste folgender Duftstoffalkohole, 10-Undecen-1-ol., 2,6-Dimethylheptan-2-ol, 2-Methylbutanol, 2-Methylpentanol, 2-Phenoxyethanol, 2-Phenylpropanol, 2-tert-Butycyclohexanol, 3,5,5-Trimethylcyclohexanol, 3-Hexanol, 3-Methyl-5-phenyl pentanol, 3-Octanol, 3-Phenylpropanol, 4-Heptenol, 4-Isopropyl cyclohexanol, 4-tert-Butycyclohexanol, 6,8-Dimethyl-2-nonanol, 6-Nonen-1-ol, 9-Decen-1-ol, alpha-Methylbenzylalkohol, alpha-Terpineol, Amylsalicylat, Benzylalkohol, Benzylsalicylat, beta-Terpineol, Butylsalicylat, Citronellol, Cyclohexylsalicylat, Decanol, Dihydromyrcenol, Dimethylbenzylcarbinol, Dimethylheptanol, Dimethyloctanol, Ethylsalicylat, Ethylvanilin, Eugenol, Geraniol, Heptanol, Hexylsalicylat, Isoborneol, Isoeugenol, Isopulegol, Linalool, Menthol, Myrtenol, n-Hexanol, Nerol, Nonanol, Octanol, para-Menthan-7-ol, Phenylethylalkohol, Phenol, Phenylsalicylat, Tetrahydrogeraniol, Tetrahydrolinalool, Thymol, trans-2-cis-6-Nonadicnol, trans-2-Nonen-1-ol, trans-2-Octenol, Undecanol, Vanillin, Zimtalkohol, und n Werte zwischen 2 und 20 annimmt.

**23.** Kieselsäureester nach Anspruch 22, **dadurch gekennzeichnet, daß** n Werte zwischen 2 und 15, vorzugsweise zwischen 2 und 12 und insbesondere zwischen 3 und 10 unter besonderer Bevorzugung der Werte 4, 5, 6, 7 und 8, annimmt.

## Revendications

**1.** Utilisation d'esters d'acides siliciques répondant à la formule générale I

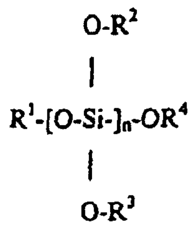 (I)

dans laquelle $R^1$ et $R^4$ sont choisis, indépendamment l'un de l'autre, parmi le groupe des radicaux d'hydrocarbures en $C_1$-$C_6$ substitués ou non substitués, saturés ou insaturés, à chaînes droites ou ramifiées et des radicaux d'alcools de matières odoriférantes, chacun des radicaux $R^2$ et $R^3$ est choisi, indépendamment l'un de l'autre, parmi le groupe des radicaux d'alcools de matières odoriférantes et n prend des valeurs entre 2 et 20, pour prolonger l'effet odoriférant d'autres matières odoriférantes.

**2.** Utilisation des esters d'acides siliciques répondant à la formule I selon la revendication 1, **caractérisée en ce que** $R^1$ et $R^4$ sont choisis, indépendamment l'un de l'autre, parmi le groupe comprenant un groupe méthyle, un groupe éthyle, un groupe n-propyle, un groupe isopropyle, un groupe n-butyle, un groupe isobutyle et un groupe tert-butyle.

**3.** Utilisation des esters d'acides siliciques selon l'une quelconque des revendications 1 ou 2, **caractérisée en ce que** chacun des radicaux $R^2$ et $R^3$ est choisi, indépendamment l'un de l'autre, parmi le groupe des radicaux d'alcools de matières odoriférantes suivants : le 10-undécén-1-ol, le 2,6-diméthylheptan-2-ol, le 2-méthylbutanol, le 2-méthylpentanol, le 2-phénoxyéthanol, le 2-phénylpropanol, le 2-tert-butylcyclohexanol, le 3,5,5-triméthylcyclohexanol, le 3-hexanol, le 3-méthyl-5-phényl-pentanol, le 3-octanol, le 3-phénylpropanol, le 4-hepténol, le 4-isopropylcyclohexanol, le 4-tert-butylcyclohexanol, le 6,8-diméthyl-2-nonanol, le 6-nonén-1-ol, le 9-décén-1-ol, l'alcool -méthylbenzylique, l'-terpinéol, le salicylate d'amyle, l'alcool benzylique, le salicylate de benzyle, le -terpinéol, le salicylate de butyle, le citronnellol, le salicylate de cyclohexyle, le décanol, le dihydromyrcénol, le diméthylbenzylcarbinol, le diméthylheptanol, le diméthyloctanol, le salicylate d'éthyle, l'éthylvanilline, l'eugénol, le géraniol, l'heptanol, le salicylate d'hexyle, l'isobornéol, l'isoeugénol, l'isopulégol, le linalol, le menthol, le myrténol, le n-hexanol, le nérol, le nonanol, l'octanol, le para-menthan-7-ol, l'alcool phényléthylique, le phénol, le salicylate de phényle, le tétrahydrogéraniol, le tétrahydrolinalol, le thymol, le trans-2-cis-6-nonadicnol, le trans-2-nonén-1-ol, le trans-2-octénol, le undécanol, la vanilline, l'alcool cinnamique.

**4.** Utilisation des esters d'acides siliciques selon l'une quelconque des revendications 1 à 3, **caractérisée en ce que** chacun des radicaux $R^1$ à $R^4$ est choisi, indépendamment l'un de l'autre, parmi le groupe des radicaux d'alcools de matières odoriférantes suivants : le 10-undécén-1-ol, le 2,6-diméthylheptan-2-ol, le 2-méthylbutanol, le 2-méthylpentanol, le 2-phénoxyéthanol, le 2-phénylpropanol, le 2-tert-butylcyclohexanol, le 3,5,5-triméthylcyclohexanol, le 3-hexanol, le 3-méthyl-5-phényl-pentanol, le 3-octanol, le 3-phénylpropanol, le 4-hepténol, le 4-isopropylcyclohexanol, le 4-tert-butylcyclohexanol, le 6,8-diméthyl-2-nonanol, le 6-nonén-1-ol, le 9-décén-1-ol, l'alcool -méthylbenzylique, l'-terpinéol, le salicylate d'amyle, l'alcool benzylique, le salicylate de benzyle, le -terpinéol, le salicylate de butyle, le citronnellol, le salicylate de cyclohexyle, le décanol, le dihydromyrcénol, le diméthylbenzylcarbinol, le diméthylheptanol, le diméthyloctanol, le salicylate d'éthyle, l'éthylvanilline, l'eugénol, le géraniol, l'heptanol, le salicylate d'hexyle, l'isobornéol, l'isoeugénol, l'isopulégol, le linalol, le menthol, le myrténol, le n-hexanol, le nérol, le nonanol, l'octanol, le para-menthan-7-o1, l'alcool phényléthylique, le phénol, le salicylate de phényle, le tétrahydrogéraniol, le tétrahydrolinalol, le thymol, le trans-2-cis-6-nonadicnol, le trans-2-nonén-1-ol, le trans-2-octénol, le undécanol, la vanilline, l'alcool cinnamique.

**5.** Utilisation des esters d'acides siliciques selon l'une quelconque des revendications 1 à 4, **caractérisée en ce que** n prend des valeurs entre 2 et 15, de préférence entre 2 et 12 et en particulier entre 3 et 10, de manière particulièrement préférée les valeurs 4, 5, 6, 7 et 8.

**6.** Utilisation des esters d'acides siliciques selon l'une quelconque des revendications 1 à 5, **caractérisée en ce que** les radicaux $R^1$ à $R^4$ sont identiques.

**7.** Utilisation des esters d'acides siliciques selon l'une quelconque des revendications 1 à 6, de manière conjointe avec d'autres matières odoriférantes dans des agents de lavage et de nettoyage liquides ou solides.

**8.** Utilisation des esters d'acides siliciques selon l'une quelconque des revendications 1 à 6, de manière conjointe avec d'autres matières odoriférantes dans des agents cosmétiques pour le traitement de la peau ou des cheveux.

**9.** Utilisation selon l'une quelconque des revendications 7 ou 8, **caractérisée en ce que** les esters d'acides siliciques répondant à la formule I sont mis en oeuvre dans des quantités de 0,001 à 10 % en poids, de préférence de 0,01 à 5 % en poids, de manière particulièrement préférée de 0,02 à 3 et en particulier dans des quantités de 0,05 à 2 % en poids, chaque fois rapportés à l'agent.

**10.** Mélanges de matières odoriférantes ou compositions de parfums, contenant des esters d'acides siliciques répondant à la formule I

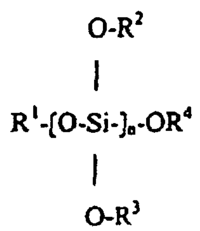

(I)

dans laquelle $R^1$ et $R^4$ sont choisis, indépendamment l'un de l'autre, parmi le groupe des radicaux d'hydrocarbures en $C_1$-$C_6$ substitués ou non substitués, saturés ou insaturés, à chaînes droites ou ramifiées et des radicaux d'alcools de matières odoriférantes, chacun des radicaux $R^2$ et $R^3$ est choisi, indépendamment l'un de l'autre, parmi le groupe des radicaux d'alcools de matières odoriférantes et n prend des valeurs entre 2 et 20, et d'autres matières

odoriférantes.

**11.** Agent de lavage ou de nettoyage contenant des agents selon la revendication 10, les esters d'acides siliciques répondant à la formule I étant contenus dans des quantités de 0,001 à 10 % en poids, de préférence de 0,01 à 5 % en poids, de manière particulièrement préférée de 0,02 à 3 et en particulier dans des quantités de 0,05 à 2 % en poids, chaque fois rapportés à l'agent de lavage et de nettoyage.

**12.** Agent de lavage ou de nettoyage selon la revendication 11, **caractérisé en ce qu'**il s'agit d'un agent sous forme liquide ou sous forme de gel, en particulier d'un agent de lavage liquide ou d'un agent liquide pour le lavage mécanique de la vaisselle ou encore d'un gel de nettoyage, en particulier d'un agent de nettoyage sous forme de gel pour des toilettes avec chasse d'eau.

**13.** Agents de lavage ou de nettoyage selon l'une quelconque des revendications 11 ou 12, **caractérisés en ce qu'**il s'agit d'agents de nettoyage choisis parmi le groupe comprenant des agents de nettoyage sous forme liquide ou sous forme de gel pour des surfaces dures, en particulier ce que l'on appelle des agents de nettoyage pour tous usages, des agents de nettoyage pour les vitres, des agents de nettoyage pour sols ou pour salles de bains, ainsi que des formes de réalisation spécifiques d'agents de nettoyage de ce type, dont font partie des formes acides ou alcalines d'agents de nettoyage pour tous usages au même titre que des agents de nettoyage pour les vitres possédant ce qu'il est convenu d'appeler un effet anti-pluie.

**14.** Agents de lavage ou de nettoyage selon l'une quelconque des revendications 11 à 13, **caractérisés en ce qu'**il s'agit d'agents de nettoyage liquides qui sont présents dans plusieurs phases, en particulier dans deux phases.

**15.** Agent de lavage ou de nettoyage selon la revendication 11, **caractérisé en ce qu'**il s'agit d'un agent sous forme pulvérulente ou sous la forme d'un produit de granulation.

**16.** Agent de lavage ou de nettoyage selon la revendication 11, **caractérisé en ce que** l'agent est présent sous la forme de corps moulés à propos desquels il s'agit de préférence de comprimés qui peuvent être constitués par une phase unique ou encore par plusieurs phases différentes, en particulier par 2 ou 3 phases différentes.

**17.** Agent cosmétique pour le traitement des cheveux ou de la peau, contenant des agents selon la revendication 10, les esters d'acides siliciques répondant à la formule I étant contenus, de préférence, dans des quantités de 0,001 à 10 % en poids, de préférence de 0,01 à 5 % en poids, de manière particulièrement préférée de 0,02 à 3 et en particulier dans des quantités de 0,05 à 2 % en poids, chaque fois rapportés à l'agent cosmétique.

**18.** Agent cosmétique selon la revendication 17, **caractérisé en ce qu'**il s'agit d'une préparation aqueuse qui contient des substances tensioactives et qui est appropriée en particulier pour le traitement de fibres kératiniques, en particulier de cheveux humains, ou pour le traitement de la peau.

**19.** Agent cosmétique selon la revendication 17, **caractérisé en ce qu'**il s'agit d'un corps moulé qui contient des constituants tensioactifs.

**20.** Agent cosmétique selon l'une quelconque des revendications 17 ou 19, **caractérisé en ce qu'**il s'agit d'un agent pour influencer l'odeur corporelle, en particulier d'un agent désodorisant.

**21.** Agent cosmétique selon la revendication 17, **caractérisé en ce qu'**il s'agit d'un agent de permanente des cheveux qui contient, pour la permanente, des polymères, de manière particulièrement préférée au moins un polyuréthane.

**22.** Esters d'acides siliciques répondant à la formule la

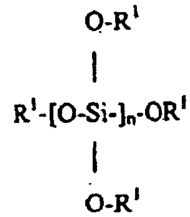

(Ia)

dans laquelle $R^1$ est choisi parmi le groupe des radicaux d'alcools de matières odoriférantes, en particulier parmi le groupe des radicaux d'alcools de matières odoriférantes suivants : le 10-undécén-1-ol, le 2,6-diméthyl-heptan-2-ol, le 2-méthylbutanol, le 2-méthylpentanol, le 2-phénoxyéthanol, le 2-phénylpropanol, le 2-tert-butylcyclohexanol, le 3,5,5-triméthylcyclohexanol, le 3-hexanol, le 3-méthyl-5-phényl-pentanol, le 3-octanol, le 3-phénylpropanol, le 4-hepténol, le 4-isopropylcyclohexanol, le 4-tert-butylcyclohexanol, le 6,8-diméthyl-2-nonanol, le 6-nonén-1-ol, le 9-décén-1-ol, l'alcool -méthylbenzylique, l'-terpinéol, le salicylate d'amyle, l'alcool benzylique, le salicylate de benzyle, le -terpinéol, le salicylate de butyle, le citronnellol, le salicylate de cyclohexyle, le décanol, le dihydromyrcénol, le diméthylbenzylcarbinol, le diméthylheptanol, le diméthyloctanol, le salicylate d'éthyle, l'éthylvanilline, l'eugénol, le géraniol, l'heptanol, le salicylate d'hexyle, l'isobornéol, l'isoeugénol, l'isopulégol, le linalol, le menthol, le myrténol, le n-hexanol, le nérol, le nonanol, l'octanol, le para-menthan-7-ol, l'alcool phényléthylique, le phénol, le salicylate de phényle, le tétrahydrogéraniol, le tétrahydrolinalol, le thymol, le trans-2-cis-6-nonadicnol, le trans-2-nonén-1-ol, le trans-2-octénol, le undécanol, la vanilline, l'alcool cinnamique, et n prend des valeurs entre 2 et 20.

**23.** Esters d'acides siliciques selon la revendication 22, **caractérisés en ce que** n prend des valeurs entre 2 et 15, de préférence entre 2 et 12, et en particulier entre 3 et 10, de manière particulièrement préférée les valeurs 4, 5, 6, 7 et 8.

## Claims

**1.** Use of silicic acid esters of the general formula I

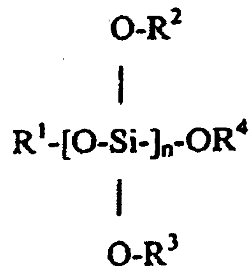

(I)

in which $R^1$ and $R^4$, independently of one another, are chosen from the group of straight-chain or branched, saturated or unsaturated, substituted or unsubstituted $C_{1-6}$-hydrocarbon radicals and fragrance alcohol radicals, each $R^2$ and $R^3$, independently of the other, is chosen from the group of fragrance alcohol radicals and n assumes values between 2 and 20, for prolonging the scent effect of other fragrances.

**2.** Use of the silicic acid esters of the formula I according to Claim 1, **characterized in that** $R^1$ and $R^4$, independently of one another, are chosen from the group consisting of methyl, ethyl, n-propyl, isopropyl, n-butyl, isobutyl and tert-butyl.

**3.** Use of the silicic acid esters according to either of Claims 1 and 2, **characterized in that** each $R^2$ and $R^3$, independently of the other, is chosen from the group of the radicals of the following fragrance alcohols: 10-undecen-1-ol, 2,6-dimethylheptan-2-ol, 2-methylbutanol, 2-methylpentanol, 2-phenoxyethanol, 2-phenylpropanol, 2-tert-butylcyclohexanol, 3,5,5-trimethylcyclohexanol, 3-hexanol, 3-methyl-5-phenylpentanol, 3-octanol, 3-phenylpropanol, 4-heptenol, 4-isopropylcyclohexanol, 4-tert-butylcyclohexanol, 6,8-dimethyl-2-nonanol, 6-nonen-1-ol, 9-decen-1-ol, alpha-methylbenzyl alcohol, alpha-terpineol, amyl salicylate, benzyl alcohol, benzyl salicylate, beta-terpineol, butyl salicylate, citronellol, cyclohexyl salicylate, decanol, dihydromyrcenol, dimethylbenzylcarbinol, dimethylheptanol, dimethyloctanol, ethyl salicylate, ethylvanillin, eugenol, geraniol, heptanol, hexyl salicylate, isoborneol, isoeugenol, isopulegol, linalool, menthol, myrtenol, n-hexanol, nerol, nonanol, octanol, para-menthan-7-ol, phenylethyl alcohol, phenol, phenyl salicylate, tetrahydrogeraniol, tetrahydrolinalool, thymol, trans-2-cis-6-nonadienol, trans-2-nonen-1-ol, trans-2-octenol, undecanol, vanillin, cinnamyl alcohol.

**4.** Use of the silicic acid esters according to one of Claims 1 to 3, **characterized in that** each radical $R^1$ to $R^4$, independently of the others, is chosen from the group of radicals of the following fragrance alcohols: 10-undecen-1-ol, 2,6-dimethyl-heptan-2-ol, 2-methylbutanol, 2-methylpentanol, 2-phenoxyethanol, 2-phenylpropanol, 2-tert-butylcyclohexanol, 3,5,5-trimethylcyclohexanol, 3-hexanol, 3-methyl-5-phenylpentanol, 3-octanol, 3-phenylpropanol, 4-heptenol, 4-isopropylcyclohexanol, 4-tert-butylcyclohexanol, 6,8-dimethyl-2-nonanol, 6-nonen-1-ol, 9-decen-1-ol, alpha-methylbenzyl alcohol, alpha-terpineol, amyl salicylate, benzyl alcohol, benzyl salicylate, beta-terpineol, butyl salicylate, citronellol, cyclohexyl salicylate, decanol, dihydromyrcenol, dimethylbenzylcarbinol, dimethylheptanol, dimethyloctanol, ethyl salicylate, ethylvanillin, eugenol, geraniol, heptanol, hexyl salicylate, isoborneol, isoeugenol, isopulegol, linalool, menthol, myrtenol, n-hexanol, nerol, nonanol, octanol, para-menthan-7-ol, phenylethyl alcohol, phenol, phenyl salicylate, tetrahydrogeraniol, tetrahydrolinalool, thymol, trans-2-cis-6-nonadienol, trans-2-nonen-1-ol, trans-2-octenol, undecanol, vanillin, cinnamyl alcohol.

**5.** Use of the silicic acid esters according to one of Claims 1 to 4, **characterized in that** n assumes values between 2 and 15, preferably between 2 and 12 and in particular between 3 and 10, with particular preference the values 4, 5, 6, 7 and 8.

**6.** Use of the silicic acid esters according to one of Claims 1 to 5, **characterized in that** the radicals $R^1$ to $R^4$ are identical.

**7.** Use of the silicic acid esters according to one of Claims 1 to 6 together with other fragrances in liquid or solid detergents and cleaners.

**8.** Use of the silicic acid esters according to one of Claims 1 to 6 together with other fragrances in cosmetic compositions for the treatment of skin or hair.

**9.** Use according to either of Claims 7 and 8, **characterized in that** the silicic acid esters of the formula I are used in amounts of from 0.001 to 10% by weight, preferably from 0.01 to 5% by weight, particularly preferably from 0.02 to 3% by weight and in particular in amounts of from 0.05 to 2% by weight, in each case based on the composition.

**10.** Fragrance mixtures or perfume compositions comprising silicic acid esters of the formula I

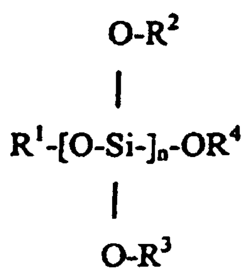

(I)

in which $R^1$ and $R^4$, independently of one another, are chosen from the group of straight-chain or branched, saturated or unsaturated, substituted or unsubstituted $C_{1-6}$-hydrocarbon radicals and fragrance alcohol radicals, each $R^2$ and $R^3$, independently of the other, is chosen from the group of fragrance alcohol radicals and n assumes values between 2 and 20, and other fragrances.

**11.** Detergents or cleaners comprising compositions according to Claim 10, where the silicic acid esters of the formula I are present in amounts of from 0.001 to 10% by weight, preferably from 0.01 to 5% by weight, particularly preferably from 0.02 to 3% by weight and in particular in amounts of from 0.05 to 2% by weight, in each case based on the detergent and cleaner.

**12.** Detergent or cleaner according to Claim 11, **characterized in that** it is a liquid or gel-like composition, in particular a liquid detergent or a liquid dishwashing detergent or a cleaning gel, in particular a gel-like cleaning composition for flush toilets.

**13.** Detergents or cleaners according to either of Claims 11 and 12, **characterized in that** they are cleaners from the group of liquid or gel-like cleaners for hard surfaces, in particular so-called all-purpose cleaners, glass cleaners, floor or bathroom cleaners, and specific variants of such cleaners, which include acidic or alkaline forms of all-purpose cleaners as well as glass cleaners with so-called antirain effect.

**14.** Detergents or cleaners according to one of Claims 11 to 13, **characterized in that** they are liquid cleaners which are present in a plurality of phases, in particular in two phases.

**15.** Detergent or cleaner according to Claim 11, **characterized in that** it is a pulverulent or granular composition.

**16.** Detergent or cleaner according to Claim 11, **characterized in that** the composition is present in the form of shaped bodies, which are preferably tablets which can consist of a single phase or else of a plurality of phases, in particular 2 or 3 different phases.

**17.** Cosmetic composition for the treatment of hair or skin, comprising compositions according to Claim 10, where the silicic acid esters of the formula I are advantageously present in amounts of from 0.001 to 10% by weight, preferably from 0.01 to 5% by weight, particularly preferably from 0.02 to 3% by weight and in particular in amounts of from 0.05 to 2% by weight, in each case based on the cosmetic composition.

**18.** Cosmetic composition according to Claim 17, **characterized in that** it is an aqueous preparation which comprises surface-active ingredients and is particularly suitable for the treatment of keratin fibres, in particular human hair, or for the treatment of skin.

**19.** Cosmetic composition according to Claim 17, **characterized in that** it is a shaped body which comprises surface-active ingredients.

**20.** Cosmetic composition according to either of Claims 17 or 19, **characterized in that** it is a composition for influencing body odour, in particular a deodorizing composition.

**21.** Cosmetic composition according to Claim 17, **characterized in that** it is a hair-setting composition which, for the setting, comprises polymers, preferably in particular at least one polyurethane.

**22.** Silicic acid esters of the formula Ia

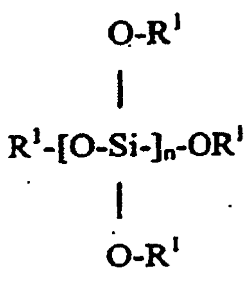

(Ia)

in which $R^1$ is chosen from the group of fragrance alcohol radicals, in particular from the group of radicals of the following fragrance alcohols 10-undecen-1-ol, 2,6-dimethylheptan-2-ol, 2-methylbutanol, 2-methylpentanol, 2-phenoxyethanol, 2-phenylpropanol, 2-tert-butylcyclohexanol, 3,5,5-trimethylcyclohexanol, 3-hexanol, 3-methyl-5-phenylpentanol, 3-octanol, 3-phenylpropanol, 4-heptenol, 4-isopropylcyclohexanol, 4-tert-butylcyclohexanol, 6,8-dimethyl-2-nonanol, 6-nonen-1-ol, 9-decen-1-ol, alpha-methylbenzyl alcohol, alpha-terpineol, amyl salicylate,

benzyl alcohol, benzyl salicylate, beta-terpineol, butyl salicylate, citronellol, cyclohexyl salicylate, decanol, dihydromyrcenol, dimethylbenzylcarbinol, dimethylheptanol, dimethyloctanol, ethyl salicylate, ethylvanillin, eugenol, geraniol, heptanol, hexyl salicylate, isoborneol, isoeugenol, isopulegol, linalool, menthol, myrtenol, n-hexanol, nerol, nonanol, octanol, para-menthan-7-ol, phenylethyl alcohol, phenol, phenyl salicylate, tetrahydrogeraniol, tetrahydrolinalool, thymol, trans-2-cis-6-nonadienol, trans-2-nonen-1-ol, trans-2-octenol, undecanol, vanillin, cinnamyl alcohol, and n assumes values between 2 and 20.

**23.** Silicic acid esters according to Claim 22, **characterized in that** n assumes values between 2 and 15, preferably between 2 and 12 and in particular between 3 and 10, with particular preference the values 4, 5, 6, 7 and 8.